# EUROPEAN PATENT SPECIFICATION

(11) **EP 4 447 804 B1**
(45) Date of publication and mention of the grant of the patent: **03.12.2025**
(21) Application number: 23714978.6
(22) Date of filing: 09.03.2023
(51) Int. Cl.: A61B 5/1455, A61B 5/00

(54) **FOOT WORN PHYSIOLOGICAL SENSOR AND SYSTEMS INCLUDING SAME**
AM FUSS GETRAGENER PHYSIOLOGISCHER SENSOR UND SYSTEME DAMIT
CAPTEUR PHYSIOLOGIQUE PORTÉ AU PIED ET SYSTÈMES LE COMPRENANT

(30) Priority: 10.03.2022 US 202263318568 P; 12.12.2022 US 202263387045 P
(43) Date of publication of application: 23.10.2024
(73) Proprietor: Masimo Corporation, Irvine, CA 92618 (US)
(72) Inventor: SCRUGGS, Stephen, Irvine, California 92618 (US); SCHRAMM, James Ford, Irvine, California 92618 (US); AMBROSINI, Mitchell Lloyd, Irvine, California 92618 (US); DEJONG, Chad A., Irvine, California 92618 (US); AL-ALI, Ammar, Irvine, California 92618 (US); PRIDDELL, Richard, Irvine, California 92618 (US); HWANG, Sujin, Irvine, California 92618 (US)
(74) Representative: Finlayson, Scott Henry
(86) International application number: PCT/US2023/064061
(87) International publication number: WO 2023/173031

(56) References cited:
- WO-A1-2021/231326
- US-A1- 2015 157 263
- US-A1- 2015 374 293
- US-A1- 2018 098 734

## Description

This application claims the benefit of priority under 35 U.S.C. § 119(e) to U.S. Provisional Patent Application No. 63/387045, filed December 12, 2022, and U.S. Provisional Patent Application No. 63/318568, filed March 10, 2022.

### TECHNICAL FIELD

The present disclosure relates to wearable systems for measuring and/or monitoring a subject's physiological information.

### BACKGROUND

Pulse oximetry is a widely accepted noninvasive procedure for measuring the oxygen saturation level of arterial blood, an indicator of a person's oxygen supply. Pulse oximetry sensors generally include one or more light sources transmitting optical radiation into or reflecting off through a portion of the body. After attenuation by tissue and fluids of the portion of the body, one or more photodetection devices detect the attenuated light and output one or more detector signals responsive to the detected attenuated light. The pulse oximetry sensor can be utilized for determination of a variety of physiological parameters and/or characteristics, including but not limited to oxygen saturation (SpO₂), pulse rate, a plethysmograph waveform, perfusion index (PI), pleth variability index (PVI), methemoglobin (MetHb), carboxyhemoglobin (CoHb), total hemoglobin (tHb), glucose, and/or otherwise, and the pulse oximetry sensor can be utilized for display on one or more monitors the foregoing parameters individually, in groups, in trends, as combinations, or as an overall wellness or other index. Devices incorporating pulse oximetry can be utilized in medical setting (such as hospitals and nursing homes) as well as in non-hospital settings (such as in a home).

US 2015/0157263 A1 discloses a system for wirelessly monitoring the health of an infant comprising a sensing module removably disposed within a wearable article. At least a portion of the sensing module can be in contact with an infant's foot. The sensing module can include a processing unit configured to receive and process health readings received by the sensing module. A wireless transmitter can also be in communication with the processing unit. The wireless transmitter can be configured to transmit the processed health readings to a receiving station. The receiving station can indicate an alarm if the processed health readings indicate a health trend that falls outside of a particular threshold.

### SUMMARY

The present invention provides a system for measuring at least one physiological parameter of a subject as defined in claim 1. Optional embodiments of the invention are defined in the dependent claims. Further implementations are outlined in the following paragraphs.

In some circumstances, particularly for infants with small hands and fingers, it can be advantageous to select a foot as a site for pulse oximetry. The present disclosure describes various implementations of systems which secure to a subject (for example, to a foot, an ankle, and/or a lower leg of the subject). Some implementations include one or more sensors for determining physiological data and/or motion data. Some implementations of the systems disclosed herein employ pulse oximetry at the foot of the subject. Various implementations disclosed herein provide increased user comfort, increased ergonomics, increased convenience, facilitate better sensor-skin contact and engagement in order to provide more accurate physiological parameter determination, and provide better stability in securement. Various implementations of the systems disclosed herein can be utilized in a medical setting (such as a hospital or other care facility) as well as in non-hospital settings (such as in a home).

Some implementations of the systems disclosed herein include a wearable device configured to be secured to a subject's foot and a sensor component. In some implementations, the sensor component is removably secureable to the wearable device. The sensor component can include one or more sensors for determining physiological data and/or motion data. In some implementations, the sensor component includes at least one emitter and at least one detector providing for pulse oximetry functionality. In some implementations, the sensor component includes: a sensor hub that is removably securable to a portion of the wearable device; and a sensor strap that is configured to be wrapped around a portion of the subject's foot and secured to a portion of the wearable device, thereby securing the sensor hub and wearable device to the subject's foot. The sensor hub and the sensor strap can include various electronic components, and can form a unitary structure with one another in some implementations. In some implementations, the sensor hub includes a power source and one or more processors. In some implementations, the wearable device does not include a power source. In some variants, the wearable device and the sensor component are integral with one another (for example, are not separable from one another).

Advantageously, some implementations of the systems disclosed herein can easily be adapted and/or customized to fit subjects with body parts (e.g., feet, ankles, and/or lower legs) of various sizes and/or shapes. For example, some implementations of the systems disclosed herein include a wearable device that is removable from electronic component(s) of the system (for example, the sensor component discussed above), which allows different sizes of the wearable device to be selected and utilized with the same electronic component(s) of the system. As another example, the systems disclosed herein can have one or more adjustable straps that can allow for a customized fit of the system to the subject's foot. Further, the systems described herein (or portions thereof such as the wearable device) can be made of a resilient material that can accommodate and/or adapt to a foot, ankle, and/or lower leg of various sizes and/or shapes. Additionally, the systems disclosed herein (or portions thereof such as the wearable device) can be provided in various sizes and/or shapes (e.g., small, medium, large) to further enable a customized fit for a subject.

Some implementations of the systems disclosed herein can advantageously provide for a system that is reusable and/or durable (e.g., lasting weeks and/or months). Some implementations of the systems disclosed herein incorporate at least one detector in a strap that, when wrapped around a portion of the subject's foot, operably position the at least one detector adjacent a top portion of the subject's foot. Some of such implementations include at least one emitter in a portion of the system that is operably positioned adjacent a bottom portion of the subject's foot and/or substantially aligned with the at least one detector. Some variants include alternative positioning of such at least one emitter and such at least one detector.

Disclosed herein is a system for measuring at least one physiological parameter of a subject, the system comprising: a wearable device configured to be secured to a foot of the subject; and a sensor component removably securable to the wearable device and comprising one or more sensors for measuring said at least one physiological parameter of the subject, said sensor component further comprising a sensor strap configured to be wrapped around a portion of the subject's foot and secured to a portion of the wearable device, thereby securing the wearable device and the sensor component to the subject's foot.

In some implementations: said sensor strap comprises a first portion of the sensor component that is configured to be wrapped around said portion of the subject's foot and secured to a first portion of the wearable device; and a second portion of the sensor component is configured to be removably secured to a second portion of the wearable device. In some implementations, the wearable device defines a first volume configured to receive the subject's foot and a second volume configured to removably receive said second portion of the sensor component. Said second portion of the sensor component can be any of the sensor hubs described and/or illustrated herein. In some implementations, the wearable device comprises: a base configured to contact at least a portion of a bottom of the subject's foot, said second volume of said wearable device formed by a cavity of said base; and a wall extending outward from the base and configured to surround a heel and at least a portion of one or more sides of the subject's foot. In some implementations: the wearable device further comprises a frame arranged within said cavity, said frame configured to removably secure said second portion of the sensor component; said base and said wall form a unitary structure made of a first material; and said frame is made of a second material that is more rigid than the first material. The wearable device can be formed by, for example, overmolding (e.g., via injection molding) the base and/or wall over the frame. In some implementations, said first portion of the wearable device is arranged on a portion of said wall. In some implementations, said first portion of the wearable device comprises an opening in said portion of said wall, and wherein said sensor strap is configured to be inserted through said opening. In some implementations, said first volume is defined by said base and said wall at a location above said cavity of said base. In some implementations, said wall extends around a portion of a perimeter edge of said base. In some implementations, said wall extends around less than an entirety of said perimeter edge of said base. In some implementations, said wall does not extend around an entirety of said cavity.

In some implementations, said sensor component comprises: a sensor hub comprising one or more processors, said sensor hub configured to be removably secured to said second portion of the wearable device, wherein said sensor strap is connected to and extends outward from the sensor hub; one or more emitters configured to emit optical radiation into tissue of the subject's foot, said one or more emitters located within the sensor hub; and one or more detectors configured to detect at least a portion of the emitted optical radiation after passing through the tissue and output at least one signal responsive to the detected optical radiation, said one or more detectors located within the sensor strap, wherein the one or more processors of the sensor hub are configured to receive the at least one signal outputted by the one or more detectors to determine said at least one physiological parameter of the subject.

In some implementations, the system is configured such that, when the sensor hub is secured to said second portion of the wearable device and the sensor strap is secured to said first portion of the wearable device: the one or more detectors are positioned adjacent a top or side portion of the subject's foot; and the one or more emitters are positioned adjacent a bottom portion of the subject's foot.

In some implementations, the sensor hub and the sensor strap form a unitary structure. In some implementations, the sensor strap comprises: a first section connected to and extending outward from the sensor hub, wherein the one or more detectors are positioned within the first section; and a second section that is releasably connectable to the first section, wherein the second section is configured to secure to said first portion of the wearable device. In some implementations: the first and second sections have different lengths; and/or the first and second sections comprise different materials. In some implementations, the first section is more stretchable than the second section. In some implementations, the sensor strap is configured to be stretched to allow adjustment of a position of the one or more detectors relative to the subject's foot. In some implementations, the sensor hub comprises: a housing, the housing comprising an opening configured to be positioned adjacent skin of the subject's foot when the sensor hub is secured to said second portion of the wearable device; a thermally conductive probe positioned at least partially within said opening; and a temperature sensor positioned within said housing. In some implementations, said thermally conductive probe is configured to transmit thermal energy from the skin at least partially toward said temperature sensor. In some implementations, said thermally conductive probe extends through said opening and is configured to contact the skin of the subject's foot.

In some implementations: said sensor strap is configured to be wrapped around the portion of the subject's foot and secured to a first portion of the wearable device; and the system further comprises an additional strap removably securable to a second portion of the wearable device and configured to be: (i) wrapped around another portion of the subject's foot or a portion of an ankle or a leg of the subject and (ii) secured to a third portion of the wearable device. The sensor strap can be secured to the first portion of the wearable device in a variety of ways, for example, by inserting a portion of the sensor strap through an opening in the first portion of the wearable device and then securing a portion of the sensor strap to itself. Similarly, the additional strap can be secured to the third portion of the wearable device by inserting a portion of the additional strap through an opening in the third portion of the wearable device and then securing a portion of the additional strap to itself. In some implementations: said sensor strap is configured to be wrapped around the portion of the subject's foot and secured to a first portion of the wearable device; and the system further comprises an additional strap having a first end that is connected to a second portion of the wearable device and a second end that is configured to be: (i) wrapped around another portion of the subject's foot or a portion of the subject's ankle or leg and (ii) secured to a third portion of the wearable device. The sensor strap can be secured to the first portion of the wearable device in a variety of ways, for example, by inserting a portion of the sensor strap through an opening in the first portion of the wearable device and then securing a portion of the sensor strap to itself. Similarly, the additional strap can be secured to the third portion of the wearable device by inserting a portion of the additional strap through an opening in the third portion of the wearable device and then securing a portion of the additional strap to itself.

Disclosed herein is a system for measuring at least one physiological parameter of a subject, the system comprising: a wearable device configured to be secured to a foot of the subject, said wearable device comprising a cavity; a sensor hub configured to be removably secured within the cavity of the wearable device, said sensor hub comprising one or more processors; a sensor strap connected to and extending outward from the sensor hub, said sensor strap configured to be wrapped around a portion of the subject's foot and secured to a portion of the wearable device; one or more emitters configured to emit optical radiation into tissue of the subject's foot, said one or more emitters arranged within one of the sensor hub and the sensor strap; and one or more detectors configured to detect at least a portion of the emitted optical radiation after passing through the tissue and output at least one signal responsive to the detected optical radiation, said one or more detectors arranged within the other one of the sensor hub and the sensor strap. In some implementations, the one or more processors of the sensor hub are configured to receive the at least one signal outputted by the one or more detectors to determine the at least one physiological parameter of the subject.

In some implementations, the wearable device is configured such that the cavity is positioned adjacent a bottom portion of the subject's foot when the wearable device is secured to the subject's foot. In some implementations, the system is configured such that: the one or more detectors are configured to be positioned adjacent a top portion of the subject's foot when the system is in use; and the one or more emitters are configured to be positioned adjacent a bottom portion of the subject's foot when the system is in use. In some implementations, when the sensor hub is secured within the cavity and the sensor strap is secured to the portion of the wearable device: the one or more detectors are arranged within the sensor strap to face toward the sensor hub; and the one or more emitters are arranged within the sensor hub to face toward the sensor strap. In some implementations, the sensor hub and the sensor strap form a unitary structure. In some implementations, the sensor strap comprises: a first section connected to and extending outward from the sensor hub, wherein the one or more detectors are positioned within the first section; and a second section that is releasably connectable to the first section, wherein the second section is configured to secure to the portion of the wearable device. In some implementations, the first and second sections have different lengths. In some implementations, the first section is more stretchable than the second section.

In some implementations, the wearable device comprises: a main body and a frame. The main body can comprise: a base configured to contact at least a portion of a bottom of the subject's foot, the base comprising said cavity; and a wall extending outward from the base and configured to surround a heel and at least a portion of one or more sides of the subject's foot. The frame can be positioned within said cavity, said frame configured to removably secure to the sensor hub. In some implementations, the main body is made of a first material and the frame is made of a second material that is more rigid than the first material. The wearable device can be formed by, for example, overmolding (e.g., via injection molding) the base and/or wall over the frame. In some implementations: said base comprises a base surface that is configured to contact said at least the portion of the bottom of the subject's foot; said cavity has a first depth below said base surface; and the wearable device further comprises a recess positioned along an exterior edge of the base and adjacent said cavity, said recess having a second depth below said base surface, said second depth being smaller than said first depth and substantially equal to a thickness of the sensor strap, said recess configured to receive a portion of the sensor strap when the sensor hub is secured within said cavity such that the sensor hub and said portion of the sensor strap form a substantially flush surface with said base surface.

In some implementations: said sensor strap is configured to be wrapped around the portion of the subject's foot and secured to the portion of the wearable device, said portion of the wearable device being a first portion of the wearable device; and the system further comprises an additional strap separate from said sensor strap and configured to be: (i) wrapped around another portion of the subject's foot or a portion of an ankle or a leg of the subject and (ii) secured to a second portion of the wearable device. The sensor strap can be secured to the first portion of the wearable device in a variety of ways, for example, by inserting a portion of the sensor strap through an opening in the first portion of the wearable device and then securing a portion of the sensor strap to itself. Similarly, the additional strap can be secured to the second portion of the wearable device by inserting a portion of the additional strap through an opening in the second portion of the wearable device and then securing a portion of the additional strap to itself. In some implementations, the sensor hub comprises: a housing, the housing comprising an opening configured to be positioned adjacent skin of the subject's foot when the sensor hub is secured within the cavity of the wearable device; a thermally conductive probe positioned at least partially within said opening; and a temperature sensor positioned within said housing. In some implementations, said thermally conductive probe is configured to transmit thermal energy from said skin at least partially toward said temperature sensor. In some implementations, said thermally conductive probe extends through said opening and is configured to contact said skin when the system is in use.

In some implementations: said one or more detectors are arranged within the sensor strap and said one or more emitters are arranged within the sensor hub; the wearable device further comprises a flexible circuit extending within a portion of the sensor hub and a portion of the sensor strap and electrically connecting the one or more detectors with the one or more processors or another circuit to which the one or more processors are connected; said portion of the sensor strap is configured to be stretched from a first state to a second state, said portion of the sensor strap having a greater length when in said second state than when in said first state; said one or more detectors are arranged at a first location within said portion of the sensor strap that is spaced a first distance from the sensor hub; and a length of a portion of the flexible circuit that is positioned within said portion of the sensor strap is greater than said first distance to allow the flexible circuit to accommodate said stretching of said portion of the sensor strap from the first state to the second state while maintaining connection between the one or more detectors with the one or more processors or said another circuit to which the one or more processors are connected. In some implementations: said one or more detectors are arranged within the sensor strap and said one or more emitters are arranged within the sensor hub; and said sensor strap is configured to be stretched to allow adjustment of a position of the one or more detectors relative to the subject's foot.

For purposes of summarizing the disclosure, certain aspects, advantages and novel features of several implementations have been described herein. It is to be understood that not necessarily all such advantages are achieved in accordance with any particular implementation of the technology disclosed herein. Thus, the implementations disclosed herein can be implemented or carried out in a manner that achieves or optimizes one advantage or group of advantages as taught herein without necessarily achieving other advantages that can be taught or suggested herein.

### BRIEF DESCRIPTION OF THE DRAWINGS

Certain features of this disclosure are described below with reference to the drawings. The illustrated implementations are intended to illustrate, but not to limit, the implementations. Various features of the different disclosed implementations can be combined to form further implementations, which are part of this disclosure.
FIGS. 1A-1B illustrate perspective views of a system secured to a subject's foot in accordance with aspects of this disclosure.
FIG. 1C illustrates the system of FIGS. 1A-1B in wireless communication with an example computing device in accordance with aspects of this disclosure.
FIG. 1D illustrates a cross-sectional view of the system of FIGS. 1A-1B secured to a subject's foot in accordance with aspects of this disclosure.
FIGS. 2A-2C illustrate perspective views of the system of FIGS. 1A-1B in accordance with aspects of this disclosure.
FIG. 2D illustrates a perspective view of the system of FIGS. 1A-1B with a sensor hub detached from a sensor dock and a wearable device in accordance with aspects of this disclosure.
FIG. 2E illustrates a perspective view of the system of FIGS. 1A-1B with the sensor hub detached from the sensor dock and the sensor dock detached from the wearable device in accordance with aspects of this disclosure.
FIG. 3 illustrates a schematic diagram of certain optional features of the system of FIGS. 1A-1B in accordance with aspects of this disclosure.
FIGS. 4A-4G illustrate various perspective views of the sensor dock of the system of FIGS. 1A-1B in accordance with aspects of this disclosure.
FIGS. 5A-5B illustrate perspective views of the sensor hub of the system of FIGS. 1A-1B in accordance with aspects of this disclosure.
FIGS. 5C-5H illustrate bottom, top, side, side, front, and back views, respectively, of the sensor hub of FIGS. 5A-5B in accordance with aspects of this disclosure.
FIGS. 5I-5J illustrate exploded perspective views of the sensor hub of FIGS. 5A-5B in accordance with aspects of this disclosure.
FIGS. 6A-6D illustrate various perspective views of the wearable device of the system of FIGS. 1A-1B in accordance with aspects of this disclosure.
FIGS. 7A-7B illustrate perspective views of a charging station in accordance with aspects of this disclosure.
FIGS. 7C-7H illustrate top, bottom, front, back, side, and side views, respectively, of the charging station of FIGS. 7A-7B in accordance with aspects of this disclosure.
FIGS. 7I-7J illustrate enlarged top perspective views of a portion of the charging station of FIGS. 7A-7B in accordance with aspects of this disclosure.
FIG. 7K illustrates a perspective cross-sectional view taken through the charging station of FIGS. 7A-7B in accordance with aspects of this disclosure.
FIGS. 8A-8B illustrate perspective views of another implementation of a system secured to a subject's foot in accordance with aspects of this disclosure.
FIG. 8C illustrates a cross-sectional view of the system of FIGS. 8A-8B secured to a subject's foot in accordance with aspects of this disclosure.
FIGS. 9A-9C illustrate perspective views of the system of FIGS. 8A-8B in accordance with aspects of this disclosure.
FIG. 9D illustrates a perspective view of the system of FIGS. 8A-8B with a sensor hub detached from a sensor dock and a wearable device in accordance with aspects of this disclosure.
FIG. 9E illustrates a perspective view of the system of FIGS. 8A-8B with the sensor hub detached from the sensor dock and the sensor dock detached from the wearable device in accordance with aspects of this disclosure.
FIGS. 10A-10F illustrate various perspective views of the sensor dock of the system of FIGS. 8A-8B in accordance with aspects of this disclosure.
FIGS. 11A-11E illustrate various perspective views of the wearable device of the system of FIGS. 8A-8B in accordance with aspects of this disclosure.
FIGS. 11F-11G illustrate perspective views of connector portions of a wearable device strap and the wearable device of the system of FIGS. 8A-8B in accordance with aspects of this disclosure.
FIGS. 11H-11I illustrate perspective views of connector portions of a sensor strap of the system of FIGS. 8A-8B in accordance with aspects of this disclosure.
FIGS. 12A-12B illustrate perspective views of another implementation of a system secured to a subject's foot in accordance with aspects of this disclosure.
FIG. 12C illustrates a cross-sectional view of the system of FIGS. 12A-12B secured to a subject's foot in accordance with aspects of this disclosure.
FIGS. 13A-13C illustrate perspective views of the system of FIGS. 12A-12B in accordance with aspects of this disclosure.
FIG. 13D illustrates a perspective view of the system of FIGS. 12A-12B with a sensor component detached from a wearable device in accordance with aspects of this disclosure.
FIG. 13E illustrates a perspective view of the system of FIGS. 12A-12B with the sensor component detached from the wearable device in accordance with aspects of this disclosure.
FIGS. 14A-14G illustrate various perspective views of the sensor component of the system of FIGS. 12A-12B in accordance with aspects of this disclosure.
FIGS. 15A-15E illustrate various perspective views of the wearable device of the system of FIGS. 12A-12B in accordance with aspects of this disclosure.
FIGS. 16A-16B illustrate perspective views of another implementation of a system configured to be secured to a subject's foot in accordance with aspects of this disclosure.
FIGS. 16C-16D illustrate perspective views of the system of FIGS. 16A-16B with a sensor component detached from a wearable device in accordance with aspects of this disclosure.
FIGS. 17A-17D illustrate various views of the sensor component (and portions thereof) of the system of FIGS. 16A-16B in accordance with aspects of this disclosure.
FIGS. 18A-18D illustrate various perspective views of the wearable device of the system of FIGS. 16A-16B in accordance with aspects of this disclosure.
FIG. 19A illustrates an example monitoring system in accordance with aspects of this disclosure.
FIG. 19B illustrates a schematic representation of the monitoring system of FIG. 19A in accordance with aspects of this disclosure.
FIG. 20 illustrates a schematic diagram of certain optional features of an aspect of the monitoring system of FIGS. 19A-19B in accordance with aspects of this disclosure.
FIGS. 21A-21B illustrate various perspective views of a camera of the monitoring system of FIG. 19A in accordance with aspects of this disclosure.
FIG. 21C illustrates a schematic diagram of certain optional features of the camera of FIGS. 21A-21B in accordance with aspects of this disclosure.
FIGS. 22A-22C illustrate various perspective views of a hub of the monitoring system of FIG. 19A in accordance with aspects of this disclosure.
FIG. 22D illustrates a schematic diagram of certain optional features of the hub of FIGS. 22A-22C in accordance with aspects of this disclosure.

### DETAILED DESCRIPTION

Various features and advantages of this disclosure will now be described with reference to the accompanying figures. The following description is merely illustrative in nature and is in no way intended to limit the disclosure, its application, or uses. This disclosure extends beyond the specifically disclosed implementations and/or uses and obvious modifications and equivalents thereof. Thus, it is intended that the scope of this disclosure should not be limited by any particular implementations described below. The features of the illustrated implementations can be modified, combined, removed, and/or substituted as will be apparent to those of ordinary skill in the art upon consideration of the principles disclosed herein. Furthermore, implementations disclosed herein can include several novel features, no single one of which is solely responsible for its desirable attributes or which is essential to practicing the systems, devices, and/or methods disclosed herein.

Disclosed herein are systems that can be used to measure, monitor, transmit (for example, wirelessly or via wired connection), process, and/or determine one or more physiological parameters (which may also be referred to herein as "physiological data"), motion data, and/or location data of a subject (which may also be referred to herein as a "user", "patient", or "wearer"). The disclosed systems can generate one or more signals associated with and/or indicative of one or more physiological parameters, motion data, and/or location data of a subject and process such one or more signals to determine such physiological parameters, motion data, and/or location data. Some implementations of the disclosed systems generate and transmit one or more signals associated with and/or indicative of one or more physiological parameters, motion data, and/or location data of a subject to a separate monitoring and/or computing device (wirelessly or via wired connection), for example, a patient monitor, which is capable of processing and/or determining such physiological parameters, motion data, and/or location data based on the transmitted signals. The systems disclosed herein can measure, monitor, transmit, process, and/or determine such physiological parameters, motion data, and/or location data continuously or intermittently. Any of the disclosed systems and/or devices in communication with the disclosed systems can include hardware and/or software capable of determining and/or monitoring a variety of physiological parameters, including but not limited to blood oxygenation levels in veins and/or arteries, heart rate, blood flow, respiratory rates, body temperature, and/or other physiological parameters or characteristics such as those discussed herein. Any of the systems described herein can include and/or employ pulse oximetry (for example, via an optical sensor) to measure physiological parameters of the subject and/or to generate, transmit, and/or process one or more signals associated with and/or indicative of such physiological parameters and/or to determine such physiological parameters. As discussed below, such optical sensor can include one or more emitters configured to emit optical radiation (e.g., light) of one or more wavelengths (e.g., wavelength(s) in the visible spectrum, near infrared wavelength(s), infrared wavelength(s), far infrared wavelength(s), etc.) and one or more detectors configured to detect at least a portion of the emitted optical radiation after attenuation and/or after passing through tissue of the subject.

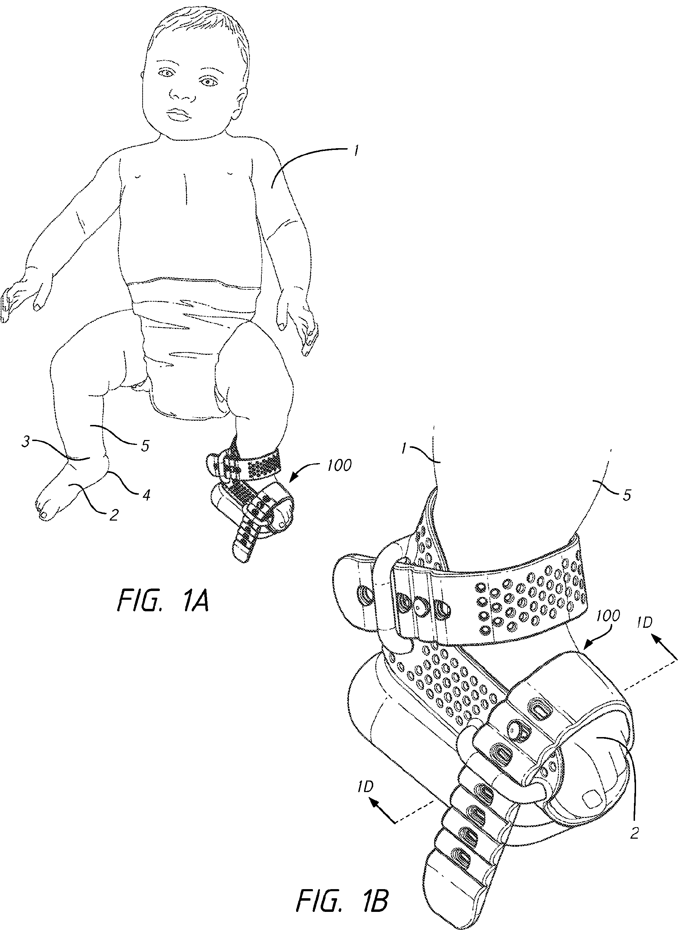

FIGS. 1A-1B illustrate perspective views of a system 100 (which can also be referred to herein as a "wearable system," "wearable sensor system," or "wearable physiological sensor system") secured to a foot 2 of a subject 1. As shown, in securing to the subject's foot 2, the system 100 can also be secured to an ankle 3, a heel 4, and/or a lower leg 5 of the subject 1. Further as shown, when secured to the subject's foot 2, the system 100 can support the subject's foot 2, ankle 3, heel 4, and/or lower leg 5. The system 100 can include a wearable device, a sensor dock, and a sensor hub, all of which are discussed further below.

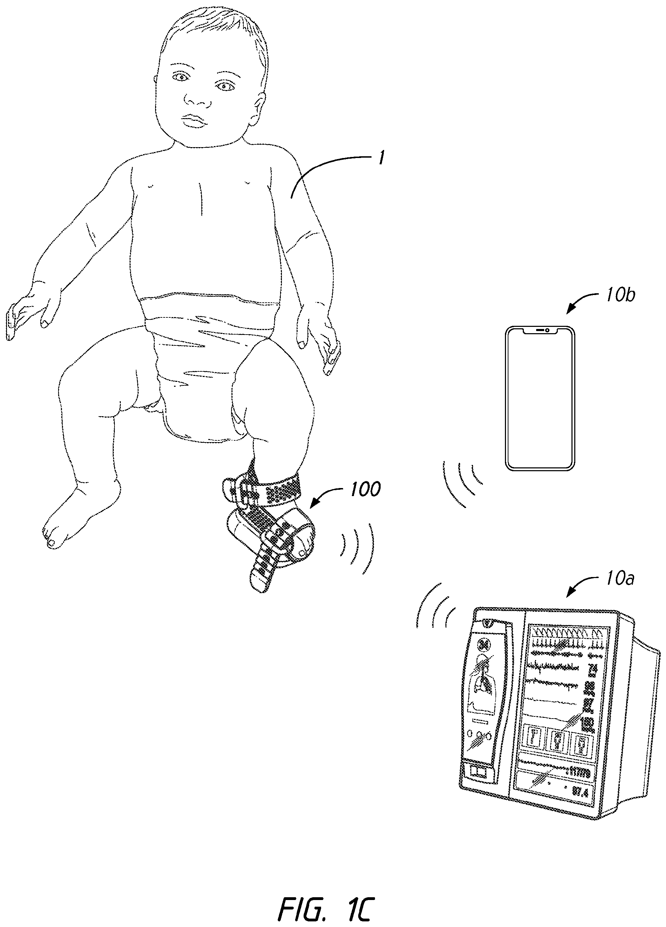

Although FIGS. 1A-1B show the system 100 secured to a foot 2 of the subject 1 in a particular manner which can provide certain advantages as described herein, such illustrated manner and/or location of securement is not intended to be limiting. System 100 can be secured to various portions of the subject's foot 2, ankle 3, heel 4, and/or lower leg 5 in a variety of manners and/or using a variety of methods. Accordingly, while system 100 is described herein primarily with reference to a foot 2, ankle 3, heel 4, and/or lower leg 5 of the subject 1, such description is not intended to be limiting. Further, while system 100 is shown secured to a left foot of the subject 1, the system 100 can be secured to either a left or a right foot of the subject 1. FIG. 1C illustrates the system 100 of FIGS. 1A-1B secured to the subject 1 and wirelessly communicating with one or more separate computing device(s), which can be for example, a patient monitor 10a (which can also be referred to herein as an "external patient monitor") and/or a mobile phone 10b as shown, via any of a variety of wireless communication protocols (such as any of those discussed herein). The system 100 can wirelessly transmit subject physiological data, motion data, and/or location data to the separate computing device 10 (e.g., 10a, 10b, or others) as described further herein.

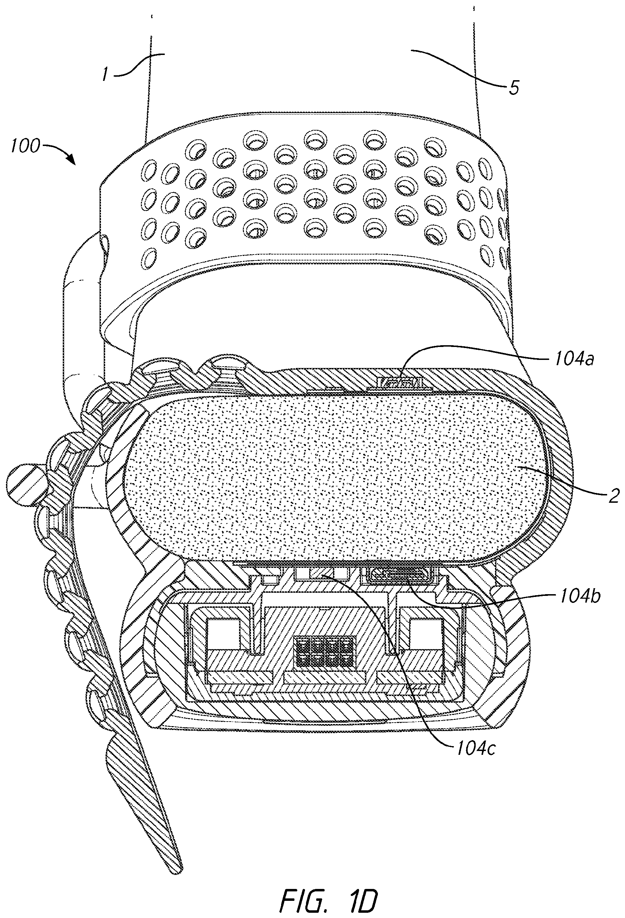

FIG. 1D illustrates a cross-section of the system of FIGS. 1A-1B secured to the subject's foot 2. As shown, when secured to the subject's foot 2, the system 100 can operably position one or more emitters 104a and one or more detectors 104b at opposite sides of the subject's foot 2. Also shown, when secured to the subject's foot 2, the system 100 can operably position one or more temperature sensors 104c adjacent a bottom of the subject's foot 2.

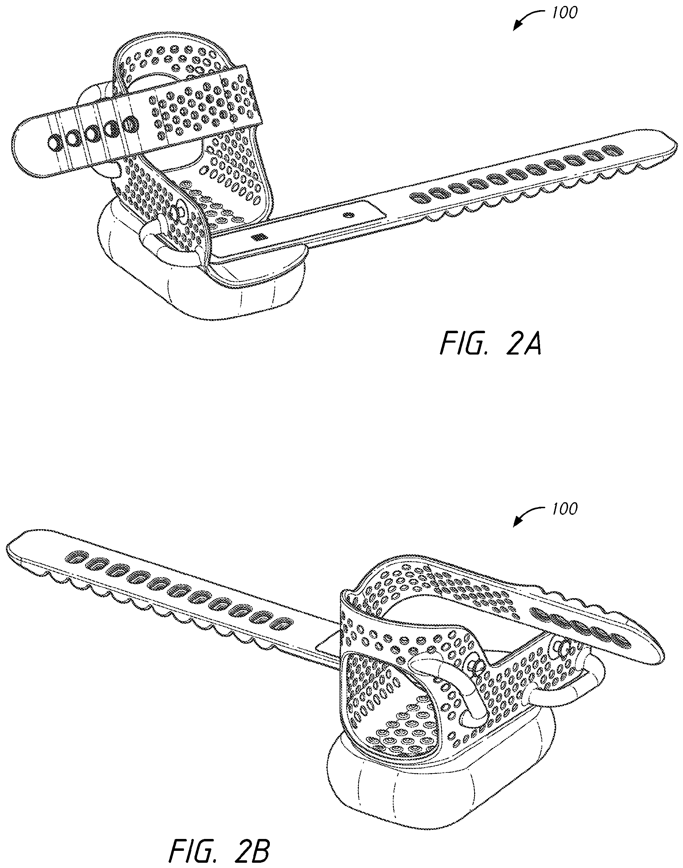

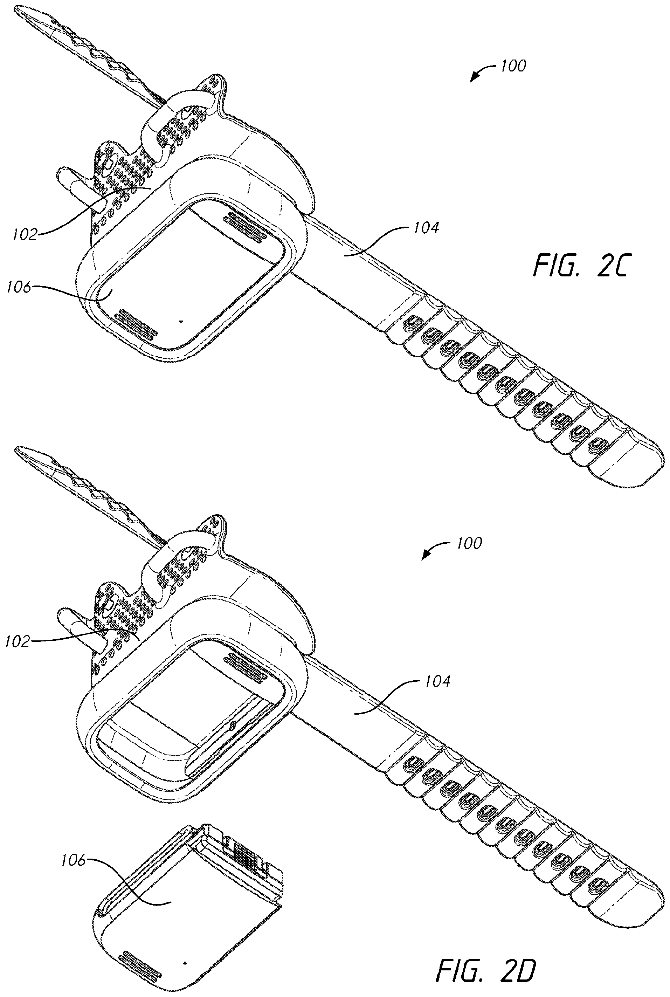

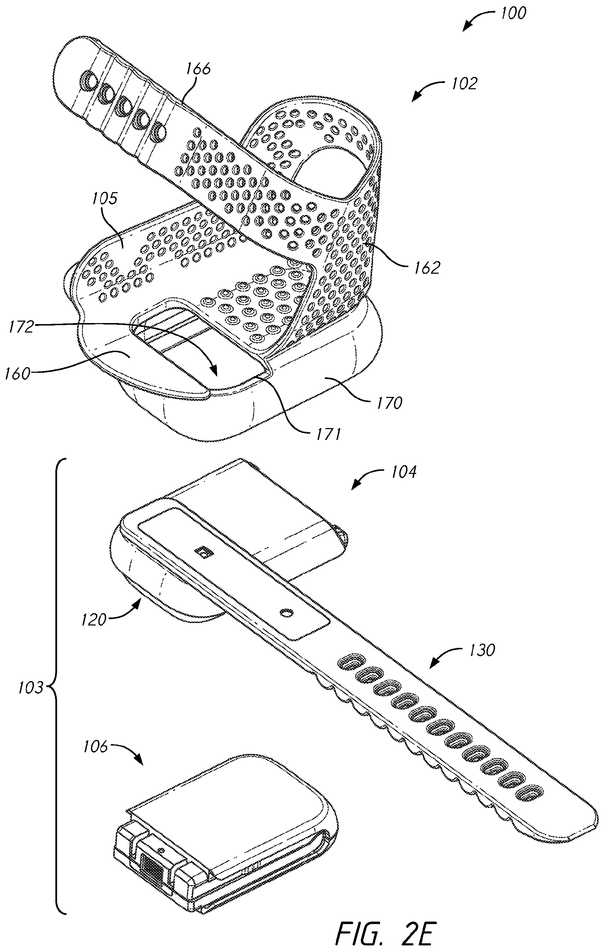

FIGS. 2A-2E illustrate various perspective views of the system 100 of FIG. 1A. The system 100 can include a wearable device 102. The wearable device 102 can be configured to receive and/or secure an electronic device including one or more sensors for monitoring information relating to physiological, motion, and/or location of the subject 1. For example, the wearable device can be configured to receive and/or secure a sensor component 103 (which may also be referred to herein as a "sensor assembly") or a portion thereof, as described further herein. Such sensor component 103 can include a sensor dock 104 and a sensor hub 106. In some implementations, the system 100 can include the wearable device 102, the sensor dock 104, and the sensor hub 106. As shown in FIGS. 2A-2C, the wearable device 102, the sensor dock 104, and the sensor hub 106 can form a unitary structure configured to be secured to the subject's foot. FIG. 2D illustrates the wearable device 102 and sensor dock 104 connected to one another and the sensor hub 106 disconnected from the wearable device 102 and sensor dock 104. FIG. 2E illustrates an exploded view of system 100, illustrating the wearable device 102, sensor dock 104, and sensor hub 106 separated from one another. Although the figures illustrate implementations of the system 100 in which the wearable device 102, sensor dock 104, and sensor hub 106 are removably connectable to one another, various ones of these components may be integrally formed with one another. For example, in some variants, the wearable device 102 and sensor dock 104 are integrally formed and are removably connectable to the sensor hub 106. As another example, in some variants, the sensor dock 104 and sensor hub 106 are integrally formed and are removably connectable to the wearable device 102. As another example, in some variants, the wearable device 102, sensor dock 104, and sensor hub 106 are integrally formed with one another. Implementations of the system 100 in which wearable device 102 is removably connectable from sensor dock 104 and/or sensor hub 106 can advantageously allow for a wearable device 102 of various sizes (e.g., small, medium, and large) and/or shapes to be utilized with the system 100, for example, so as to accommodate various sizes and/or shapes of a subject's foot 2, ankle 3, heel 4, and/or lower leg 5. In this way, the system 100 can be customized to a subject 1 by selecting an appropriately configured wearable device 102 while allowing for all other aspects of the system 100, such as the sensor dock 104 and sensor hub 106, to remain the same and/or be universal across subjects. In some implementations the sensor dock 104 and the sensor hub 106 can advantageously be configured to removably connect from each other (e.g., so that the sensor hub 106 can be recharged separate of the sensor dock 104). In some implementations, for example as shown in FIG. 2E, the sensor dock 104 and the sensor hub 106 form the sensor component 103 that can be removably connected to the wearable device 102.

As mentioned above, FIG. 2E illustrates an exploded view of system 100. The wearable device 102 can have a base 160 and a wall 162. The wall 162 can extend from the base 160. For example, the wall 162 can extend from a periphery of the base 160. In some implementations, the wall 162 can extend around a portion of a perimeter edge of the base 160. The base 160 and the wall 162 can form a main body 105 of the wearable device 102. In various places in the present disclosure, the "base" and "wall" may be referred to as being part of the "main body" for ease of reference. However, this is not intended to be limiting nor to require that the "wearable device" requires a "main body". In some implementations, the wearable device 102 can have a main body 105 and a holder 170 extending outward from the main body 105. The main body 105 can include the base 160, an opening 171 in the base 160, and the wall 162 extending from the base 160. In some implementations, the main body 105 additionally includes a wearable device strap 166 (which can also be referred to herein as an "additional strap") extending from the wall 162. The base 160 (which may also be referred to herein as "bottom portion") of the wearable device 102 can be configured to contact a bottom portion of the subject's foot 2 when the system 100 is in use. For example, the base 160 can be configured to contact a heel, an arch, a ball, and/or one or more toes of the subject's foot 2. The opening 171 in the base 160 can be configured to be positioned adjacent a bottom portion of the subject's foot 2 when the system 100 is in use. For example and as shown, the opening 171 can extend through the base 160 and be positioned such that it underlies the ball of the subject's foot 2 when the wearable device is secured to the subject's foot 2. The holder 170 extending outward from the main body 105 can, as shown, extend from the main body 105 adjacent the opening 171 of the base 160 and away from the bottom portion of the subject's foot 2 when the system 100 is in use. The holder 170 can include a cavity 172 configured to removably receive the sensor dock 104 and the sensor hub 106, for example, when the sensor hub 106 is connected to the sensor dock 104. Further, the opening 171 can open into the cavity 172 of the holder 170 as shown. The sensor dock 104 can have a main body 120 and a sensor strap 130 (also referred to herein as "strap") connected to and extending from the main body 120. The sensor strap 130 of the sensor dock 104 can operably position one or more emitters 104a and one or more detectors 104b of the system 100 and can be configured to be positioned at least partially within and extend outward from the opening 171 when the sensor dock 104 is connected to the holder 170. The above and other aspects of the system 100 are discussed further below.

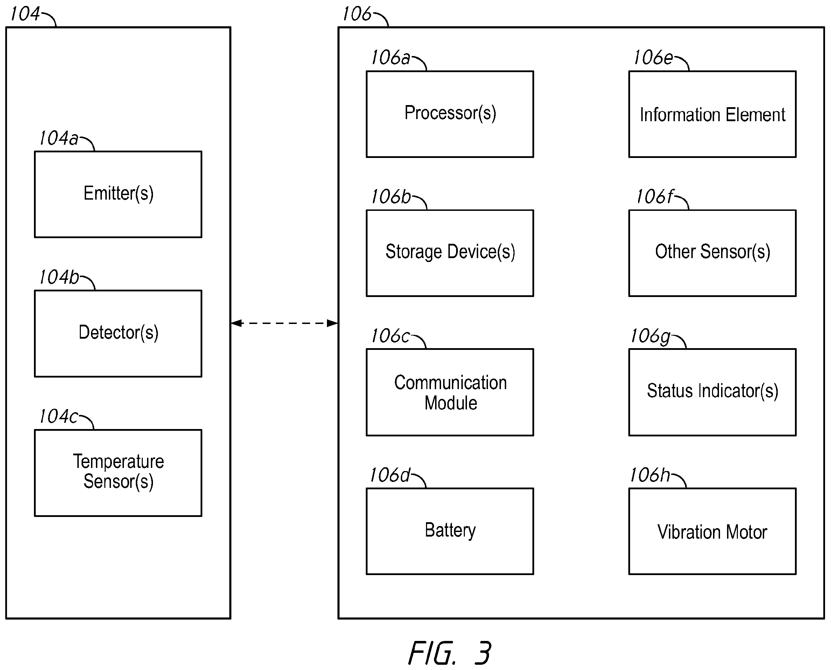

FIG. 3 illustrates a schematic diagram of certain features which can be incorporated in the system 100 as well as any other implementations of systems described herein. FIG. 3 schematically illustrates sensor dock 104 and sensor hub 106. As shown, the sensor dock 104 can include one or more emitters 104a, one or more detectors 104b, and one or more temperature sensors 104c. Also shown, the sensor hub 106 can include one or more processors 106a, one or more storage devices 106b, a communication module 106c, a battery 106d, an information element 106e, one or more other sensors 106f, one or more status indicators 106g, and/or a vibration motor 106h.

The one or more emitters 104a and the one or more detectors 104b of the system 100 can be utilized to obtain physiological information indicative of one or more physiological parameters of the subject. These parameters can include various blood analytes such as oxygen, carbon monoxide, methemoglobin, total hemoglobin, glucose, proteins, glucose, lipids, a percentage thereof (for example, concentration or saturation), and the like. The one or more emitters 104a and the one or more detectors 104b of the system 100 can also be used to obtain a photoplethysmograph, a measure of plethysmograph variability, pulse rate, a measure of blood perfusion, and the like. Information such as oxygen saturation (SpO₂), pulse rate, a plethysmograph waveform, respiratory effort index (REI), acoustic respiration rate (RRa), EEG, ECG, pulse arrival time (PAT), perfusion index (PI), pleth variability index (PVI), methemoglobin (MetHb), carboxyhemoglobin (CoHb), total hemoglobin (tHb), and/or glucose, can be obtained from the system 100 and data related to such information can be processed and/or transmitted by the system 100 (for example, via communication module 106c) to a separate computing device 10 (such as a computing device at a caregiver's workstation, a patient monitor, and/or a mobile phone). The one or more emitters 104a and the one or more detectors 104b can be optically based and, for example, utilize optical radiation. Further, the one or more emitters 104a can serve as a source of optical radiation that can be directed towards tissue of the subject 1 when the system 100 is in use. The system 100 can include one, two, three, four, five, six, seven, or eight or more emitters 104a and/or one, two, three, four, five, six, seven, or eight or more detectors 104b. The one or more emitters 104a can be one or more light-emitting diodes (LEDs) (for example, such as low-power, high-brightness LEDs), laser diodes, incandescent bulbs with appropriate frequency-selective filters, and/or any other source(s) of optical radiation and/or any combinations of the same, or the like. The one or more emitters 104a can emit optical radiation of one or more wavelengths and can emit visible and near-infrared optical radiation. The one or more detectors 104b can be configured to detect optical radiation generated by the one or more emitters 104a. The one or more detectors 104b can detect optical radiation that attenuates through and/or is reflected by tissue of the subject 1, for example, tissue of the subject's foot 2. The one or more detectors 104b can output one or more signals responsive to the detected optical radiation. In some implementations, the one or more detectors 104b can be one or more photodiodes, phototransistors, or the like.

The one or more processors 106a can be configured, among other things, to process data, execute instructions to perform one or more functions, and/or control the operation of the system 100. For example, the one or more processors 106a can control operation of the one or more emitters 104a, the one or more detectors 104b, the one or more temperature sensors 104c, and/or the one or more other sensors 106f of the system 100. As another example, the one or more processors 106a can process signals and/or physiological data received and/or obtained from the one or more detectors 104b, the one or more temperature sensors 104c, and/or the one or more other sensors 106f of the system 100. Further, the one or more processors 106a can execute instructions to perform functions related to storing and/or transmitting such signals and/or physiological data received and/or obtained from the one or more detectors 104b and/or the one or more other sensors 106f of the system 100. The processor 106a can execute instructions to perform functions related to storing and/or transmitting any or all of such received data.

The one or more storage devices 106b can include one or more memory devices that store data, including without limitation, dynamic and/or static random access memory (RAM), programmable read-only memory (PROM), erasable programmable read-only memory (EPROM), electrically erasable programmable read-only memory (EEPROM), and the like. Such stored data can be processed and/or unprocessed physiological data obtained from the system 100, for example.

The communication module 106c can facilitate communication (via wires and/or wireless connection) between the system 100 (and/or components thereof) and separate devices, such as separate monitoring, computing, electrical, and/or mobile devices, such as patient monitor 10a and/or mobile phone 10b shown in FIG. 1C. For example, the communication module 106c can be configured to allow the system 100 to wirelessly communicate with other devices, systems, and/or networks over any of a variety of communication protocols. The communication module 106c can be configured to use any of a variety of wireless communication protocols, such as Wi-Fi (802.11x), Bluetooth^{®}, ZigBee^{®}, Z-wave^{®}, cellular telephony, infrared, near-field communications (NFC), RFID, satellite transmission, proprietary protocols, combinations of the same, and the like. The communication module 106c can allow data and/or instructions to be transmitted and/or received to and/or from the system 100 and separate computing devices. The communication module 106c can be configured to transmit (for example, wirelessly) processed and/or unprocessed physiological parameters, data and/or other information to one or more separate computing devices, which can include, among others, a patient monitor, a mobile device (for example, an iOS or Android enabled smartphone, tablet, laptop), a desktop computer, a server or other computing or processing device for display and/or further processing, among other things. Such separate computing devices can be configured to store and/or further process the received physiological parameters, data, and/or other information, to display information indicative of or derived from the received parameters, data, and/or information, and/or to transmit information -- including displays, alarms, alerts, and notifications -- to various other types of computing devices and/or systems that can be associated with a hospital, a caregiver (for example, a primary care provider), and/or a user (for example, an employer, a school, friends, family) that have permission to access the subject's data. As another example, the communication module 106c of the system 100 can be configured to wirelessly transmit processed and/or unprocessed obtained physiological parameters, data, information and/or other information (for example, motion and/or location data) to a mobile phone which can include one or more processors configured to execute an application that generates a graphical user interface displaying information representative of the processed or unprocessed physiological parameters, data, information and/or other information obtained from the system 100. The communication module 106c can be and/or include a wireless transceiver. The communication module 106c can be embodied in an antenna and/or an NFC chip.

The battery 106d can provide power for hardware components of the system 100 described herein. The battery 106d can be rechargeable. For example, the battery 106d can be a lithium, a lithium polymer, a lithium-ion, a lithium-ion polymer, a lead-acid, a nickel-cadmium, or a nickel-metal hydride battery. In some implementations, the battery 106d can be non-rechargeable. Additionally or alternatively, the system 100 can be configured to obtain power from a power source that is external to the system 100. For example, the system 100 can include or can be configured to connect to a cable which can itself connect to an external power source to provide power to the system 100.

The information element 106e can be a memory storage element that stores, in non-volatile memory, information used to help maintain a standard of quality associated with the system 100. Illustratively, the information element 106e can store information regarding whether the system 100 has been previously activated and whether the system 100 has been previously operational for a prolonged period of time, such as, for example, four hours, one day, two days, five days, ten days, twenty days, a month, multiple months, or any period of time. The information stored in the information element 106e can be used to help detect improper re-use of the system 100, for example.

**In** some implementations, the system 100 can include one or more other sensor(s) 106f. The other sensor(s) 106f can comprise a motion sensor, for example, including one or more accelerometers and/or gyroscopes, that can be utilized to determine motion of the subject and/or a portion of the subject's body (for example, foot 2, ankle 3, heel 4, and/or lower leg 5). In some implementations where the system 100 (for example, sensor hub 106) includes a motion sensor, the processor(s) 106a can determine whether the subject's foot 2, ankle 3, heel 4, and/or lower leg 5 are moving and, responsive to such determination, not receive, not process, and/or not determine one or more physiological parameters (since such determinations can include inaccuracies because of such movement). The other sensor(s) 106f can be disposed on, within, and/or be operably positioned by any one or more of the aspects of the system 100. For example, the other sensor(s) can be disposed on, within, and/or be operably positioned by any one or more of the wearable device 102, the sensor dock 104, and/or the sensor hub 106. The other sensor(s) 106f can be operably connected to the one or more processors 106a, which can control operation of the other sensor(s) 106f and/or process data received from the other sensor(s) 106f.

The one or more status indicators 106g can be configured to provide and/or indicate a status of the system 100 and/or a status of one or more physiological parameters of the subject 1 determined by the system 100 and/or any devices in communication with the system 100. In some implementations, the one or more status indicators 106g can be configured to indicate a status of the system 100, such as whether the system 100 is in an operational ("on") mode, whether the system 100 is pairing or has paired with a separate device, whether an error has been detected, and/or a power level of the system 100 (for example, a charge of battery 106d of sensor hub 106). For example, the one or more status indicators 106g can be configured to light up and/or cast optical radiation of one or more wavelengths from one or more portions of the system 100. As another example, the one or more status indicators 106g can be configured to light up and/or emit optical radiation from one or more portions of the sensor hub 106 of the system 100. The one or more processors 106a can be in communication with the one or more status indicators 106g and can be configured to instruct the one or more status indicators 106g to cause any of such above-described status indications and/or lighting.

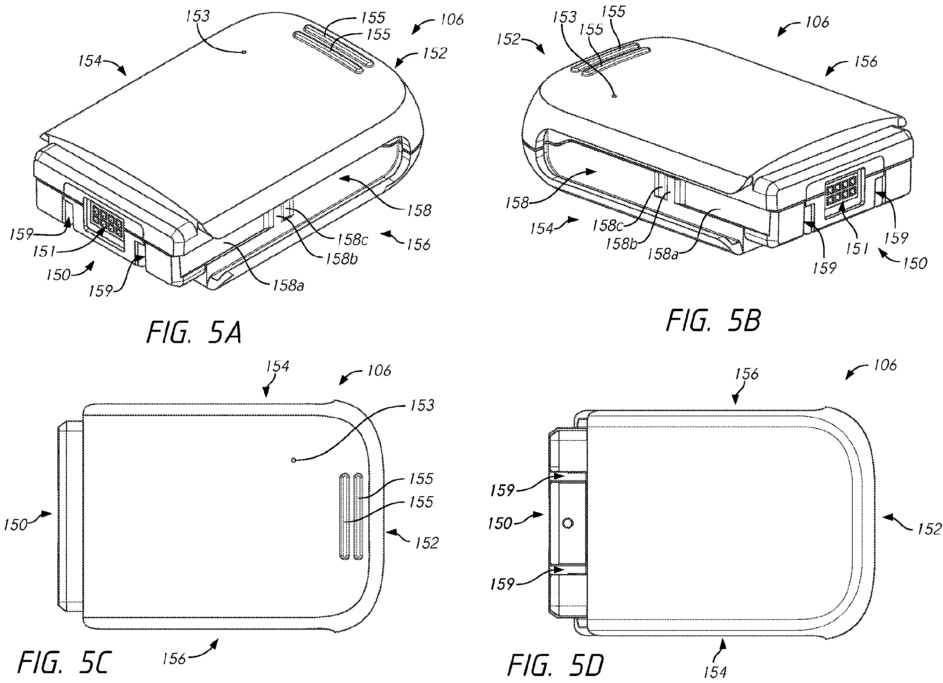

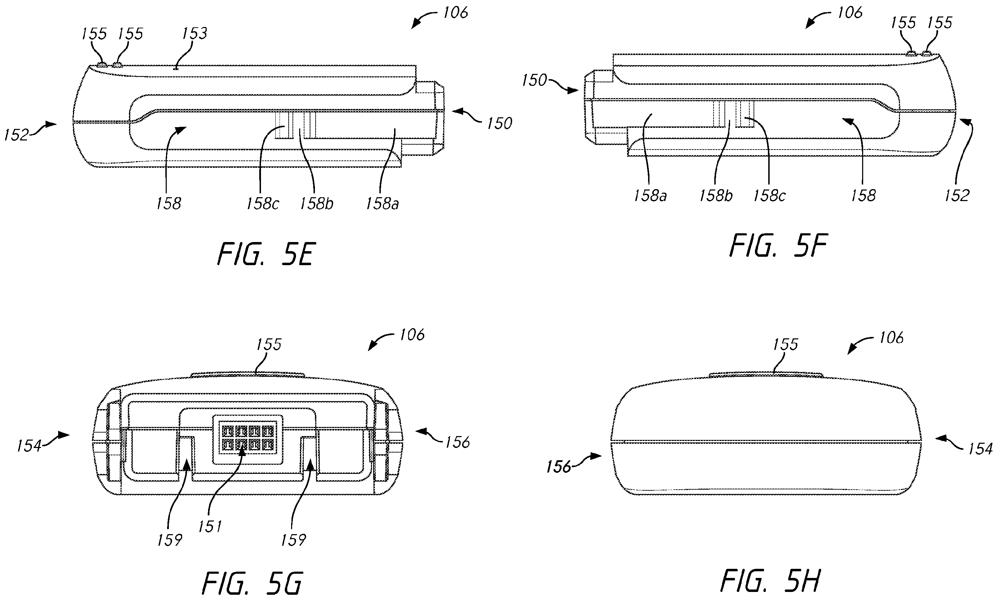

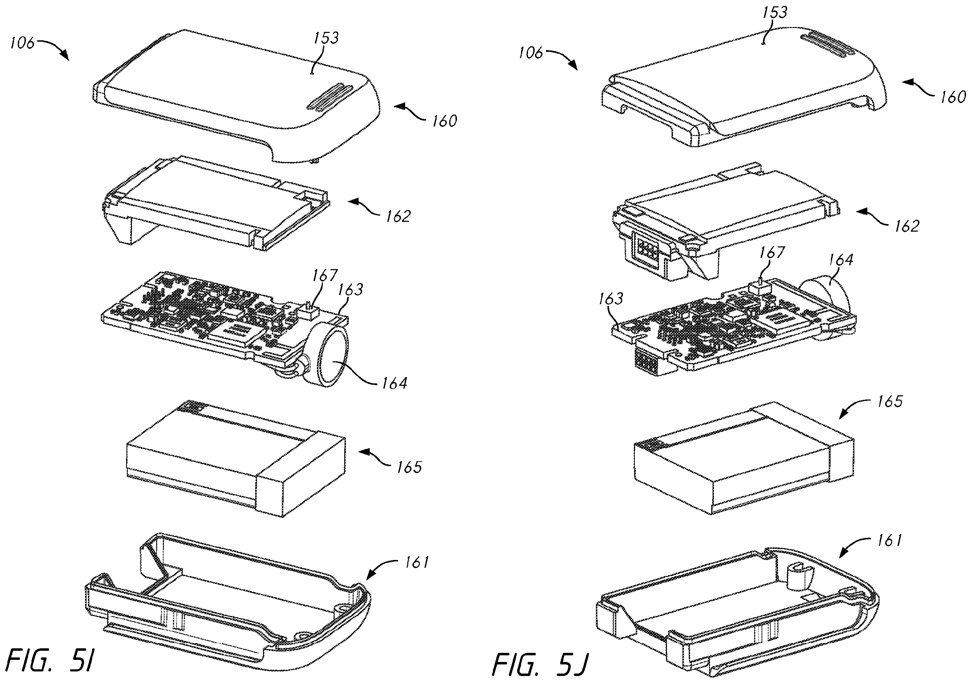

In some cases, the one or more status indicators 106g can be configured to provide optical radiation (e.g., light) feedback to the subject when the system 100 is secured to the subject and/or when sensor hub 106 and sensor dock 104 are connected together. In some implementations, system 100 can be configured to cause optical radiation feedback to the subject 1 (when the system 100 is secured to the subject) responsive to one or more physiological parameters determined by system 100 and/or by any devices (such as separate computing and/or mobile devices, for example, a patient monitor) in communication with the system 100. The one or more processors 106a can instruct the one or more status indicators 106g to emit or stop emitting optical radiation and/or instruct the one or more status indicators 106g to alter a characteristic of optical radiation (for example, increase/reduce optical radiation brightness, change optical radiation wavelength and/or color, change a rate of blinking of optical radiation, etc.) responsive to the one or more determined physiological parameters. Such action by the one or more processors 106a can dynamically track with physiological parameter determination over time, for example. As an example, in some implementations, the one or more processors 106a can provide instructions to the one or more status indicators 106g (such as those discussed above) responsive to a condition of the subject using the system 100. For example, if one or more physiological parameters determined by the system 100 and/or any devices in communication with the system 100 are indicative of hypoxemia (low blood oxygen) when the subject is using the system 100, the one or more processors 106a can instruct the one or more status indicators 106g to produce optical radiation to notify the subject and/or their care providers to restore proper breathing and/or safe blood oxygen levels. As another example, if one or more physiological parameters determined by the system 100 and/or any devices in communication with the system 100 are indicative of edema (swelling caused by excess fluid trapped in body tissue) when the subject is using the system 100, the one or more processors 106a can instruct the one or more status indicators 106g to cause optical radiation to be emitted from the system 100 as described above. In some implementations, the one or more processors 106a and/or any devices in communication with the system 100 can instruct the one or more status indicators 106g to cause optical radiation to be emitted if a determined subject physiological parameter of interest meets and/or exceeds a set threshold, meets and/or falls below a set threshold, and/or meets, exceeds, and/or falls below a set range. In some cases, optical radiation emitted from the one or more status indicators 106g can correspond to an alert, an alarm, a notification, and/or any other situation wherein the subject and/or a care provider may need to intervene in the subject's care. The one or more status indicators 106g can be positioned within various portions of the system 100, for example, within sensor hub 106, such that optical radiation emitted from the one or more status indicators emit out of and/or through a hole and/or opening in the sensor hub 106, such as by status indicator 167 shown in and described with respect to FIGS. 5I and 5J through hole 153 of the sensor hub 106 shown and described with respect to FIGS. 5A, 5B, and 5C.

The vibration motor 106h can be configured to vibrate one or more portions of the system 100 (for example, the wearable device 102, the sensor hub 106 and/or the sensor dock 104 when sensor hub 106 and sensor dock 104 are coupled together), which in turn can vibrate one or more portions of a subject's body (for example, foot) when the system 100 is secured to the subject. For example, vibration motor 106h can be configured to vibrate the sensor hub 106 or portions thereof. The one or more processors 106a can be in communication with vibration motor 106h and can be configured to instruct vibration motor 106h to cause any of such above-described vibration.

In some cases, the vibration motor 106h can be utilized to provide haptic feedback to the subject when the system 100 is secured to the subject. In some implementations, the system 100 can be configured to cause vibration of and/or provide haptic feedback to one or more portions of the subject's body (when the system 100 is secured to the subject) via the vibration motor 106h responsive to one or more physiological parameters determined by system 100 and/or by any devices (such as separate computing, electrical, and/or mobile devices, for example, a patient monitor 10) in communication with the system 100. The one or more processors 106a can instruct the vibration motor 106h to cause vibration, cease vibrating, and/or instruct the vibration motor 106h to alter a characteristic of vibration (for example, increase/reduce vibration rate, increase/reduce vibration strength, change vibration pattern, etc.) responsive to the one or more determined physiological parameters. Such action by the one or more processors 106a can dynamically track with physiological parameter determination over time, for example. As an example, in some implementations, the one or more processors 106a can provide instructions to vibration motor 106h (such as those discussed above) responsive to a condition of the subject using the system 100. For example, if one or more physiological parameters determined by the system 100 and/or any devices in communication with the system 100 are indicative of hypoxemia (low blood oxygen) when the subject is using the system 100, the one or more processors 106a can instruct the vibration motor 106h to vibrate to cause the subject to wake up in an attempt to restore proper breathing and/or safe blood oxygen levels. This can be significantly beneficial when the system 100 is worn by an infant or young child where continuous or intermittent monitoring is important. As another example, if one or more physiological parameters determined by the system 100 and/or any devices in communication with the system 100 are indicative of edema (swelling caused by excess fluid trapped in body tissue) when the subject is using the system 100, the one or more processors 106a can instruct the vibration motor 106h to cause vibration of a portion of the subject's body, such as their foot, ankle, heel, lower leg, and/or any other portion of the subject's body. In some implementations, the one or more processors 106a and/or any devices in communication with the system 100 can instruct the vibration motor 106h to cause a vibration if a determined subject physiological parameter of interest meets and/or exceeds a set threshold, meets and/or falls below a set threshold, and/or meets, exceeds, and/or falls below a set range. In some cases, a vibration of the vibration motor 106h can correspond to an alert, an alarm, a notification, and/or any other situation wherein the subject and/or a care provider can need to intervene in the subject's care. In some implementations, the one or more processors 106a can instruct the vibration motor 106h to vibrate responsive to a status of battery 106d (for example, when a charge of the battery 106d drops below a certain threshold). In some implementations, system 100 can include more than one vibration motor 106h, for example, two, or three or more vibration motors 106h. Vibration motor(s) 106h can be positioned within various portions of the system 100, for example, within sensor hub 106.

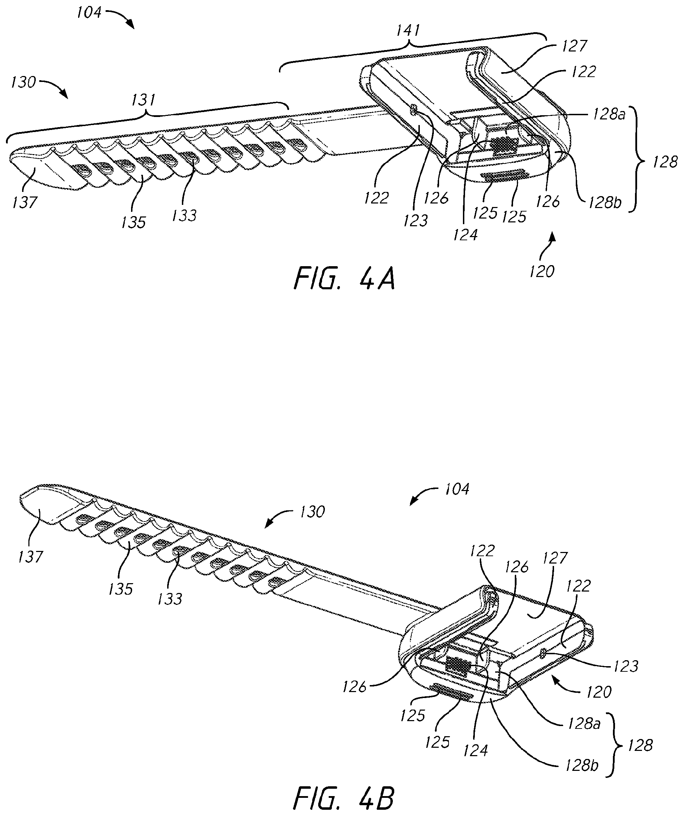

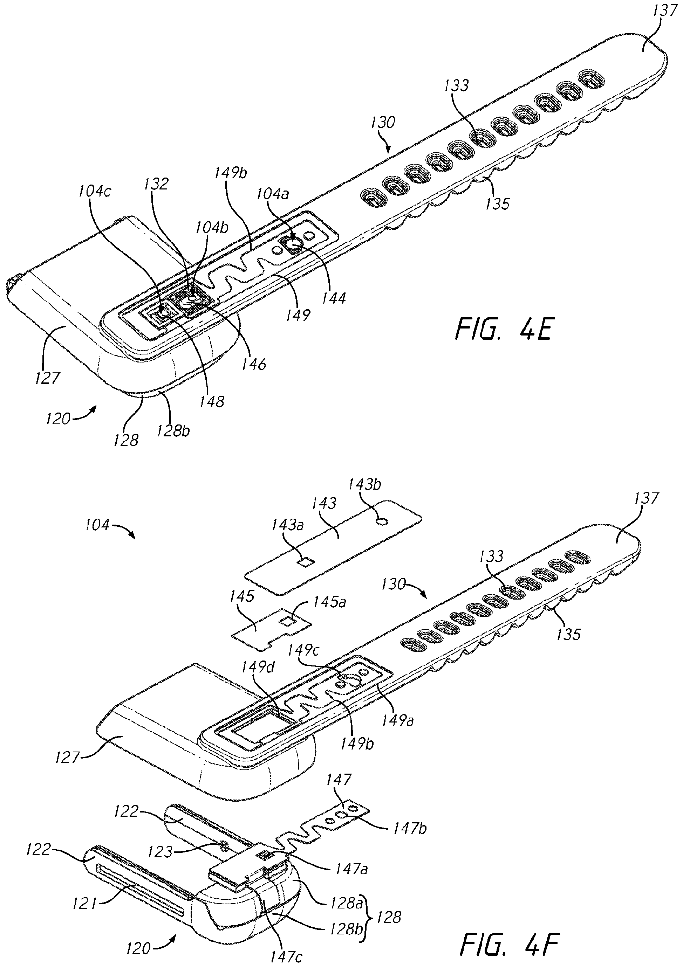

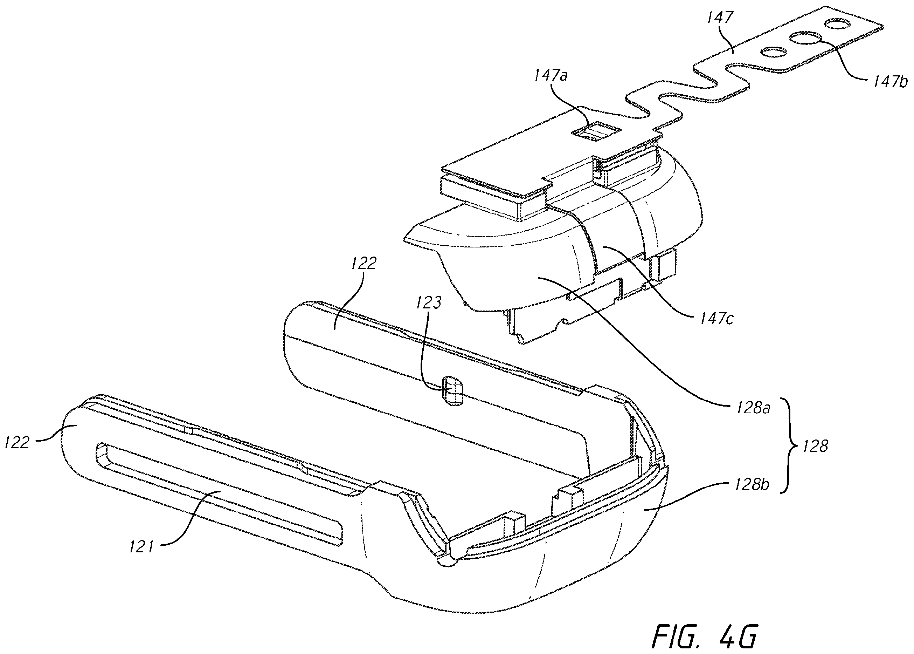

FIGS. 4A-4G illustrate various perspective views of the sensor dock 104 of the system 100. As shown, the sensor dock 104 can have a main body 120 and a sensor strap 130 (which also may be referred to herein as "strap") connected to and extending outward from the main body 120. The main body 120 can include a base 128 and arm(s) 122 extending outward from the base 128. In some implementations, the main body 120 further includes a shell 127 which is described further below. The sensor strap 130 can include the one or more emitters 104a and the one or more detectors 104b and can be configured to secure the system 100 to the subject's foot 2 as described further herein (for example, alone or in combination with wearable device strap 166 of wearable device 102). In some implementations, the system 100 includes an emitter package 144 (shown in FIG. 4E) comprising the one or more emitters 104a. Similarly, in some implementations the system 100 includes a detector package 146 (shown in FIG. 4E) comprising the one or more detectors 104b. The sensor strap 130 can be configured to receive the emitter package 144 to operably position the one or more emitters 104a. Similarly, the sensor strap 130 can be configured to receive the detector package 146 to operably position the one or more detectors 104b. The one or more emitters 104a in emitter package 144 and the one or more detectors 104b in detector package 146 can be in electrical communication with an electrical connector 124 of the sensor dock 104 via a circuit layer 147 disposed within the sensor strap 130 and a portion 147c of the circuit layer 147 that extends from the circuit layer 147 in the sensor strap 130 to the electrical connector 124 (shown in FIG. 4F).

The electrical connector 124 can be configured to releasably electrically connect the sensor dock 104 (and therefore the one or more emitters 104a and the one or more detectors 104b) to the sensor hub 106. The sensor dock 104 can also include features for mechanically engaging/connecting with the sensor hub 106. For example and as shown, the arm(s) 122 extending from the base 128 can be configured to releasably mechanically engage/connect with the sensor hub 106. In some implementations, when the sensor hub 106 is mechanically engaged/connected with the arm(s) 122 of the sensor dock 104, an electrical connector of the sensor hub 106 (for example, electrical connector 151 shown in FIGS. 5A-5B) can releasably mechanically and electrically engage/connect with the electrical connector 124 of the sensor dock 104.

As shown in FIGS. 4A-4G, in some implementations the main body 120 of the sensor dock 104 has a length and/or a width that are greater than a height of the sensor dock 104. In some implementations, the sensor dock 104 includes two arm(s) 122. The arm(s) 122 of the sensor dock 104 can extend from the base 128 in the same direction so as to form a generally U-shaped structure. The arm(s) 122 can be generally parallel to each other, such that a gap is formed between the arm(s) 122. Such a gap can be, for example, sized to accommodate the sensor hub 106 and/or at least a portion of the sensor hub 106. In some implementations, the arm(s) 122 are the same length. Furthermore, the arm(s) 122 can mirror each other in size, shape, and other features. In some implementations, the sensor dock 104 can include one or more retaining features configured to engage the sensor hub 106. For example, each of the arm(s) 122 of the sensor dock 104 can include a protrusion 123 configured to engage with the sensor hub 106 to allow the sensor dock 104 to connect to the sensor hub 106. The protrusion(s) 123 can be disposed along an inner surface of each of the arm(s) 122, such that they face towards the sensor hub 106 when the sensor hub 106 is connected to the sensor dock 104. The protrusion(s) 123 can smoothly transition from the inner surface(s) of the arm(s) 122 such that the sensor hub 106 can slidably engage with the protrusion(s) 123. For example, the protrusion(s) 123 can include ramp-like structures that define a smooth transition between the inner surface of the arm(s) 122 and the maximum "height" of the protrusion(s) 123. As another example, the protrusion(s) 123 can be rounded, have a rounded tip, and/or have a parabolic cross-section that allow for a smooth transition with the inner surface of the arm(s) 122. The protrusion(s) 123 can interact with corresponding recess(es) in the sensor hub 106, which can serve to releasably lock the sensor hub 106 in place with the sensor dock 104. In some implementations, interaction between the protrusion(s) 123 of the sensor dock 104 and recess(es) of the sensor hub 106 can provide tactile feedback to the subject that indicates complete engagement/connection of the sensor hub 106 with the sensor dock 104. Details of such recess(es) of the sensor hub 106 are described later with respect to FIGS. 5A-5J. Any number of retaining features can be provided on the sensor dock 104 to aid in releasably connecting the sensor hub 106 to the sensor dock 104. In some cases, other types of retaining features can be utilized. For example, edges of the arm(s) 122, which can be defined as the transition between the inner surface of the arm(s) 122 and outer surfaces of the arm(s) 122, can be configured to aid in the connection between the sensor dock 104 and the sensor hub 106. In some implementations, the arm(s) 122 are sized and shaped to releasably connect to the sensor hub 106. In some cases, the sensor hub 106 slidably connects to the sensor dock 104.

As discussed above and as shown in FIGS. 4A-4B, the sensor dock 104 can include an electrical connector 124. The electrical connector 124 can be configured to releasably electrically and mechanically connect to a corresponding electrical connector of the sensor hub 106, such that when connected to each other, the sensor hub 106 is placed in electrical communication with the one or more emitters 104a and the one or more detectors 104b of the system 100. The electrical connector 124 can include any number of pins. For example, with reference to FIGS. 4A-4B, the electrical connector 124 can include 8 pins. In some implementations, the electrical connector 124 includes an alternative number of pins. In some implementations, the electrical connector 124 can include a number of openings that correspond to a number of pins of a corresponding electrical connector of the sensor hub 106. As shown in FIGS. 4A-4B, the electrical connector 124 can be disposed at an inner portion of the base 128 of the sensor dock 104, such that it faces the sensor hub 106 when the sensor hub 106 is connected to the sensor dock 104. In some implementations, the sensor dock 104 can include one or more features to aid in aligning the electrical connector 124 to the corresponding electrical connector of the sensor hub 106. For example, the sensor dock 104 can include walls 126 positioned adjacent the electrical connector 124, and the walls 126 can be configured to aid in releasably connecting the electrical connector 124 with the corresponding electrical connector of the sensor hub 106.

The base 128 of the sensor dock 104 can include a top portion 128a and a bottom portion 128b (shown, for example, in FIGS. 4F-4G) that can be integrally formed (or alternatively, separable from one another). The top portion 128a can include the electrical connector 124 and the walls 126, and the bottom portion 128b can include the arm(s) 122. In some implementations, a shell 127 fits over and connects to the base 128 and the arm(s) 122, and/or is integral with the base 128 and the arm(s) 122. For example, the shell 127 can connect to the base 128 and arm(s) 122 via connecting portions 121 disposed on the arm(s) 122. In some cases, the shell 127 is integral with the sensor strap 130 as shown. In some implementations, the main body 120 of the sensor dock 104 does not include the shell 127. The main body 120 of the sensor dock 104 can be configured to connect to the holder 170 of the wearable device 102. For example, the main body 120 of the sensor dock 104 including the base 128 and arm(s) 122 can be sized and/or shaped to fit within at least a portion of the cavity 172 of the holder 170.

The sensor dock 104 can include one or more features for aiding in gripping and/or holding the sensor dock 104, such as for gripping and/or holding the sensor dock 104 when connecting and/or disconnecting sensor hub 106 to the sensor dock 104. For example, the sensor dock 104 can include one or more ribs 125 disposed on a portion of the base 128 (e.g., the bottom portion 128b) of the sensor dock 104, the ribs 125 configured to aid in gripping and/or holding the sensor dock 104. The ribs 125 can include generally linear protrusions that protrude out from the surface of the sensor dock 104 and extend along a portion of the sensor dock opposite of where it would contact the wearable device 102 when connected to the holder 170 (e.g., the ribs can be disposed along a "bottom" portion of the sensor dock 104). Alternatively, or in addition, in some implementations the sensor dock 104 can include other features configured to aid in gripping and/or holding the sensor dock 104, such as bumps, a roughened surface texture, etc.

As discussed above and as shown in FIGS. 4A-4F, the sensor dock 104 can include a sensor strap 130 that connects to and extends outward from the main body 120 of the sensor dock 104. As shown and in some implementations, the sensor strap 130 connects to a top of the main body 120 of the sensor dock 104 such that the sensor strap 130 is raised (e.g., forms a raised surface) relative to the rest of the sensor dock 104. Furthermore, the sensor strap 130 can be disposed at an end of the main body 120 adjacent the electrical connector 124. Additionally, the sensor strap 130 can extend from the main body 120 in a direction substantially perpendicular to the arm(s) 122 of the sensor dock 104. The sensor strap 130 can be configured to fit within and extend from the opening 171 in the wearable device 102 when the main body 120 of the sensor dock 104 is connected to the holder 170 of the wearable device 102 (for example, when the main body 120 of the sensor dock 104 is disposed within the cavity 172 of the holder 170). A portion of the sensor strap 130 that fits within the opening 171 can be configured to form a substantially flush surface (e.g., a substantially coplanar surface) with the base 160 of the wearable device 102 for receiving a bottom portion of the subject's foot 2. Additionally, a portion of the sensor strap 130 that extends from the opening 171 can be configured to wrap around at least a portion of the subject's foot and secure the wearable device 102 to the subject's foot 2.

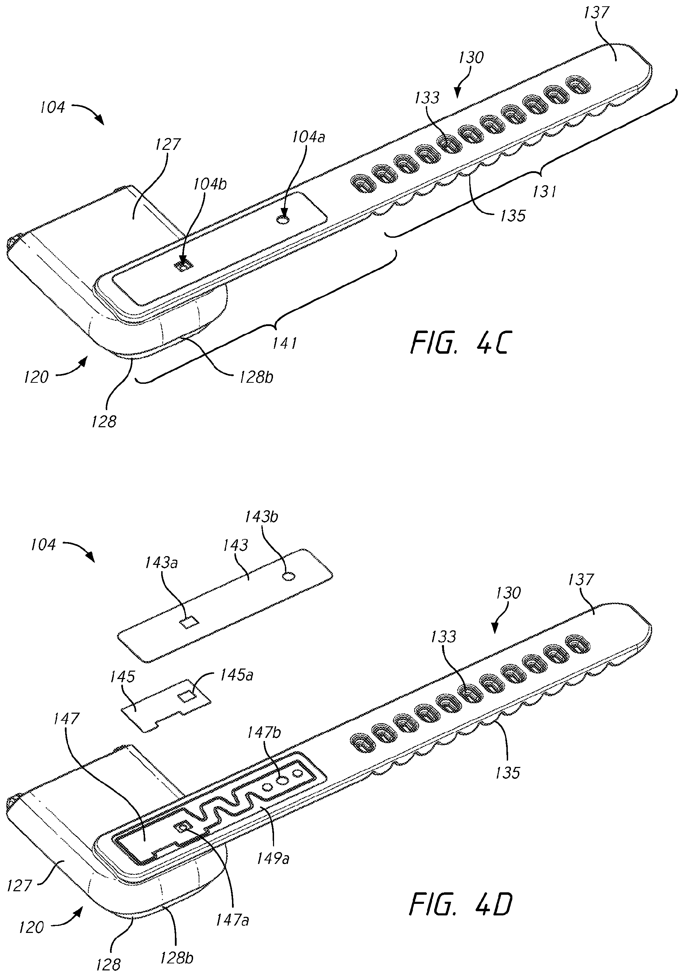

Strap 130 can include a sensor section 141 and a securement section 131 as shown in FIGS. 4A and 4C. The sensor section 141 can connect to and extend outward from the main body 120 of the sensor dock 104, while the securement section can be disposed at an end 137 of the sensor strap 130 opposite the sensor section 141. The securement section 141 can include one or more features for securing the system 100 to the subject's foot 2. For example and as shown, the securement section 141 can include hole(s) 133 and/or ridge(s) 135 that can interact with one or more features of the wearable device 102 described later herein for securing the system 100 to the subject's foot 2, although other methods of securement can be used. In some implementations, at least a portion of the strap 130 is stretchable. For example, at least a portion of the sensor section 141 and/or at least a portion of the securement section 131 can be configured to be stretchable.

FIGS. 4C-4G illustrate perspective views of the sensor dock 104 that progressively show the various aspects, components, and/or features that the sensor dock 104, in particular the sensor strap 130, can include. As shown in FIG. 4D, the sensor strap 130 can include a cover 143 (which may also be referred to as a "cover plate") configured to cover the electrical circuitry of the sensor strap 130, such as the circuit layer 147. The cover 143 can fit into a recess 149a of the sensor strap 130, such that the cover 143 and the surface of the sensor strap 130 adjacent the cover 143 form a substantially flush surface. The cover 143 can include an opening 143a configured to overlie the one or more detectors 104b in detector package 146 so as to allow optical radiation emitted from the one or more emitters 104a in emitter package 144 to reach the detectors. Similarly, the cover 143 can include an opening 143b configured to overlie the one or more emitters 104a in emitter package 144 so as to allow optical radiation emitted from the one or more emitters 104a to pass through. In some implementations, openings 143a, 143b are covered by transparent material (for example, to prevent ingress of liquid therethrough). However, in alternative implementations, openings 143a, 143b are not covered.

In addition to the cover 143, the sensor strap 130 can include a stiffener 145 configured to increase the stiffness of a portion of the sensor strap 130 that is positioned within the opening 171 of the wearable device 102 when the sensor dock 104 is connected to the holder 170. The stiffener 145 can be disposed below the cover 143 and can include an opening 145a configured to allow optical radiation to pass through (e.g., so as not to block optical radiation from being emitted by the one or more emitters 104a or optical radiation from being received by the one or more detectors 104b). Also shown in FIG. 4D is the circuit layer 147, which can be disposed below the cover 143 and the stiffener 145 and can fit into a recess 149b of the sensor strap 130 (shown in FIG. 4E-4F). As mentioned above, the circuit layer 147 can electrically connect the one or more emitters 104a in emitter package 144 and the one or more detectors 104b in detector package 146 to the electrical connector 124 of the sensor dock 104. In some implementations, the circuit layer 147 also electrically connects the temperature sensor(s) 104c of the sensor dock 104 to the electrical connector 124. The circuit layer 147 can be configured as a flexible circuit that can bend freely with the sensor strap 130. For this, the circuit layer 147 can have a length that is greater than a distance between where the circuit layer 147 electrically connects to the one or more emitters 104a in emitter package 144 and the one or more detectors 104b in detector package 146. For example, the circuit layer 147 can have one or more bends (e.g., can be serpentine). Similar to the opening 143a and the opening 143b in the cover 143, the circuit layer 147 can include an opening 147a and an opening 147b configured to overlie the one or more detectors 104b and one or more emitters 104a, respectively, and allow optical radiation to be received and/or emitted therethrough.

FIG. 4E shows the sensor dock 104 with the cover 143, the stiffener 145, and the circuit layer 147 removed from view. This view shows the emitter package 144, the detector package 146, and the temperature sensor(s) 104c in a temperature sensor package 148 positioned by the sensor strap 130. The one or more emitters 104a in emitter package 144 can be positioned within a first portion of the sensor strap 130 that is outside of and/or spaced away from the opening 171 of the wearable device 102 when the sensor dock 104 is connected to the holder 170. For example, the emitter package 144 can be disposed within a cavity 149c of the sensor strap 130 (shown in FIG. 4F). The one or more detectors 104b in detector package 146 and the temperature sensor(s) 104c in temperature sensor package 148 can be positioned within a second portion of the sensor strap 130 that can be positioned within the opening 171 of the wearable device 102 when the sensor dock 104 is connected to the holder 170. The temperature sensor(s) 104c in temperature sensor package 148 can be positioned proximate to but spaced from the one or more detectors 104b in detector package 146. For example, the detector package 146 and the temperature sensor package 148 can be disposed within a cavity 149d of the sensor strap 130 (shown in FIG. 4F). The sensor section 141 of the sensor strap 130 can include both of such first portion and second portion described above.

The one or more emitters 104a in emitter package 144 can be aligned with, for example vertically aligned, and/or aimed towards the one or more detectors 104b in detector package 146 when the sensor strap 130 is wrapped around a top portion of the subject's foot 2 (for example, as shown in FIG. 1D). The temperature sensor(s) 104c in temperature sensor package 148 can be horizontally aligned with the detector package 146 and not vertically aligned with the emitter package 144 when the sensor strap 130 is wrapped around the top portion of the subject's foot 2 (also shown in FIG. 1D). In such positions and with the sensor strap 130 wrapped around the top portion of the subject's foot 2, the emitter package 144 can be operably positioned against and/or adjacent to tissue of the top of the subject's foot 2, and the detector package 146 and temperature sensor package 148 can be operably positioned against and/or adjacent to tissue of the bottom of the subject's foot 2 (e.g., tissue of the ball of the subject's foot 2). Thus, the detector package 146 can be operably positioned by the sensor strap 130 such that optical radiation emitted from the emitter package 144 can pass through and/or be attenuated by the tissue of the subject's foot 2 before being detected by the detector package 146. In some implementations, the locations of the one or more emitters 104a in emitter package 144 and the one or more detectors 104b in detector package 146 can be switched. In such implementations, and with the sensor strap 130 wrapped around the top portion of the subject's foot 2, the detector package 146 can be operably positioned against and/or adjacent to tissue of the top of the subject's foot 2, and the emitter package 144 can be operably positioned against and/or adjacent to tissue of the bottom of the subject's foot 2 (e.g., tissue of the ball of the subject's foot 2).

With continued reference to FIG. 4E, the sensor strap 130 can also include a detector shield 132. The detector shield 132 can at least partially enclose and/or surround the detector package 146 comprising the one or more detectors 104b. The detector shield 132 can be configured to inhibit, prevent, and/or reduce ambient light, stray light, and/or light emitted from the emitter package 144 that does not pass through tissue from being received by the detector package 146, which can advantageously improve the integrity of physiological parameter determination. Additionally, or alternatively, detector shield 132 can shield the detector package 146 against and/or with respect to electromagnetic noise. For example, in some implementations, the detector shield 132 can act as a Faraday cage or a shield to block electromagnetic fields. The sensor strap 130 can also include optical transmission material configured to direct optical radiation toward the detector package 146 after passing through tissue of the subject's foot 2. In some cases, the optical transmission material can include a lens. In some cases, the optical transmission material can include a diffuser configured to diffuse, spread out, disseminate, and/or scatter optical radiation attenuated by tissue prior to being received by the detector package 146. The optical transmission material can form a part of the detector package 146, or it can be configured to be positioned between the detector package 146 and tissue of the subject 1 when the system 100 is secured to the subject 1. In some implementations, the optical transmission material is disposed within the opening 143a of the cover 143 overlying the detector package 146. Similarly, the sensor strap 130 can include optical transmission material configured to focus or diffuse optical radiation emitted from the emitter package 144. In some cases, the optical transmission material can include a lens. In some cases, the optical transmission material can include a diffuser configured to diffuse, spread out, disseminate, and/or scatter optical radiation emitted from the emitter package 144 prior to such optical radiation entering the subject's tissue. In some cases, this can permit optical radiation emitted from the emitter package 144 to pass through a greater amount of tissue and can facilitate more accurate determination of physiological parameters (such as any of those discussed herein). The optical transmission material can form a part of the emitter package 144, or it can be configured to be positioned between the emitter package 144 and tissue of the subject 1 when the system 100 is secured to the subject 1. In some implementations, the optical transmission material is disposed within the opening 143b of the cover 143 overlying the emitter package 144.

Advantageously, at least a portion of the sensor strap 130 can be made of a pliable material. For example, at least a portion of the sensor strap 130 can be made of silicone, such as a medical grade and/or biocompatible silicone, a thermoplastic elastomer, such as a medical grade and/or biocompatible thermoplastic elastomer, and/or any biocompatible material and/or polymer that is pliable, flexible, stretchy, soft, and/or conformable. A pliable sensor strap 130 can advantageously position the emitter package 144 and the detector package 146 close to, against, and or adjacent to a portion of the subject's body, such as the tissue of the subject's foot 2, for optimal function of the system 100. For example, by conforming to the subject's foot 2, the sensor strap 130 can optimally position the emitter package 144 against, adjacent, and/or near to the subject's foot 2 such that optical radiation emitted from the emitter package 144 is directed to/through the subject's foot 2. By way of another example, by the sensor strap 130 conforming to the subject's foot 2, ambient and/or stray optical radiation and/or optical radiation not produced/emitted by the emitter package 144 can be reduced, eliminated, and/or prevented from being received by the detector package 146. Furthermore, a pliable sensor strap 130 can advantageously improve comfort for the subject 1 when the system 100 is secured to and/or worn by the subject 1. In some implementations, the sensor strap 130 and/or portions thereof can be rigid or semi-rigid. In some cases, the sensor strap 130 can be a composite material and/or a composite of rigid, semi-rigid, and/or pliable/conforming material.

In some implementations, the sensor strap 130 can be configured to inhibit, prevent, and/or reduce an amount of ambient light, stray light, and/or any optical radiation not emitted from the emitter package 144 from reaching the detector package 146. Additionally, or alternatively, the sensor strap 130 can be configured to inhibit, prevent, and/or reduce an amount of optical radiation emitted by the emitter package 144 that has not been attenuated by, reflected by, and/or passed through tissue of the subject from being received by the detector package 146. In some cases, the sensor strap 130 can be opaque and/or generally light blocking and/or have a light blocking coating. In some implementations, sensor strap 130 can be semi-transparent or transparent. In some implementations, the sensor strap 130 can include portions that are opaque and/or light blocking and portions that are semi-transparent and/or transparent.

FIGS. 5A-5B illustrate perspective views, FIG. 5C illustrates a bottom view, FIG. 5D illustrates a top view, FIGS. 5E-5F illustrate side views, and FIGS. 5G-5H illustrate front and back views, respectively, of the sensor hub 106. FIGS. 5I-5J illustrate perspective exploded views of the sensor hub 106.

As shown in FIGS. 5A-5J, the sensor hub 106 can include a first end 150, a second end 152 opposite the first end 150, a first side 154, and a second side 156 opposite the first side 154. Sensor hub 106 can comprise a length along sides 154, 156 and/or a width along ends 150, 152 greater than a height of the sensor hub 106. As discussed above with respect to FIG. 3, the sensor hub 106 can include one or more processors 106a, one or more storage devices 106b, a communication module 106c, a battery 106d, an information element 106e, one or more other sensors 106f, one or more status indicators 106g, and/or a vibration motor 106h. Further as discussed above, the sensor hub 106 can be configured to releasably mechanically and electrically connect with the sensor dock 104. For this, the sensor hub 106 can be sized and/or shaped and/or include one or more features (for example, recesses) for releasably mechanically and electrically connecting to the sensor dock 104. In some cases, the sensor hub 106 includes one or more features for engaging with one or more retaining features of the sensor dock 104. For example, in some implementations the sensor hub 106 includes recessed portion(s) 158 disposed along at least a portion of the sides 154, 156 configured to slidably and releasably mechanically connect with the arm(s) 122 of the sensor dock 104. The recessed portion(s) 158 can extend from end 150 along sides 154, 156 towards end 152, and in some cases can terminate adjacent to and/or near the end 152. In addition to recessed portion(s) 158, the sensor hub 106 can include one or more features for releasably mechanically connecting to the sensor dock 104. For example, the sensor hub 106 can include recess(es) 158a, non-recessed portion(s) 158b, and recess(es) 158c, which can as shown be disposed along recessed portion(s) 158. In some implementations, the recess(es) 158a and the recess(es) 158c can be configured to slidably receive the protrusion(s) 123 of the sensor dock 104 when the recessed portion(s) 158 of the sensor hub 106 slidably engage with the arm(s) 122 of the sensor dock 104. For example, the sensor hub 106 can be mechanically connected to the sensor dock 104 by aligning recessed portion(s) 158 with arm(s) 122 of the sensor dock 104 while the sensor hub 106 is positioned away from the sensor dock 104 but generally in the same plane as the sensor dock 104 and with end 150 of the sensor hub 106 facing the sensor dock 104, sliding the sensor hub 106 towards the sensor dock 104 such that the arm(s) 122 engage with the recessed portion(s) 158, continuing to slide the sensor hub 106 towards the sensor dock 104 such that the recess(es) 158a receive the protrusion(s) 123, continuing to slide the sensor hub 106 towards the sensor dock 104 such that the non-recessed portion(s) 158b engage and/or interact with the protrusion(s) 123, and continuing to slide the sensor hub 106 towards the sensor dock 104 until the recess(es) 158c receive the protrusion(s) 123. In the example above, the interaction between the non-recessed portion(s) 158b of the sensor hub 106 and the protrusion(s) 123 of the sensor dock 104 can provide tactile feedback to the subject, such as the feel of a "snap" when the non-recessed portion(s) 158b are slid past the protrusion(s) 123. In the case of connecting the sensor hub 106 to the sensor dock 104, such a "snap" feel can indicate to the subject that the sensor hub 106 is fully connected to the sensor dock 104 once the protrusion(s) 123 have slid past the non-recessed portion(s) 158b and the recess(es) 158c have received the protrusion(s) 123.

With continued reference to FIGS. 5A-5J and as discussed above, the sensor hub 106 can include an electrical connector 151 configured to electrically and mechanically connect with the corresponding electrical connector 124 of the sensor dock 104. The sensor hub 106 and its components, such as processor(s) 106a and battery 106d, can be operably connected to the one or more emitters 104a and the one or more detectors 104b of the system 100 when the electrical connector 151 of the sensor hub 106 is connected to the electrical connector 124 of the sensor dock 104. In some implementations, the electrical connector 151 of the sensor hub 106 can be electrically and mechanically connected to the electrical connector 124 of the sensor dock 104 when the sensor hub 106 is mechanically connected to the sensor dock 104, such as by the connection between the arm(s) 122 of the dock and the recessed portion(s) 158 of the sensor hub 106 as described above. The electrical connector 151 of the sensor hub 106 can include one or more openings configured to receive one or more pins of the corresponding electrical connector 124 of the sensor dock 104. For example and as shown in FIGS. 5A-5B, the electrical connector 151 of the sensor hub 106 can include 8 openings, the openings configured to electrically and mechanically connect with corresponding pins of the electrical connector 124 of the sensor dock 104. In some implementations, the electrical connector 151 can include any number of openings. In some implementations, the electrical connector 151 can include one or more pins configured to electrically and mechanically connect with one or more corresponding openings in the electrical connector 124 of the sensor dock 104. As shown in FIGS. 5A-5B, the electrical connector 151 can be disposed at end 150 of the sensor hub 106, for example, such that it faces towards the electrical connector 124 of the sensor dock 104 when the sensor hub 106 is connected to the sensor dock 104. In some implementations, the sensor hub 106 can include one or more features to aid in aligning the electrical connector 151 to the corresponding electrical connector 124 of the sensor dock 104. For example, the sensor hub 106 can include slot(s) 159 adjacent the electrical connector 151, the slot(s) 159 configured to releasably receive the walls 126 of the sensor dock 104 and aid in releasably connecting the electrical connector 151 with the corresponding electrical connector 124.

As shown in FIGS. 5A-5C, 5E-5H, the sensor hub 106 can include one or more features for aiding in gripping, holding, moving, and/or sliding the sensor hub 106, such as for sliding the sensor hub 106 when connecting and/or disconnecting the sensor hub 106 to the sensor dock 104. For example, the sensor hub 106 can include one or more ribs 155 disposed on a portion of the sensor hub 106, the ribs 155 configured to aid in gripping, holding, moving, and/or sliding the sensor hub 106. As shown, the ribs 155 can include generally linear protrusions that protrude out from a surface of the sensor hub 106 and extend at least partially from side 154 towards side 156 along a portion of the sensor hub 106 near end 152 (e.g., the ribs can be disposed along an outward-facing portion of the sensor hub 106). Alternatively, or in addition, in some implementations the sensor hub 106 can include other features configured to aid in gripping, holding, moving, and/or sliding the sensor hub 106, such as bumps, a roughened surface texture, etc.

With continued reference to FIGS. 5A-5J and as discussed above, the sensor hub 106 can include one or more status indicators 106g. The one or more status indicators 106g can be configured, for example, to emit optical radiation out of and/or through a hole and/or opening in the sensor hub 106, such as through the hole/opening 153 in a top shell 160 of the sensor hub 106. As shown in FIGS. 5I-5J, the one or more status indicators 106g can include status indicator 167, which can be an emitter (e.g., an LED) configured to emit optical radiation. The status indicator 167 can be operably coupled to a circuit board (also referred to herein as a "PCB") 163 and the processor(s) 106a of the sensor hub 106. The hole/opening 153 can allow the optical radiation emitted by the status indicator 167 to be visible from a location external to the sensor hub 106, such as by the subject 1 when wearing/using the system 100 and/or by the subject's care providers. In some implementations, hole/opening 153 can be at least partially aligned with status indicator 167 to allow optical radiation emitted from the status indicator 167 to more easily pass through the top shell 160. Additionally, or alternatively, the top shell 160 and/or a bottom shell 161 of the sensor hub 106 can comprise a transparent or semi-transparent material that allows optical radiation emitted from the status indicator 167 to be seen from a location external to the sensor hub 106.

In some implementations, the sensor hub 106 can include an RFID reader and the sensor dock 104 can include an RFID tag. The RFID tag of the sensor dock 104 can be configured to communicate with the RFID reader of the sensor hub 106. In some implementations, the sensor hub 106 can include an RFID tag and the sensor dock 104 can include an RFID reader. The RFID tag of the sensor hub 106 can be configured to communicate with the RFID reader of the sensor dock 104.

Referring to the perspective exploded views of FIGS. 5I-5J, shown are components of the sensor hub 106 in accordance with some implementations. As shown, the sensor hub 106 can include the bottom shell 161, a battery 165, the PCB 163, and the top shell 160. In some implementations, the sensor hub 106 can also include a vibration motor 164, the status indicator 167 described above, and/or a PCB overmolding 162. The vibration motor 164 can be an example implementation of vibration motor 106h discussed herein, and can be configured to provide haptic feedback, vibration, alerts, notifications, alarms, etc. to the subject 1 and/or their care providers when the system 100 is secured to the subject. The battery 165 can correspond to battery 106d discussed herein, and can be configured to provide power to the system 100. The PCB 163 can include and/or be operably coupled with the processor(s) 106a, the storage device(s) 106b, the communication module 106c, the information element 106e, the status indicator(s) 106g and status indicator 167, and/or the vibration motor 106h and vibration motor 164 discussed previously. The PCB overmolding 162 can be configured to seal the PCB 163, at least a portion of the PCB 163, and/or at least some of the PCB's components, such as against water, other liquids, air, dust, contaminants, etc. The PCB overmolding 162 can also be configured to provide shock and/or drop protection for the PCB 163 and/or its components. The battery 165 can be configured to operably connect to the PCB 163, components of the PCB 163, and/or the electrical connector 151 of the sensor hub 106. The bottom shell 161 and the top shell 160 can be configured to contain components of the sensor hub 106 and can connect/join to each other (for example, by ultrasonic welding) to create a housing/shell of the sensor hub 106. As such, the top shell 160 and/or the bottom shell 161 can comprise the one or more features of the sensor hub 106 configured to releasably connect with the sensor dock 104, such as recessed portion(s) 158, recess(es) 158a, non-recessed portion(s) 158b, and recess(es) 158c.

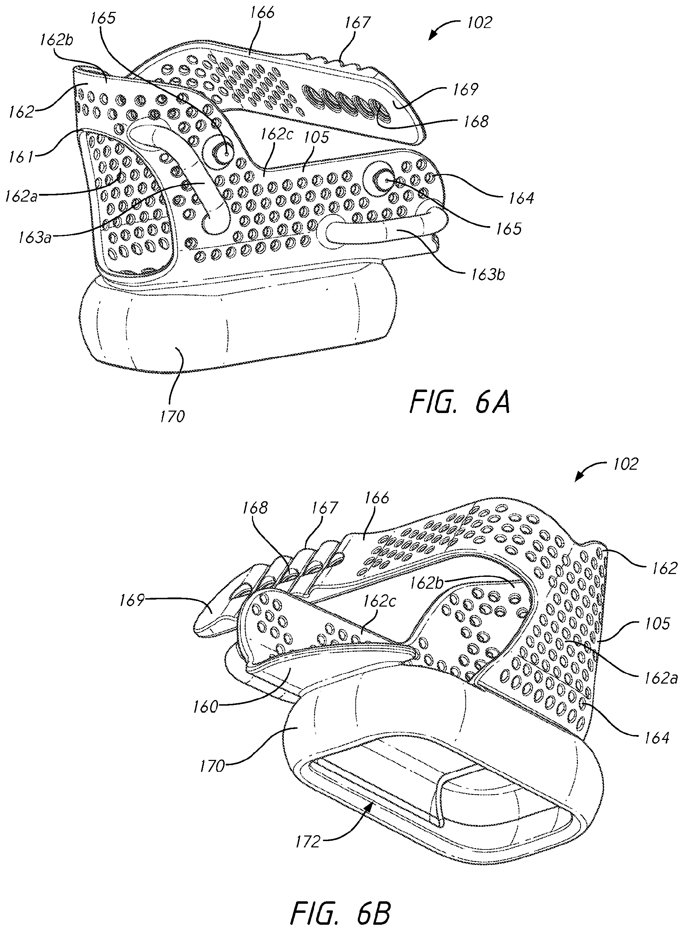

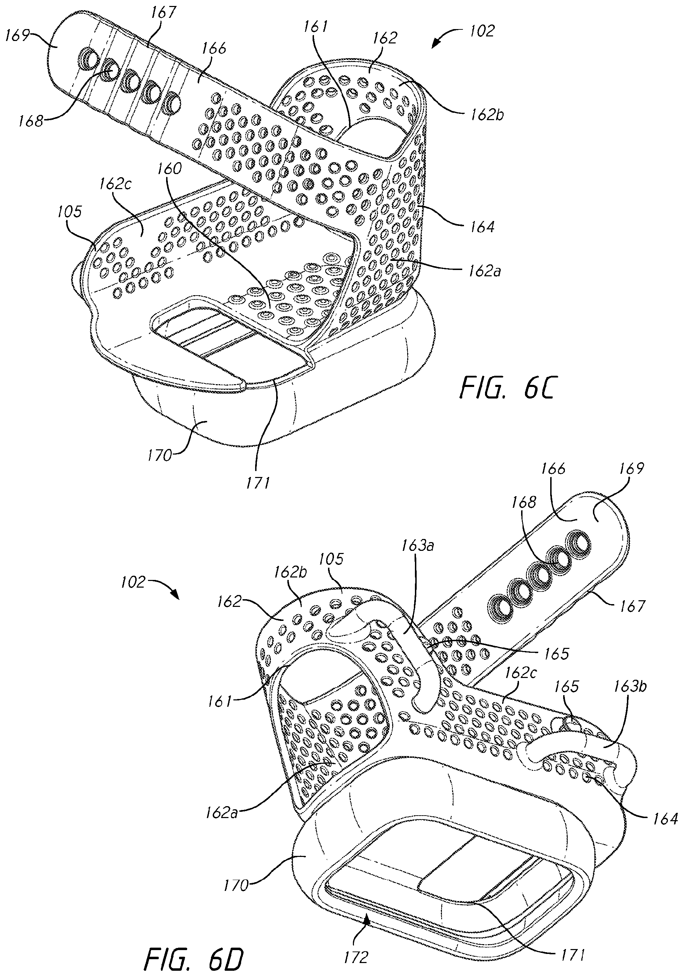

FIGS. 6A-6D illustrate perspective views of the wearable device 102 of the system 100 of FIGS. 1A-1B in accordance with some implementations of this disclosure. The wearable device 102 can be configured to receive and support the foot 2, ankle 3, heel 4, and/or lower leg 5 of the subject 1. Furthermore, the wearable device 102 can be made of a resilient material. Resilient can include the ability to return to original shape after bending, stretching, or being compressed. For example, the wearable device 102 can be made of silicone, such as a medical grade and/or biocompatible silicone, a thermoplastic elastomer, such as a medical grade and/or biocompatible thermoplastic elastomer, and/or any biocompatible material and/or polymer. As discussed with respect to FIG. 2E, the wearable device 102 can have a main body 105 including the base 160, the opening 171 in the base 160, and the wall 162 extending from the base 160. Also discussed and in some implementations, the main body 105 additionally includes the wearable device strap 166 extending from the wall 162. As shown in FIGS. 6A-6D, the wall 162 of the wearable device 102 can extend upward from a periphery of the base 160 and include a side portion 162a (which can also be referred to herein as a "sidewall portion"), a back portion 162b (which can also be referred to herein as a "back wall portion"), and a side portion 162c (which can also be referred to herein as a "sidewall portion") configured to wrap around and support portions of the subject's foot 2, such as side(s) of the subject's foot 2, the subject's ankle 3, the subject's heel 4, and/or the subject's lower leg 5. The wall 162 can be discontinuous such that it does not enclose the complete periphery of the base 160, leaving space for the sensor strap 130 to extend from the opening 171 in the base 160. Furthermore, the wall 162 can be discontinuous such that the toes of the subject's foot 2 are not enclosed. The wall 162 can have a variable height as it extends upward from the base. For example and as shown in FIGS. 6A-6D, the wall 162 can have a maximum height at the back portion 162b so as to surround and support the subject's heel 4, and a reduced height at side portions 162a and 162c. In some implementations, the main body 105 of the wearable device 102 can include a plurality of hole(s) 164 for venting and/or for tuning the resilience of the wearable device 102. For example, the wall 162 and/or the base 160 can have a plurality of hole(s) 164 therethrough. Furthermore and in some implementations, the main body 105 of the wearable device 102 can include an opening 161 configured to receive the heel 4 of the subject's foot 2. The opening 161 can be located in the back portion 162b of the wall 162.

With continued reference to FIGS. 6A-6D, the wearable device 102 can also include one or more features for securing to the subject's foot 2. For example and as shown, the main body 105 of the wearable device 102 can include the wearable device strap 166 mentioned previously that can extend outward from the wall 162, as well as a protrusion 165 and/or a strap loop 163a configured to interact with hole(s) 168 and/or ridge(s) 167 of the wearable device strap 166, respectively, positioned adjacent an end 169 of the wearable device strap 166. The wearable device strap 166 can be configured to wrap over a portion of the subject's foot 2, ankle 3, and/or lower leg 5 and be secured to the protrusion 165 and/or the strap loop 163a. The wearable device strap 166 can extend from side portion 162a of the wall 162 at a location adjacent where the wearable device 102 would receive the subject's ankle 3 if secured to the subject 1. Further, the wearable device strap 166 can extend from the side portion 162a at an acute angle with respect to the base 160 when viewed from a side of the wearable device 102. The protrusion 165 and/or the strap loop 163a can extend outward and generally orthogonal from the side portion 162c of the wall 162 (e.g., a side of the wall 162 opposite from where from the wearable device strap 166 extends from) to interact with the hole(s) 168 and/or ridge(s) 167 of the wearable device strap 166, respectively. The protrusion 165 can be generally mushroom-shaped and have an enlarged end for releasably securing through the hole(s) 168 of the wearable device strap 166. The strap loop 163a can create a generally elongate opening configured to releasably receive the end 169 and ridge(s) 167 of the wearable device strap 166. The main body 105 of the wearable device 102 can also include one or more features for releasably securing to the sensor strap 130 when the sensor dock 104 is connected to the holder 170. For example and as shown, the side portion 162c of the wall 162 can include another protrusion 165 and/or a strap loop 163b (which can be the same as or similar to the strap loop 163a) that can interact with the hole(s) 133 and/or ridge(s) 135 of the sensor strap 130 similar to or the same as how such features interact with the wearable device strap 166. To customize the fit of the system 100 to the subject's foot 2, the wearable device strap 166 and/or the sensor strap 130 can be wrapped over the subject's foot 2, pulled through strap loops 163a and 163b, respectively, and a protrusion 165 secured through an appropriate hole (e.g., one of hole(s) 168 and 133, respectively) of the strap(s).

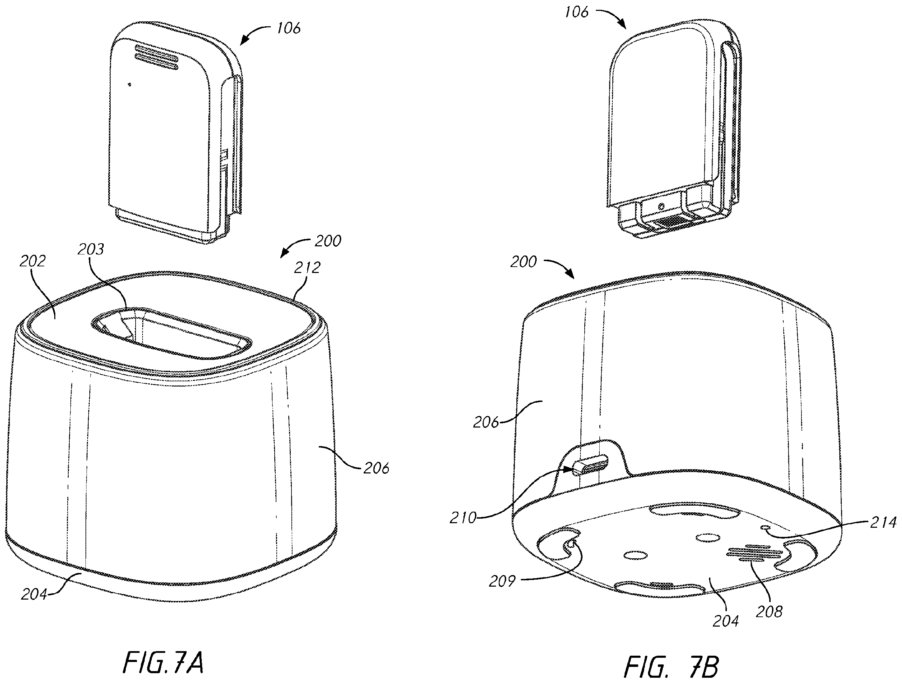

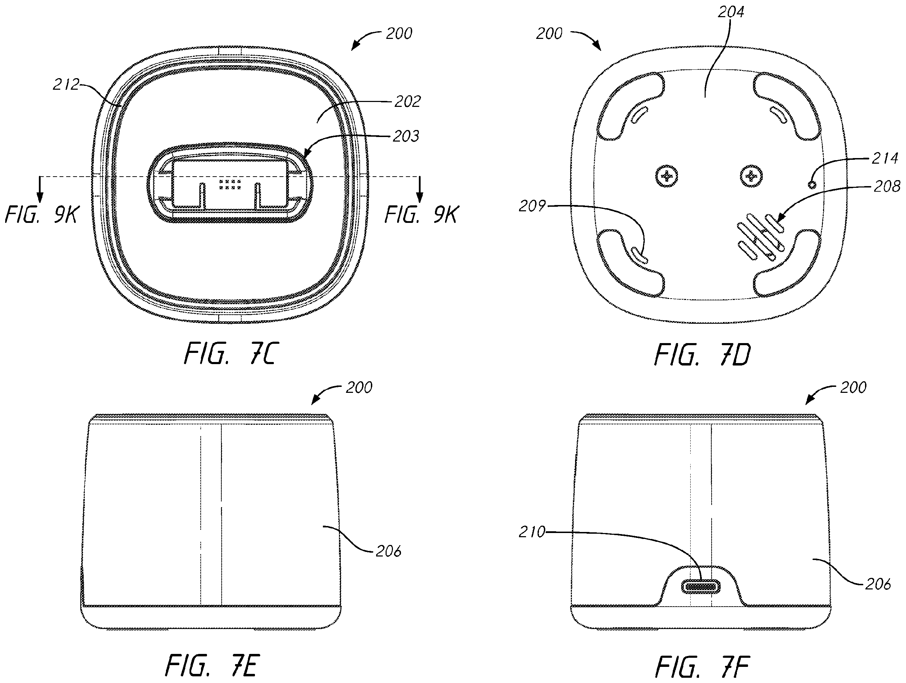

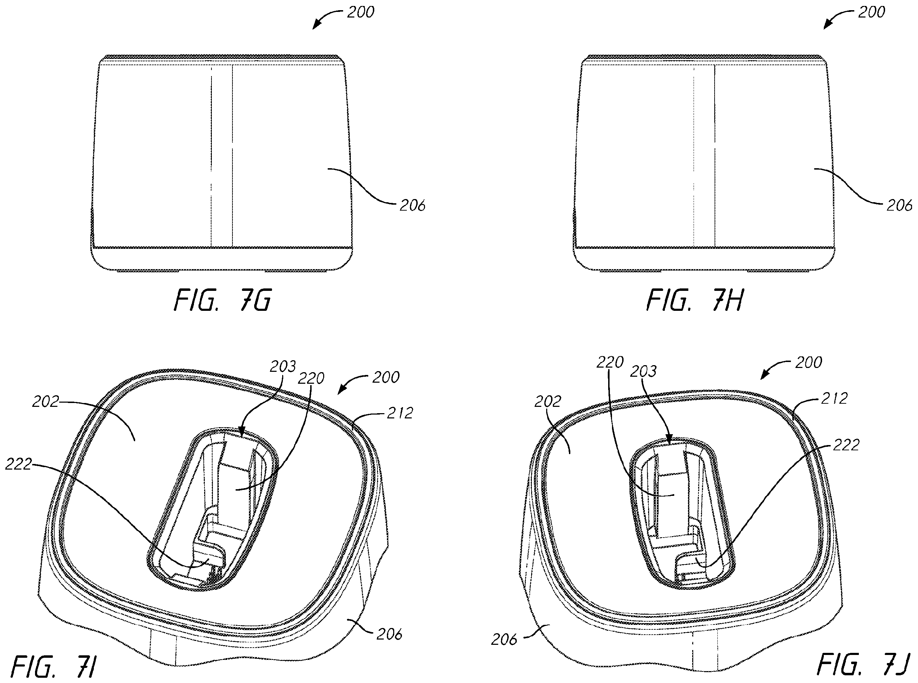

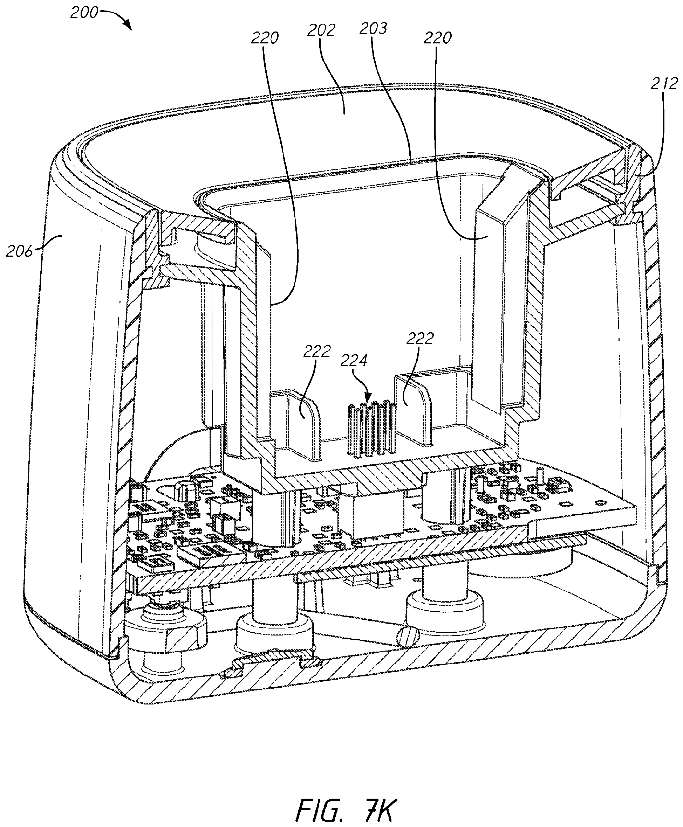

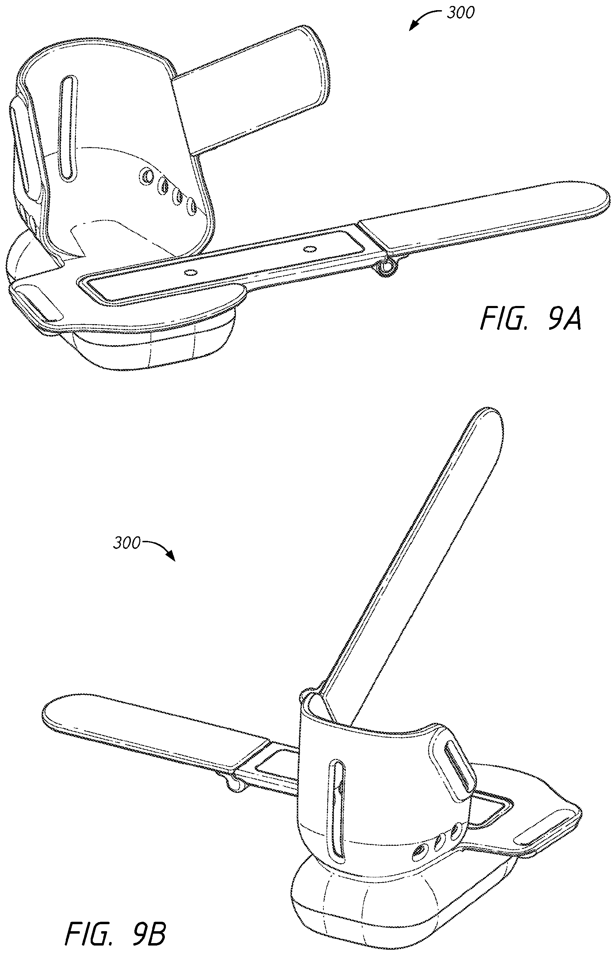

FIGS. 7A-7K illustrate various views of a charging station 200. FIGS. 7A-7B illustrate perspective views of the charging station 200 with the sensor hub 106 described herein removed from the charging station 200, FIG. 7C illustrates a top view of the charging station 200 of FIG. 7A, FIG. 7D illustrates a bottom view of the charging station 200 of FIG. 7A, FIGS. 7E-7F illustrate front and back views, respectively, of the charging station 200 of FIG. 7A, FIGS. 7G-7H illustrate side views of the charging station 200 of FIG. 7A, FIGS. 7I-7J illustrate perspective views of the charging station 200 of FIG. 7A, and FIG. 7K illustrates a perspective cross sectional view through the charging station 200 of FIG. 9A as indicated in FIG. 7C.

The charging station 200 can be configured to releasably mechanically and electrically connect to (e.g., receive) the sensor hub 106. The charging station 200 can, when electrically connected to the sensor hub 106, charge and/or recharge the battery 165 of the sensor hub 106. As shown in FIGS. 7A-7K, the charging station 200 (which can also be referred to herein as a "charging base," a "hub," and/or "base station") can comprise a generally cube like body 206 with a bottom plate 204 (which can also be referred to herein as a "bottom surface"), a top plate 202 (which can also be referred to herein as a "top surface"), a cavity 203, and an electrical connector 210. The body 206 of the charging station 200 can have a rounded square like cross section, seen most clearly in the top and bottom views of FIGS. 7C-7D. The charging station 200 can include an indicator 212, opening(s) 208, one or more speakers, opening(s) 209, a reset button 214, and other features as described further below. Furthermore, the charging station 200 can include any one or more of the features described with respect to the schematic diagram of FIG. 3, including one or more processor(s) 106a, one or more storage device(s) 106b, a communication module 106c, a battery 106d, an information element 106e, one or more other sensor(s) 106f, one or more status indicator(s) 106g, and a vibration motor 106h.

As shown in FIGS. 7A-7K, the bottom plate 204 can connect to the body 206 and form the bottom portion of the charging station 200. The opening(s) 208 can be disposed on the bottom plate 204 and can, for example, facilitate communication and/or sound from one or more speakers and/or other indicators disposed within the charging station 200 from being transmitted to outside of the charging station 200 (e.g., for a subject to hear and/or be notified). The opening(s) 209 can also be disposed on the bottom plate 204 and can, for example, allow for any foreign matter (liquids, debris, etc.) that may fall into the charging station 200 to escape. The charging station 200 can be configured to rest on a surface, such as a table top, and as such the bottom plate 204 can include one or more pads, non-slip features, etc. and/or be otherwise configured to provide a stable base for the charging station 200. The bottom plate 204 can also include the electrical connector 210, which can be disposed along a side of the bottom plate 204 such that it is accessible when the charging station 200 rests on/against a surface. As shown, the electrical connector 210 can be disposed along the side of the bottom plate 204 at an end of the charging station 200 that is the back of the charging station 200. In some implementations the electrical connector 210 can instead be disposed on a portion of the body 206, for example the side of the body 206 that is the back end of the charging station 200. The electrical connector 210 can be a connector and/or charging port, such as a USB-C connector/port, that can be configured to provide power to the charging station 200 when operably connected to a power source. In some implementations, the charging station 200 can include a reset button 214 disposed on the bottom plate 204 configured to reset the charging station 200 if pressed and/or pressed and held by the subject.

Further as shown, the top plate 202 can connect to the body 206 and form the top portion of the charging station 200. The top plate 202 can be configured as a pushbutton, such that the subject can push down on a surface (e.g, the top facing surface) of the top plate 202 to interact with the charging station 200. In some implementations, the surface of the top plate 202 slopes downward towards its center, creating a generally concave surface of the top plate 202. The indicator 212 can be disposed at and/or between the peripheral connection between the top plate 202 and the body 206. For example, the indicator 212 can at least partially circumferentially surround the top plate 202 (as shown, the indicator 212 fully circumferentially surrounds the top plate 202). The indicator 212 can be configured to emit optical radiation and/or allow emission of optical radiation. As an example, the indicator 212 can include one or more emitters configured to emit optical radiation from the charging station 200. As another example, the indicator 212 can be made of a transparent, a semi-transparent, a light transmissible, and/or a partially light transmissible material that can allow optical radiation from one or more emitters located inside the charging station 200 to pass and/or partially pass through. The indicator 212 can be configured to indicate a status of the charging station 200 and/or to indicate a status of the sensor hub 106 when the sensor hub 106 is connected to the charging station 200 (e.g., to indicate a charge state of the battery 165 of the sensor hub 106, such as low charge, medium charge, and/or fully charged).

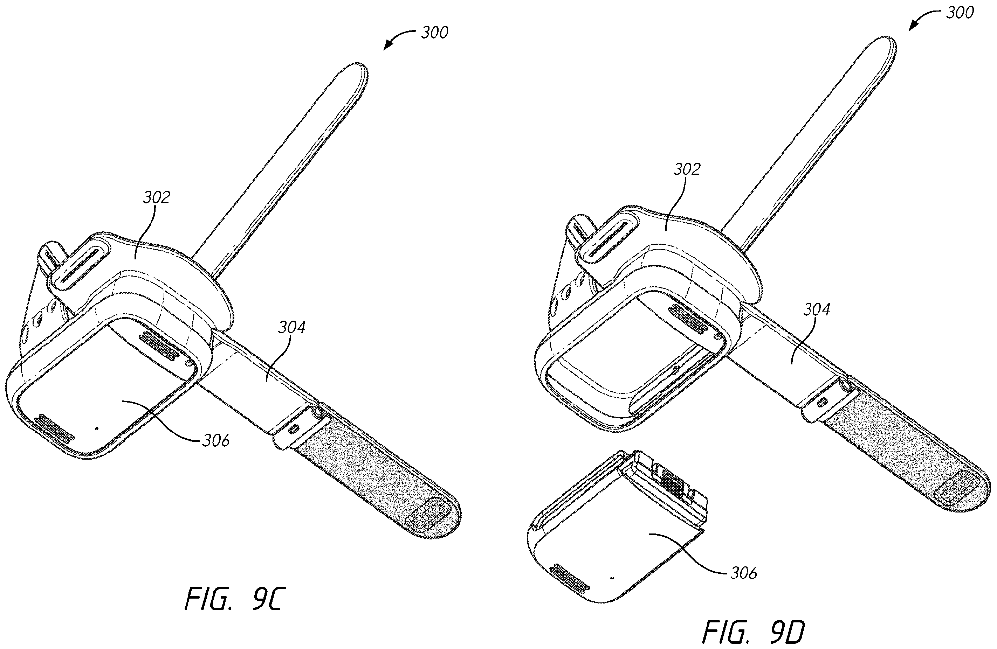

With continued reference to FIGS. 7A-7K, the cavity 203 of the charging station 200 can be configured to releasably mechanically receive the sensor hub 106. As such, the cavity 203 can be shaped and sized to receive the sensor hub 106. The cavity 203 can be disposed within and/or be defined by an opening in the top plate 202, such that the cavity 203 extends down from the top surface of the charging station 200 towards the bottom of the charging station 200. The cavity 203 can include one or more features for releasably mechanically connecting to the sensor hub 106. For example, the cavity 203 can include stem(s) 220 configured to releasably mechanically connect with the sensor hub 106. The stem(s) 220 can releasably mechanically connect with the sensor hub 106 similar to how the arm(s) 122 of the sensor dock 104 can connect with the sensor hub 106. For example, the stem(s) 220 can be disposed and extend along opposite sides of the cavity 203 and can slidably fit the recessed portion(s) 158 of the sensor hub 106 when the sensor hub 106 is slid into the cavity 203. In some implementations and as shown (e.g., in particular in the top view of FIG. 9C), the cavity 203 can have a variable contour and/or a contour on one side that is different from a contour of an opposite side (e.g., sides that do not have the stem(s) 220) to ensure and/or aid in proper alignment and placement of the sensor hub 106 with the charging station 200.

The cavity 203 can include one or more additional and/or alternative features for releasably electrically and mechanically connecting the sensor hub 106 with the charging station 200. With continued reference to FIGS. 7A-7K, the cavity 203 can include an electrical connector 224 configured to electrically and mechanically connect with the corresponding electrical connector 151 of the sensor hub 106. The sensor hub 106 and its components, such as processor(s) 106a and battery 106d/165, can be operably connected to the charging station 200 and components thereof, including electrical connector 210 for receiving electrical power, when the electrical connector 151 of the sensor hub 106 is connected to the electrical connector 224 of the charging station 200. In some implementations, the electrical connector 151 of the sensor hub 106 can be electrically and mechanically connected to the electrical connector 224 of the charging station 200 when the sensor hub 106 is mechanically connected to the charging station 200, such as by the connection between the stem(s) 220 of the charging station and the recessed portion(s) 158 of the sensor hub 106 as described above. In some cases, the sensor hub 106 can be electrically and mechanically connected to the charging station 200 when it is placed inside the cavity 203. The electrical connector 224 of the charging station 200 can include one or more pins, for example 8 pins as shown in FIGS. 7C and 7K. In some implementations, the electrical connector 224 can include any number of pins. In some implementations, the electrical connector 224 can include one or more openings configured to receive one or more pins of the corresponding electrical connector of the sensor hub 106. As shown in FIGS. 7C and 7K, the electrical connector 224 can be disposed within the cavity 203, for example at the bottom of the cavity 203, such that it faces the sensor hub 106 when the sensor hub 106 is connected to the charging station 200. In some implementations, the charging station 200 can include one or more features to aid in aligning the electrical connector 224 to the corresponding electrical connector of the sensor hub 106. For example, the cavity 203 can include walls 222 adjacent the electrical connector 224, the walls 222 configured to aid in releasably connecting the electrical connector 224 with the corresponding electrical connector 151 of the sensor hub 106.

In some implementations, the charging station 200 includes a communication module that comprises an NFC antenna (for example, within a front side of the body 206) for recognizing and/or communicating with other electronic device(s) and/or sensor(s). In some implementations, the sensor hub 106 can automatically pair with and/or begin electrical communication with the charging station 200 when the sensor hub 106 is mechanically and electrically coupled to the charging station 200, such as by when the sensor hub 106 is seated within cavity 203 of the charging station 200 and the electrical connector 151 of the sensor hub 106 is operably connected with the electrical connector 224 of the charging station 200 (e.g., when the sensor hub 106 is docked with the charging station 200). The charging station 200 can charge the battery 165 of the sensor hub 106 when the sensor hub 106 is docked with the charging station 200. Further, and in some implementations, the sensor hub 106 can download data, such as physiological data from the subject 1, to the charging station 200 and/or to a server, another electronic device, the cloud, and/or a wireless or wired network via the charging station 200. In some implementations, the charging station 200 can update software of the sensor hub 106 when the sensor hub 106 is docked with the charging station 200.

In some implementations, the charging station 200 can be configured as an array such that it can releasably electrically and mechanically connect to more than one sensor hub 106 at a time. For example, the charging station 200 can be configured to have more than one cavity 203 configured to releasably electrically and mechanically connect to more than one sensor hub 106. In some implementations, the charging station 200 can be configured as a linear array of cavities 203 (e.g., an array of two, three, four, or more cavities). In some cases, the charging station 200 can be configured as an array of cavities 203 with one or more "rows" and one or more "columns." In some implementations, the charging station 200 can be configured to slidably receive the sensor hub 106 in a vertical orientation, such as shown in FIG. 7A. In some implementations, the charging station 200 can be configured to slidably receive the sensor hub 106 in an orientation other than vertical, such as at an angle to the vertical, sideways, horizontally, etc.

In some implementations, the charging station 200 can include a battery 106d configured as a backup battery for providing power/charge to a sensor hub 106 even if the power source that provides power to the charging station 200 is unavailable. Such a backup battery can be sized/rated and/or have a capacity to provide a partial charge, a full charge, two full charges, more than two full charges or any amount of a partial or a full charge to the sensor hub 106 in the case of a power outage.

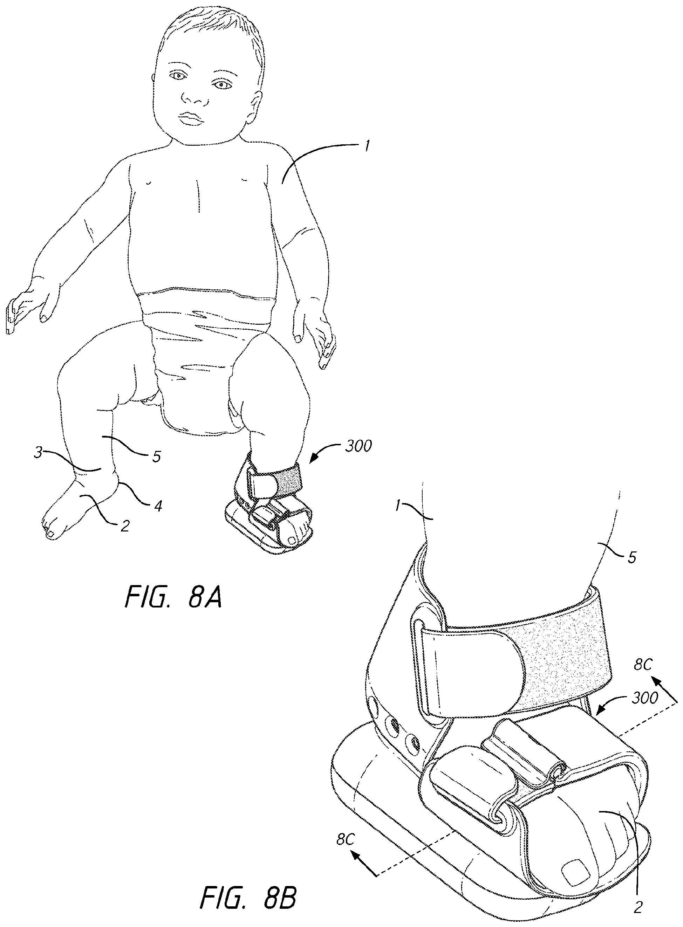

FIGS. 8A-8B illustrate perspective views of another implementation of a system 300 (which can also be referred to herein as a "wearable system," "wearable sensor system," or "wearable physiological sensor system") configured to be secured to the subject's foot 2 and measure at least one physiological parameter of the subject 1. The system 300 can be similar or identical to the system 100 in some or many respects. For example, the system 300 can have a wearable device 302, a sensor dock 304, and/or a sensor hub 306 that are similar or identical to the wearable device 102, the sensor dock 104, and the sensor hub 106 of the system 100, respectively in some or many respects. The system 300 can be secured to the subject's foot 2, ankle 3, heel 4, and/or lower leg 5 similar or identical to how the system 100 can be secured to the subject 1. Furthermore, the system 300 can include one or more emitters 304a in an emitter package 344, one or more detectors 304b in a detector package 346, and one or more temperature sensors 304c in a temperature sensor package 348 that are similar or identical to the one or more emitters 104a in emitter package 144, the one or more detectors 104b in detector package 146, and the one or more temperature sensors 304c in the temperature sensor package 148 of the system 100, respectively. The system 300 can include any of the features or components discussed with respect to FIG. 3 above. The system 300 can wirelessly communicate with one or more separate computing device(s), which can be for example, a patient monitor 10a and/or a mobile phone 10b, via any of a variety of wireless communication protocols such as any of those discussed herein with respect to the system 100. Furthermore, the system 300 can wirelessly transmit subject physiological data and/or physiological parameters to separate computing device(s) (such as patient monitor 10a and/or mobile phone 10b) as described herein with respect to the system 100.

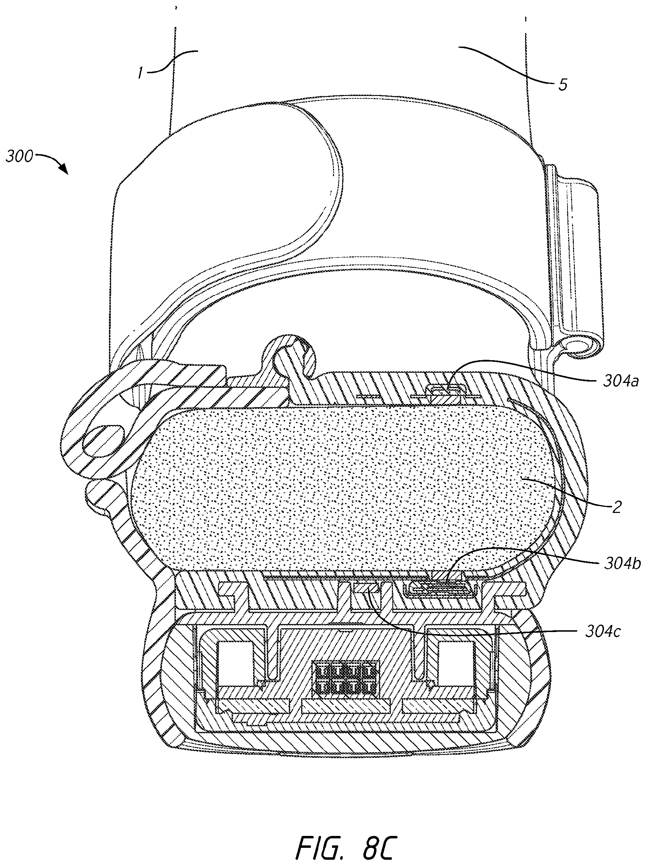

FIG. 8C illustrates a cross-section of the system 300 of FIGS. 8A-8B secured to the subject's foot 2. As shown, when secured to the subject's foot 2, the system 300 can operably position one or more emitters 304a and one or more detectors 304b at opposite sides of the subject's foot 2. Also shown, when secured to the subject's foot 2, the system 300 can operably position one or more temperature sensors 304c adjacent a bottom of the subject's foot 2.

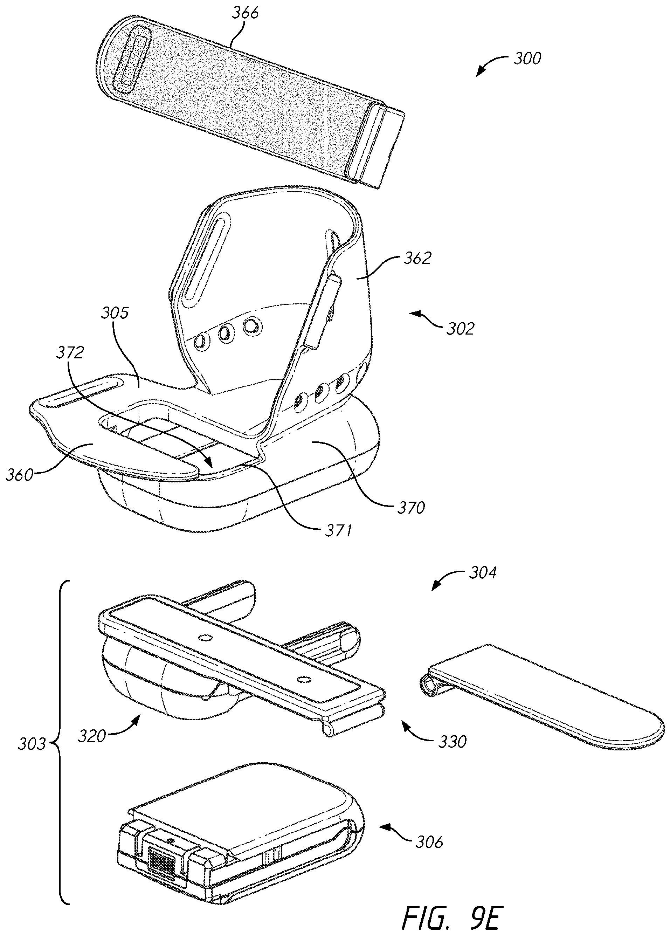

FIGS. 9A-9E illustrate various perspective views of the system 300 of FIG. 8A. The system 300 can include the wearable device 302. The wearable device 302 can be configured to receive and/or secure an electronic device including one or more sensors for monitoring information relating to physiological, motion, and/or location of the subject 1. For example, the wearable device can be configured to receive and/or secure a sensor component 303 (which may also be referred to herein as a "sensor assembly") or a portion thereof, as described further herein. Such sensor component 303 can include a sensor dock 304 and a sensor hub 306. In some implementations, the system 100 can include the wearable device 302, the sensor dock 304, and the sensor hub 306. As shown in FIGS. 9A-9C, the wearable device 302, the sensor dock 304, and the sensor hub 306 can form a unitary structure configured to be secured to the subject's foot. FIG. 9D illustrates the wearable device 302 and sensor dock 304 connected to one another and the sensor hub 306 disconnected from the wearable device 302 and sensor dock 304. FIG. 9E illustrates an exploded view of system 300, illustrating the wearable device 302, sensor dock 304, and sensor hub 306 separated from one another. Although the figures illustrate implementations of the system 300 in which the wearable device 302, sensor dock 304, and sensor hub 306 are removably connectable to one another, various ones of these components may be integrally formed with one another. For example, in some variants, the wearable device 302 and sensor dock 304 are integrally formed and are removably connectable to the sensor hub 306. As another example, in some variants, the sensor dock 304 and sensor hub 306 are integrally formed and are removably connectable to the wearable device 302. As another example, in some variants, the wearable device 302, sensor dock 304, and sensor hub 306 are integrally formed with one another. Implementations of the system 300 in which wearable device 302 is removably connectable from sensor dock 304 and/or sensor hub 306 can advantageously allow for a wearable device 302 of various sizes (e.g., small, medium, and large) and/or shapes to be utilized with the system 300, for example, so as to accommodate various sizes and/or shapes of a subject's foot 2, ankle 3, heel 4, and/or lower leg 5. In this way, the system 300 can be customized to a subject 1 by selecting an appropriately configured wearable device 302 while allowing for all other aspects of the system 300, such as the sensor dock 304 and sensor hub 306, to remain the same and/or be universal across subjects. In some implementations the sensor dock 304 and the sensor hub 306 can advantageously be configured to removably connect from each other (e.g., so that the sensor hub 306 can be recharged separate of the sensor dock 304). In some implementations, for example as shown in FIG. 9E, the sensor dock 304 and the sensor hub 306 form the sensor component 303 that can be removably connected to the wearable device 302.

As mentioned above, FIG. 9E illustrates an exploded view of system 300. The wearable device 302 can have a base 360 and a wall 362. The wall 362 can extend from the base 360. For example, the wall 362 can extend from a periphery of the base 360. In some implementations, the wall 362 can extend around a portion of a perimeter edge of the base 360. The base 360 and the wall 362 can form a main body 305 of the wearable device 302. In some implementations, the wearable device 302 can have a main body 305 and a holder 370 extending outward from the main body 305. The main body 305 can include the base 360, an opening 371 in the base 360, and the wall 362 extending from the base 360. In some implementations, the main body 305 additionally includes a wearable device strap 366 (which can also be referred to herein as an "additional strap") configured to connect to and extend from the wall 362. The base 360 (which can also be referred to herein as "bottom portion") of the wearable device 302 can be configured to contact a bottom portion of the subject's foot 2 when the system 300 is in use. For example, the base 360 can be configured to contact a heel, an arch, a ball, and/or one or more toes of the subject's foot 2. The opening 371 in the base 360 can be configured to be positioned adjacent a bottom portion of the subject's foot 2 when the system 300 is in use. For example and as shown, the opening 371 can extend through the base 360 and be positioned such that it underlies the ball of the subject's foot 2 when the wearable device is secured to the subject's foot 2. The holder 370 extending outward from the main body 305 can, as shown, extend from the main body 305 adjacent the opening 371 of the base 360 and away from the bottom portion of the subject's foot 2 when the system 300 is in use. The holder 370 can include a cavity 372 configured to removably receive the sensor dock 304 and the sensor hub 306, for example, when the sensor hub 306 is connected to the sensor dock 304. Further, the opening 371 can open into the cavity 372 of the holder 370 as shown. The sensor dock 304 can have a main body 320 and a sensor strap 330 (also referred to herein as "strap") connected to and extending from the main body 320. The sensor strap 330 of the sensor dock 304 can operably position one or more emitters 304a and one or more detectors 304b of the system 300 and can be configured to be positioned at least partially within and extend outward from the opening 371 when the sensor dock 304 is connected to the holder 370. The above and other aspects of the system 300 are discussed further below.

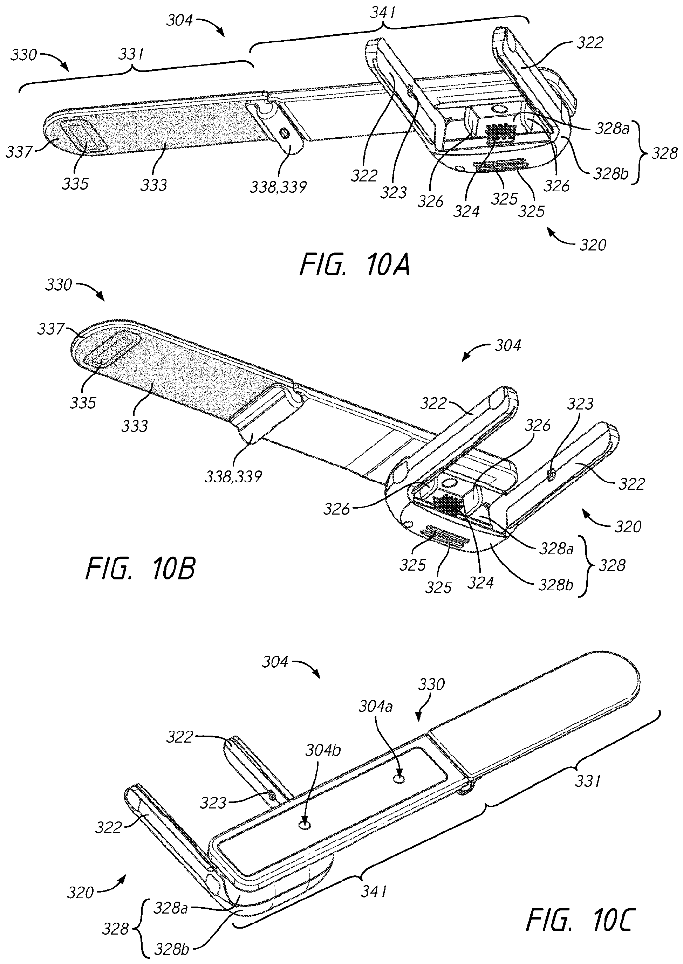

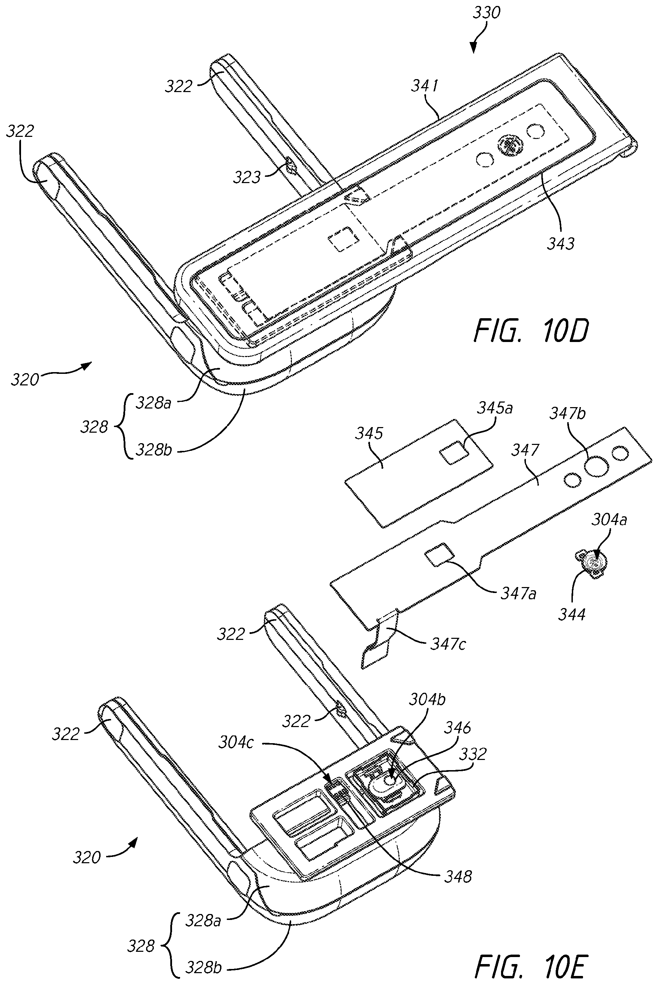

FIGS. 10A-10F illustrate various perspective views of the sensor dock 304 of the system 300. As shown, the sensor dock 304 can have a main body 320 and a sensor strap 330 (which also may be referred to herein as "strap") connected to and extending outward from the main body 320. The main body 320 can include a base 328 with arm(s) 322 extending outward from the base 328. The sensor strap 330 can include the one or more emitters 304a and the one or more detectors 304b and can be configured to secure the system 300 to the subject's foot 2 as described further herein (for example, alone or in combination with wearable device strap 366 of wearable device 302). The sensor strap 330 can be configured to receive emitter package 344 to operably position the one or more emitters 304a. Similarly, the sensor strap 330 can be configured to receive detector package 346 to operably position the one or more detectors 304b. The one or more emitters 304a in emitter package 344 and the one or more detectors 304b in detector package 346 can be in electrical communication with an electrical connector 324 of the sensor dock 304 via a circuit layer 347 disposed within the sensor strap 330 and a portion 347c of the circuit layer 347 that extends from the circuit layer 347 in the sensor strap 330 to the electrical connector 324 (shown in FIG. 10E).

The electrical connector 324 can be configured to releasably electrically connect the sensor dock 304 (and therefore the one or more emitters 304a and the one or more detectors 304b) to the sensor hub 306. The sensor dock 304 can also include features for mechanically engaging/connecting with the sensor hub 306. For example and as shown, the arm(s) 322 extending from the base 328 can be configured to releasably mechanically engage/connect with the sensor hub 306. In some implementations, when the sensor hub 306 is mechanically engaged/connected with the arm(s) 322 of the sensor dock 304, an electrical connector of the sensor hub 306 can releasably mechanically and electrically engage/connect with the electrical connector 324 of the sensor dock 304.

As shown in FIGS. 10A-10F, in some implementations the main body 320 of the sensor dock 304 has a length and/or a width that are greater than a height of the sensor dock 304. In some implementations, the sensor dock 304 includes two arm(s) 322. The arm(s) 322 of the sensor dock 304 can extend from the base 328 in the same direction so as to form a generally U-shaped structure. The arm(s) 322 can be generally parallel to each other, such that a gap is formed between the arm(s) 322. Such a gap can be, for example, sized to accommodate the sensor hub 306 and/or at least a portion of the sensor hub 306. In some implementations, the arm(s) 322 are the same length. Furthermore, the arm(s) 322 can mirror each other in size, shape, and other features. In some implementations, the sensor dock 304 can include one or more retaining features configured to engage the sensor hub 306. For example, each of the arm(s) 322 of the sensor dock 304 can include a protrusion 323 configured to engage with the sensor hub 306 to allow the sensor dock 304 to connect to the sensor hub 306. The protrusion(s) 323 can be disposed along an inner surface of each of the arm(s) 322, such that they face towards the sensor hub 306 when the sensor hub 306 is connected to the sensor dock 304. The arm(s) 322 and the protrusion(s) 323 of the sensor dock 304 can be configured similar or the same as the arm(s) 122 and the protrusion(s) 123 of the sensor dock 104. Furthermore, the sensor dock 304, including the arm(s) 322 and the protrusion(s) 323, can interact with the sensor hub 306, which can be similar or the same as the sensor hub 106, similar or the same as how the sensor dock 104 can interact and/or connect with the sensor hub 106 as described herein.

As discussed above and as shown in FIGS. 10A-10B, the sensor dock 304 can include an electrical connector 324. The electrical connector 324 can be configured to releasably electrically and mechanically connect to a corresponding electrical connector of the sensor hub 306, such that when connected to each other, the sensor hub 306 is placed in electrical communication with the one or more emitters 304a and the one or more detectors 304b of the system 300. The electrical connector 324 can be disposed at an inner portion of the base 328 of the sensor dock 304, such that it faces the sensor hub 306 when the sensor hub 306 is connected to the sensor dock 304. In some implementations, the sensor dock 304 can include one or more features to aid in aligning the electrical connector 324 to the corresponding electrical connector of the sensor hub 306. For example, the sensor dock 304 can include walls 326 positioned adjacent the electrical connector 324, and the walls 326 can be configured to aid in releasably connecting the electrical connector 324 with the corresponding electrical connector of the sensor hub 306. The releasable electrical connection between the sensor dock 304 and the sensor hub 306 can be the similar or the same as the releasable electrical connection between the sensor dock 104 and the sensor hub 106 as described herein.

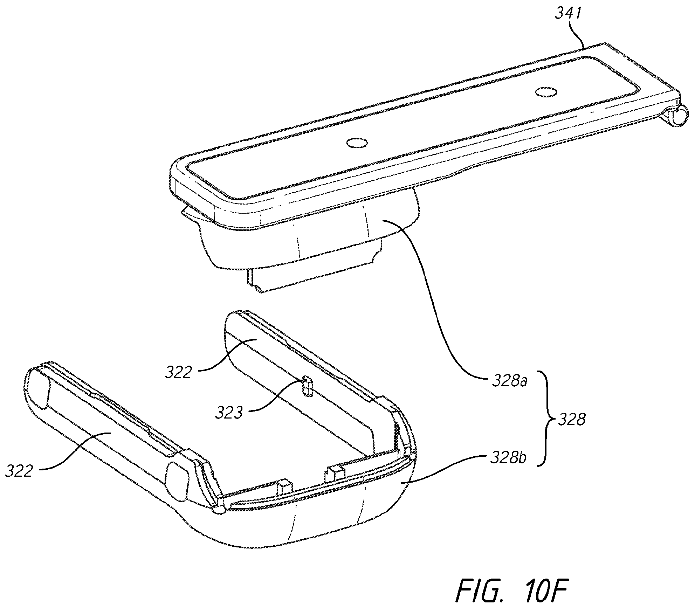

The base 328 of the sensor dock 304 can include a top portion 328a and a bottom portion 328b (shown, for example, in FIG. 10F) that can be integrally formed (or alternatively, separable from one another). The top portion 328a can include the electrical connector 324 and the walls 326, and the bottom portion 328b can include the arm(s) 322. The main body 320 of the sensor dock 304 can be configured to connect to the holder 370 of the wearable device 302. For example, the main body 320 of the sensor dock 304 including the base 328 and arm(s) 322 can be sized and/or shaped to fit within at least a portion of the cavity 372 of the holder 370.

The sensor dock 304 can include one or more features for aiding in gripping and/or holding the sensor dock 304, such as for gripping and/or holding the sensor dock 304 when connecting and/or disconnecting sensor hub 306 to the sensor dock 304. For example, the sensor dock 304 can include one or more ribs 325 disposed on a portion of the base 328 (e.g., the bottom portion 328b) of the sensor dock 304, the ribs 325 configured to aid in gripping and/or holding the sensor dock 304. The ribs 325 can be similar or the same as the ribs 125 of the sensor dock 104. Alternatively, or in addition, in some implementations the sensor dock 304 can include other features configured to aid in gripping and/or holding the sensor dock 304, such as bumps, a roughened surface texture, etc.

As discussed above and as shown in FIGS. 10A-10F, the sensor dock 104 can include the sensor strap 330 that connects to and extends outward from the main body 320 of the sensor dock 304. As shown and in some implementations, the sensor strap 330 connects to a top of the main body 320 of the sensor dock 304 such that the sensor strap 330 is raised (e.g., forms a raised surface) relative to the rest of the sensor dock 304. Furthermore, the sensor strap 130 can be disposed at an end of the main body 320 adjacent the electrical connector 324. Additionally, the sensor strap 330 can extend from the main body 320 in a direction substantially perpendicular to the arm(s) 322 of the sensor dock 304. The sensor strap 130 can be configured to fit within and extend from the opening 371 in the wearable device 302 when the main body 320 of the sensor dock 304 is connected to the holder 370 of the wearable device 302 (for example, when the main body 320 of the sensor dock 304 is disposed within the cavity 372 of the holder 370). A portion of the sensor strap 330 that fits within the opening 371 can be configured to form a substantially flush surface (e.g., a substantially coplanar surface) with the base 360 of the wearable device 302 for receiving a bottom portion of the subject's foot 2. Additionally, a portion of the sensor strap 330 that extends from the opening 371 can be configured to wrap around at least a portion of the subject's foot and secure the wearable device 302 to the subject's foot 2.

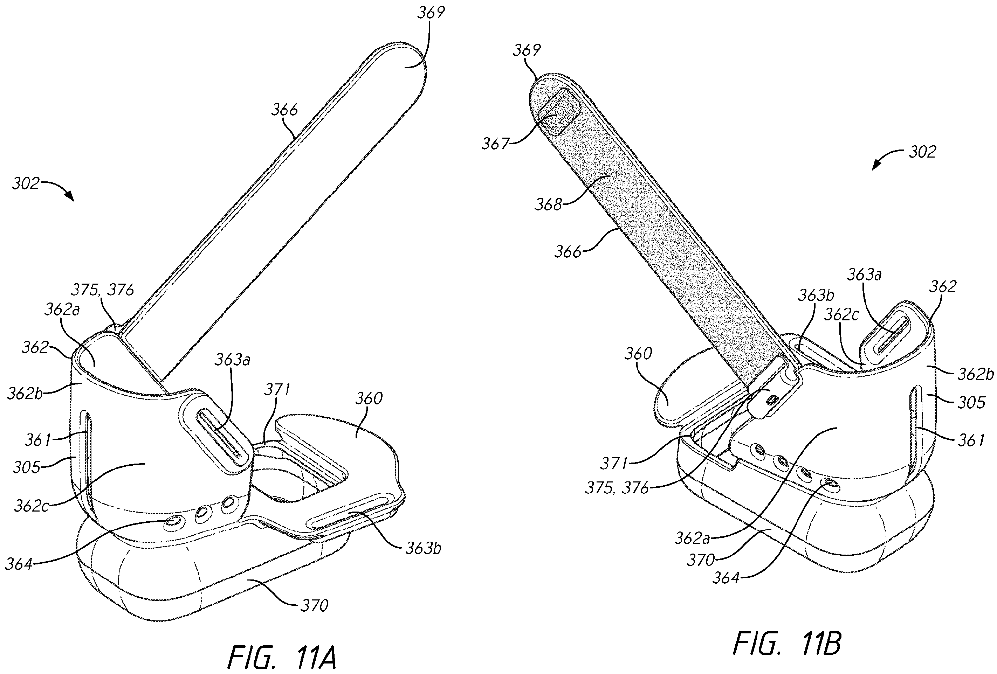

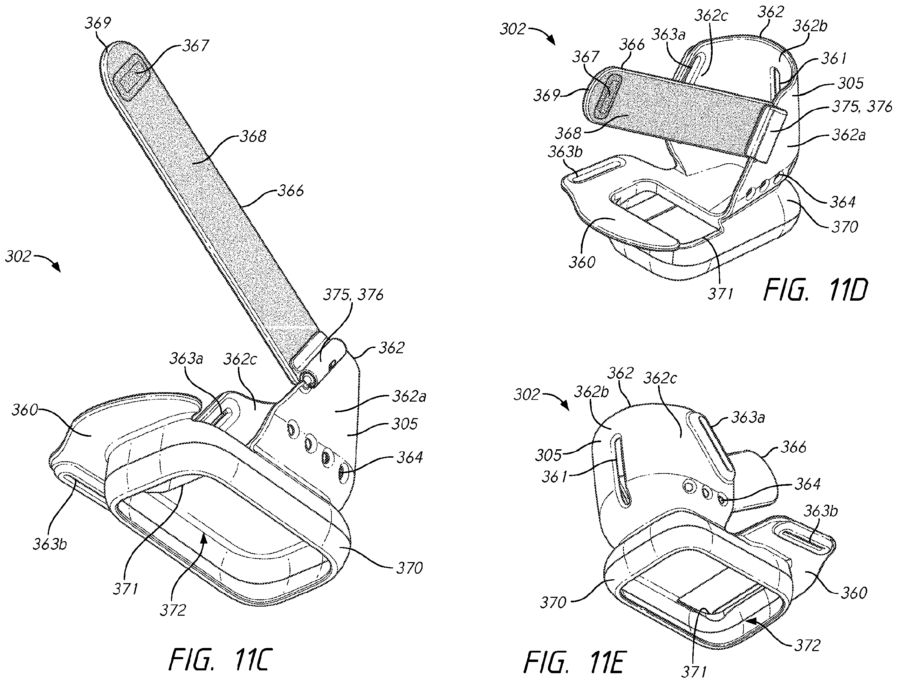

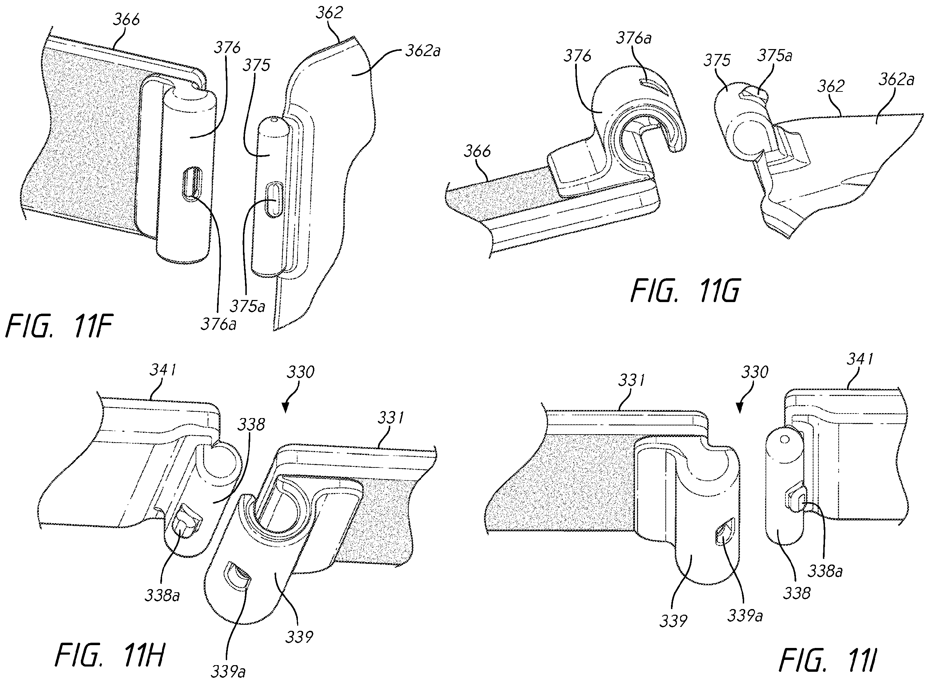

Strap 330 can include a sensor section 341 and a securement section 331 as shown in FIGS. 10A and 10C. The sensor section 341 can connect to and extend outward from the main body 320 of the sensor dock 304, while the securement section 331 can be disposed at an end 337 of the sensor strap 330 opposite the sensor section 341. The securement section 331 can include one or more features for securing the system 300 to the subject's foot 2. For example and as shown, the securement section 331 can include loop(s) 333 and/or hook(s) 335 that can interact with one or more features of the wearable device 302 and/or each other as described later herein for securing the system 300 to the subject's foot 2, although other methods of securement can be used. In some implementations, at least a portion of the strap 330 is stretchable. For example, at least a portion of the sensor section 341 and/or at least a portion of the securement section 331 can be configured to be stretchable. In some implementations, the sensor section 341 is more stretchable than the securement section 331. In some implementations, the sensor section 341 and the securement section 331 can be configured to releasably connect with each other. For example, the sensor section 341 can include a connector 338 and the securement section 331 can include a connector 339 each configured to releasably connect with each other. Details of the connectors 338 and 339 are discussed further with respect to FIGS. 11H-11I later herein. In some variants, the sensor section 341 and the securement section 331 form a unitary strap 330.

FIGS. 10C-10F illustrate perspective views of the sensor dock 304 that progressively show the various aspects, components, and/or features that the sensor dock 304, in particular the sensor strap 330, can include. As shown in FIG. 10D, the sensor strap 330 can include a cover 343 (which is shown as transparent so some of the internal aspects of the sensor strap 341 can be seen, and which may also be referred to as a "cover plate") configured to cover the electrical circuitry of the sensor strap 330, such as the circuit layer 347. The cover 343 can fit into a recess of the sensor strap 130, such that the cover 343 and the surface of the sensor strap 330 adjacent the cover 343 form a substantially flush surface. The cover 343 can include openings configured to overlie the one or more detectors 304b in detector package 346 and the one or more emitters 304a in emitter package 344 similar or the same as the openings 143a and 143b of the cover 143 described herein. In some implementations, such openings are covered by transparent material (for example, to prevent ingress of liquid therethrough. However, in alternative implementations, such openings are not covered. As shown in FIG. 10E (in which aspects of the sensor strap 341 have been removed from view), in addition to the cover 343, the sensor strap 330 can include a stiffener 345 configured to increase the stiffness of a portion of the sensor strap 330 that is positioned within the opening 371 of the wearable device 302 when the sensor dock 304 is connected to the holder 370. The stiffener 345 can be disposed below the cover 343 and can include an opening 345a configured to allow optical radiation to pass through (e.g., so as not to block optical radiation from being emitted by the one or more emitters 304a or optical radiation from being received by the one or more detectors 304b). Also shown in FIG. 10E is the circuit layer 347, which can be disposed below the cover 343 and the stiffener 345 and positioned within the sensor strap 330. As mentioned above, the circuit layer 347 can electrically connect the one or more emitters 304a in emitter package 344 and the one or more detectors 304b in detector package 346 to the electrical connector 324 of the sensor dock 304. In some implementations, the circuit layer 347 also electrically connects the one or more temperature sensors 304c (which can be disposed in temperature sensor package 348) of the sensor dock 304 to the electrical connector 324. The circuit layer 347 can be configured as a flexible circuit that can bend freely with the sensor strap 330. Similar to the openings in the cover 343, the circuit layer 347 can include an opening 347a and an opening 347b configured to overlie the one or more detectors 304b and one or more emitters 304a, respectively, and allow optical radiation to be received and/or emitted therethrough.

With continued reference to FIG. 10E, shown are the emitter package 344, the detector package 346, and the one or more temperature sensors 304c in a temperature sensor package 348 of the sensor strap 330. The one or more emitters 304a in emitter package 344 can be positioned within a first portion of the sensor strap 330 that is outside of and/or spaced away from the opening 371 of the wearable device 302 when the sensor dock 304 is connected to the holder 370. The one or more detectors 304b in detector package 346 and the one or more temperature sensors 304c in temperature sensor package 348 can be positioned within a second portion of the sensor strap 330 that can be positioned within the opening 371 of the wearable device 302 when the sensor dock 304 is connected to the holder 370. The one or more temperature sensors 304c in temperature sensor package 348 can be positioned proximate to but spaced from the one or more detectors 304b in detector package 346. The sensor section 341 of the sensor strap 330 can include both of such first portion and second portion described above.

The one or more emitters 304a in emitter package 344 can be aligned with, for example vertically aligned, and/or aimed towards the one or more detectors 304b in detector package 346 when the sensor strap 330 is wrapped around a top portion of the subject's foot 2 (for example, as shown in FIG. 8C). The temperature sensor(s) 304c in temperature sensor package 348 can be horizontally aligned with the detector package 346 and not vertically aligned with the emitter package 344 when the sensor strap 330 is wrapped around the top portion of the subject's foot 2 (also shown in FIG. 8C). In such positions and with the sensor strap 330 wrapped around the top portion of the subject's foot 2, the emitter package 344 can be operably positioned against and/or adjacent to tissue of the top of the subject's foot 2, and the detector package 346 and temperature sensor package 348 can be operably positioned against and/or adjacent to tissue of the bottom of the subject's foot 2 (e.g., tissue of the ball of the subject's foot 2). Thus, the detector package 346 can be operably positioned by the sensor strap 330 such that optical radiation emitted from the emitter package 344 can pass through and/or be attenuated by the tissue of the subject's foot 2 before being detected by the detector package 346. In some implementations, the locations of the one or more emitters 304a in emitter package 344 and the one or more detectors 304b in detector package 346 can be switched. In such implementations, and with the sensor strap 330 wrapped around the top portion of the subject's foot 2, the detector package 346 can be operably positioned against and/or adjacent to tissue of the top of the subject's foot 2, and the emitter package 344 can be operably positioned against and/or adjacent to tissue of the bottom of the subject's foot 2 (e.g., tissue of the ball of the subject's foot 2).

With continued reference to FIG. 10E, the sensor strap 330 can also include a detector shield 332. The detector shield 332 can at least partially enclose and/or surround the detector package 346 comprising the one or more detectors 304b. The detector shield 332 can be configured to inhibit, prevent, and/or reduce ambient light, stray light, and/or light emitted from the emitter package 344 that does not pass through tissue from being received by the detector package 346, which can advantageously improve the integrity of physiological parameter determination. Additionally, or alternatively, detector shield 332 can shield the detector package 346 against and/or with respect to electromagnetic noise. For example, in some implementations, the detector shield 332 can act as a Faraday cage or a shield to block electromagnetic fields. The sensor strap 330 can also include optical transmission material configured to direct optical radiation toward the detector package 346 after passing through tissue of the subject's foot 2. In some cases, the optical transmission material can include a lens. In some cases, the optical transmission material can include a diffuser configured to diffuse, spread out, disseminate, and/or scatter optical radiation attenuated by tissue prior to being received by the detector package 346. The optical transmission material can form a part of the detector package 346, or it can be configured to be positioned between the detector package 346 and tissue of the subject 1 when the system 300 is secured to the subject 1. In some implementations, the optical transmission material is disposed within the opening of the cover 343 overlying the detector package 346. Similarly, the sensor strap 330 can include optical transmission material configured to focus or diffuse optical radiation emitted from the emitter package 344. In some cases, the optical transmission material can include a lens. In some cases, the optical transmission material can include a diffuser configured to diffuse, spread out, disseminate, and/or scatter optical radiation emitted from the emitter package 344 prior to such optical radiation entering the subject's tissue. In some cases, this can permit optical radiation emitted from the emitter package 344 to pass through a greater amount of tissue and can facilitate more accurate determination of physiological parameters (such as any of those discussed herein). The optical transmission material can form a part of the emitter package 344, or it can be configured to be positioned between the emitter package 344 and tissue of the subject 1 when the system 300 is secured to the subject 1. In some implementations, the optical transmission material is disposed within the opening of the cover 343 overlying the emitter package 344.

Advantageously, at least a portion of the sensor strap 330 can be made of a pliable material. For example, at least a portion of the sensor strap 330 can be made of silicone, such as a medical grade and/or biocompatible silicone, a thermoplastic elastomer, such as a medical grade and/or biocompatible thermoplastic elastomer, and/or any biocompatible material and/or polymer that is pliable, flexible, stretchy, soft, and/or conformable. A pliable sensor strap 330 can advantageously position the emitter package 344 and the detector package 346 close to, against, and or adjacent to a portion of the subject's body, such as the tissue of the subject's foot 2, for optimal function of the system 300. For example, by conforming to the subject's foot 2, the sensor strap 330 can optimally position the emitter package 344 against, adjacent, and/or near to the subject's foot 2 such that optical radiation emitted from the emitter package 344 is directed to/through the subject's foot 2. By way of another example, by the sensor strap 330 conforming to the subject's foot 2, ambient and/or stray optical radiation and/or optical radiation not produced/emitted by the emitter package 344 can be reduced, eliminated, and/or prevented from being received by the detector package 346. Furthermore, a pliable sensor strap 330 can advantageously improve comfort for the subject 1 when the system 300 is secured to and/or worn by the subject 1. In some implementations, the sensor strap 330 and/or portions thereof can be rigid or semi-rigid. In some cases, the sensor strap 330 can be a composite material and/or a composite of rigid, semi-rigid, and/or pliable/conforming material.

In some implementations, the sensor strap 330 can be configured to inhibit, prevent, and/or reduce an amount of ambient light, stray light, and/or any optical radiation not emitted from the emitter package 344 from reaching the detector package 346. Additionally, or alternatively, the sensor strap 330 can be configured to inhibit, prevent, and/or reduce an amount of optical radiation emitted by the emitter package 344 that has not been attenuated by, reflected by, and/or passed through tissue of the subject from being received by the detector package 346. In some cases, the sensor strap 330 can be opaque and/or generally light blocking and/or have a light blocking coating. In some implementations, sensor strap 330 can be semi-transparent or transparent. In some implementations, the sensor strap 330 can include portions that are opaque and/or light blocking and portions that are semi-transparent and/or transparent.

FIGS. 11A-11E illustrate perspective views of the wearable device 302 of the system 300 of FIGS. 8A-8B in accordance with some implementations of this disclosure. The wearable device 302 can be configured to receive and support the foot 2, ankle 3, heel 4, and/or lower leg 5 of the subject 1. Furthermore, the wearable device 302 can be made of a resilient material, similar or the same as the wearable device 102 described herein. As discussed with respect to FIG. 9E, the wearable device 302 can have a main body 305 including the base 360, the opening 371 in the base 360, and the wall 362 extending from the base 360. Also discussed and in some implementations, the main body 305 additionally includes the wearable device strap 366 configured to connect to and extend from the wall 362. As shown in FIGS. 11A-11E, the wall 362 of the wearable device 302 can extend upward from a periphery of the base 360 and include a side portion 362a (which can also be referred to herein as a "sidewall portion"), a back portion 362b (which can also be referred to herein as a "back wall portion"), and a side portion 362c (which can also be referred to herein as a "sidewall portion") configured to wrap around and support portions of the subject's foot 2, such as side(s) of the subject's foot 2, the subject's ankle 3, the subject's heel 4, and/or the subject's lower leg 5. The wall 362 can be discontinuous such that it does not enclose the complete periphery of the base 360, leaving space for the sensor strap 330 to extend from the opening 371 in the base 360. Furthermore, the wall 362 can be discontinuous such that the toes of the subject's foot 2 are not enclosed. The wall 362 can have a variable height as it extends upward from the base. For example and as shown in FIGS. 11A-11E, the wall 362 can have a maximum height at the back portion 362b so as to surround and support the subject's heel 4, and a reduced height at side portions 362a and 362c. In some implementations, the main body 305 of the wearable device 302 can include a plurality of hole(s) 364 for venting and/or for tuning the resilience of the wearable device 302. For example, the wall 362 and/or the base 360 can have a plurality of hole(s) 364 therethrough. Furthermore and in some implementations, the main body 305 of the wearable device 302 can include an opening 361 located in the back portion 362b of the wall 362. The opening 361 can be configured to allow the wearable device 302 to adapt to the heel 4 of the subject's foot 2.

With continued reference to FIGS. 11A-11E, the wearable device 302 can also include one or more features for securing to the subject's foot 2. For example and as shown, the main body 305 of the wearable device 302 can include the wearable device strap 366 mentioned previously that can connect to and extend outward from the wall 362, as well as a strap slot 363a configured to interact with wearable device strap 366. The wearable device strap 366 can be configured to wrap over a portion of the subject's foot 2, ankle 3, and/or lower leg 5, have its end 369 placed through the strap slot 363a (which can also be referred to herein as an "opening"), and secure back upon itself via the interaction between hook(s) 367 and loop(s) 368 of the wearable device strap 366. The wearable device strap 366 can connect to and extend from side portion 362a of the wall 362 at a location adjacent where the wearable device 302 would receive the subject's ankle 3 if secured to the subject 1. Further, the wearable device strap 366 can connect to and extend from the side portion 362a at an acute angle with respect to the base 360 when viewed from a side of the wearable device 302. The strap slot 363a can comprise a slot through the side portion 362c of the wall 362 (e.g., a side of the wall 362 opposite from where from the wearable device strap 366 connect and extends from) configured to receive the wearable device strap 366 therethrough. In some implementations, the wearable device strap 366 can be configured to releasably connect with the wall 362. For example, the wearable device strap 366 can include a connector 376 and the wall 362 (e.g., side portion 362a) can include a connector 375 each configured to releasably connect with each other. Details of the connectors 376 and 375 are discussed further with respect to FIGS. 11F-11G later herein. In some implementations, the wearable device strap 366 is integrally formed with the wearable device 302.

The main body 305 of the wearable device 302 can also include one or more features for releasably securing to the sensor strap 330 when the sensor dock 304 is connected to the holder 370. For example and as shown, the base 360 can include a portion adjacent the opening 371 that extends out beyond where the bottom of the subject's foot 2 is received when the system 300 is worn that includes a strap slot 363b (which can be the same as or similar to the strap slot 363a, and which can also be referred to herein as an "opening") that can interact with the sensor strap 330 similar to or the same as how the strap slot 363a interacts with wearable device strap 366. To customize the fit of the system 300 to the subject's foot 2, the wearable device strap 366 and/or the sensor strap 330 can be wrapped over the subject's foot 2, the ends 369 and 337 of the straps placed through strap slots 363a and 363b, and the ends 369 and 337 secured back upon themselves via the interaction between hook(s) 367 and 335 and loop(s) 368 and 333, respectively. In some implementations, the portion of the base 360 comprising the strap slot 363b can deform when the sensor strap 330 is secured to the strap slot 363b, such as shown in FIGS. 8A-8B (e.g., it can bend and/or fold against the foot 2).

FIGS. 11F-11G illustrate perspective views of connector 375 of the wearable device strap 366 and connector 376 of the wall 362 of the system 300 in accordance with some implementations of this disclosure. As shown, the connector 375 can be in the form of an elongate cylinder with enclosed rounded ends and protrude lengthwise from the wall 362 (e.g., side portion 362a of wall 362). The connector 376 can be in the form of an elongate cylinder with an open end, a closed end, and an open side disposed at an end of the wearable device strap opposite end 369 and configured to slidably and releasably connect over the connector 375. To aid in securing the connectors 375 and 376 together, the connector 375 can include a ramp-like protrusion 375a configured to fit within a corresponding opening 376a in the connector 376. In some implementations, the connectors 375 and 376 can be swapped (e.g., the connector 375 can be positioned on the wearable device strap 366 and the connector 376 can be positioned on the wall 362). Alternatively, the connectors 375 and 376 can be omitted and the wearable device strap 366 can extend from the wall 362 (e.g., similar to wearable device strap 166).

FIGS. 11H-11I illustrate perspective views of connectors 338 and 339 of the sensor strap 330 of the system 300 in accordance with some implementations of this disclosure. As shown, the connector 338 can be in the form of an elongate cylinder with enclosed rounded ends and protrude lengthwise from an end of the sensor section 341 opposite of where the sensor section 341 connects to the main body of the sensor dock 304. The connector 339 can be in the form of an elongate cylinder with an open end, a closed end, and an open side disposed at the end of the securement section opposite the end 337 and configured to slidably and releasably connect over the connector 338. To aid in securing the connectors 338 and 339 together, the connector 338 can include a ramp-like protrusion 338a configured to fit within a corresponding opening 339a in the connector 339. In some implementations, the connectors 338 and 339 can be swapped (e.g., the connector 338 can be positioned on the securement section 331 and the connector 339 can be positioned on the sensor section 341 of the sensor strap 330). Alternatively, the connectors 338 and 339 can be omitted and the securement section 331 can extend from the sensor section 341 as a unitary body (e.g., similar to sensor strap 130).

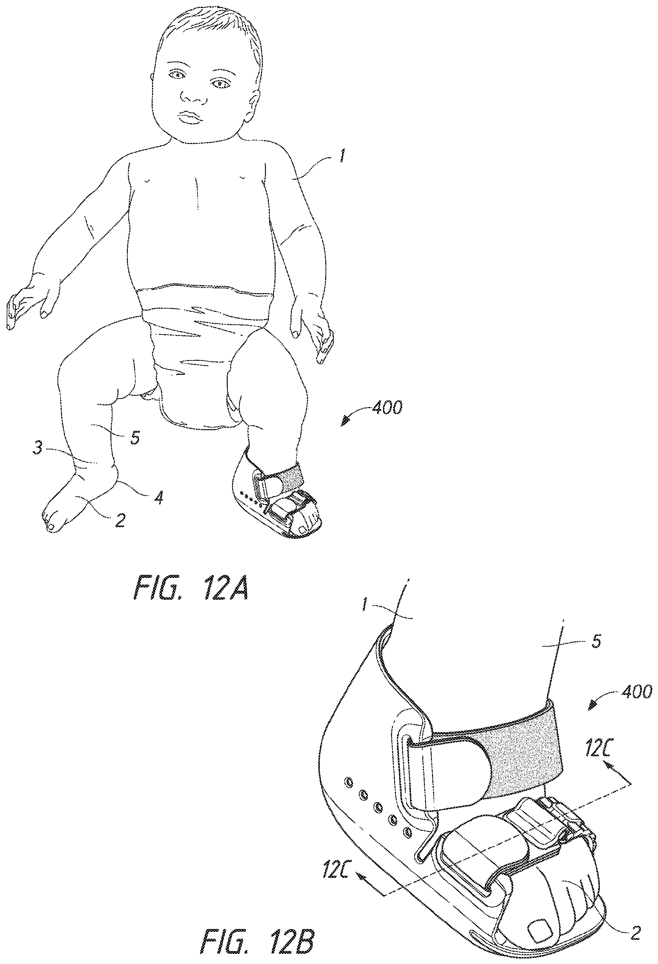

FIGS. 12A-12B illustrate perspective views of another implementation of a system 400 (which can also be referred to herein as a "wearable system," "wearable sensor system," or "wearable physiological sensor system") configured to be secured to the subject's foot 2 and measure at least one physiological parameter of the subject 1. The system 400 can have similar and/or the same features, aspects, functionality, and/or components as any of the systems described herein, such as systems 100, 300 and/or any variants thereof. For example, the system 400 can have a wearable device 402 configured the same as or similar to the wearable device 102 and/or the wearable device 302 in some or many respects. As another example, the system 400 can include a sensor hub 404 and a sensor strap 430, which can be referred to as a sensor component 403, configured similar to the sensor component 103 and/or the sensor hub 106, sensor dock 104, and sensor strap 130, and/or configured similar to the sensor component 303 and/or the sensor hub 306, sensor dock 304, and sensor strap 330 (e.g., the sensor hub 404 and the sensor strap 430 can combine any and/or all aspects of the sensor hubs 106, 306, sensor docks 104, 304, and sensor straps 130, 330, and/or sensor assemblies 103, 303). In contrast to the separate sensor docks 104, 304 and sensor hubs 106, 306, in this implementation the system 400 can include the sensor hub 404 with sensor strap 430, and the sensor hub 404 and the sensor strap 430 can include any of the functionality described with respect to the sensor assemblies 103 and 303 of systems 100 and 300. The system 400 can be secured to the subject's foot 2, ankle 3, heel 4, and/or lower leg 5 similar or identical to how the systems 100, 300 can be secured to the subject 1. Furthermore, the system 400 can include one or more emitters 404a in an emitter package, one or more detectors 404b in a detector package, and one or more temperature sensors 404c in a temperature sensor package that are similar or identical to the one or more emitters 104a, 304a in emitter packages 144, 344, the one or more detectors 104b, 304b in detector packages 146, 346, and the one or more temperature sensors 104c, 304c in the temperature sensor packages 148, 348 of the systems 100, 300, respectively. The system 400 can include any of the features or components discussed with respect to FIG. 3 above. The system 400 can wirelessly communicate with one or more separate device(s), which can be for example, a patient monitor 10a, a mobile phone 10b, a camera, a hub, or any other separate device(s) described herein via any of a variety of wireless communication protocols such as any of those discussed herein with respect to the systems 100, 300. Furthermore, the system 400 can wirelessly transmit subject physiological data and/or physiological parameters to separate device(s) (such as patient monitor 10a, mobile phone 10b, a camera, a hub, or other separate device(s)) as described herein with respect to the systems 100, 300.

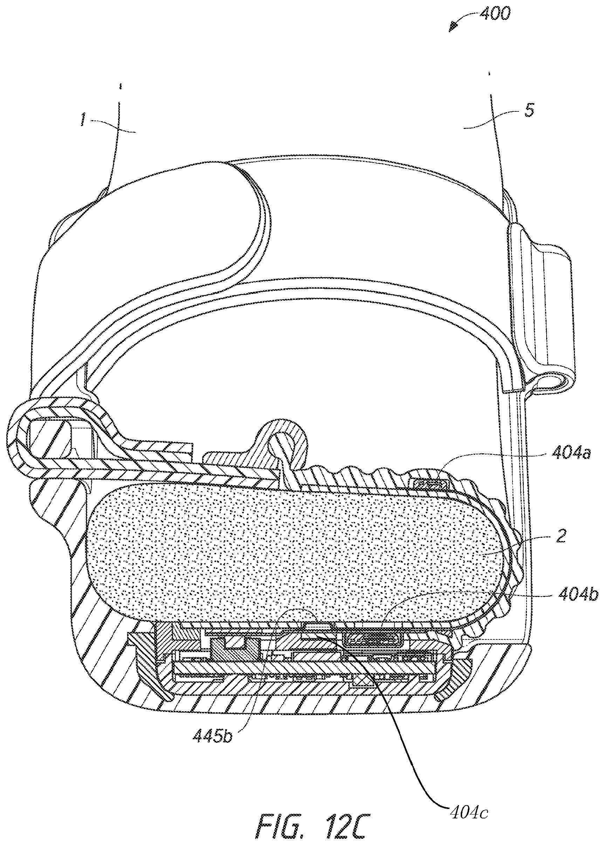

FIG. 12C illustrates a cross-section of the system 400 of FIGS. 12A-12B secured to the subject's foot 2. As shown, when secured to the subject's foot 2, the system 400 can operably position one or more emitters 404a and one or more detectors 404b at generally opposite sides of the subject's foot 2. For example, the system 400 can position the one or more emitters 404a and the one or more detectors 404b such that at least some of the optical radiation emitted by the one or more emitters 404a passes through tissue of the subject 1 before being detected by the one or more detectors 404b. The system 400 can position the one or more emitters 404a adjacent a top and/or adjacent a side of the subject's foot 2 and the one or more detectors 404b adjacent a bottom of the subject's foot 2. In other words, the one or more emitters 404a and the one or more detectors 404b do not need to be vertically aligned with one another and on different sides of the subject's foot in order for the system 400 to operate. In some implementations, the positioning of the one or more emitters 404a and the one or more detectors 404b can be reversed. In some implementations, at least a portion of the sensor strap 430 and/or at least a portion of the sensor hub 404 operably positions the one or more emitters 404a and/or the one or more detectors 404b as described. In some implementations, at least a portion of the sensor component 403 operably positions the one or more emitters 404a and/or the one or more detectors 404b as described. Also shown in FIG. 12C, the system 400 can operably position a thermally conductive probe 445b configured to transmit thermal energy adjacent a bottom of the subject's foot (e.g., such that it contacts skin of the subject 1) to transmit thermal energy from the bottom of the subject's foot toward the temperature sensor 404c.

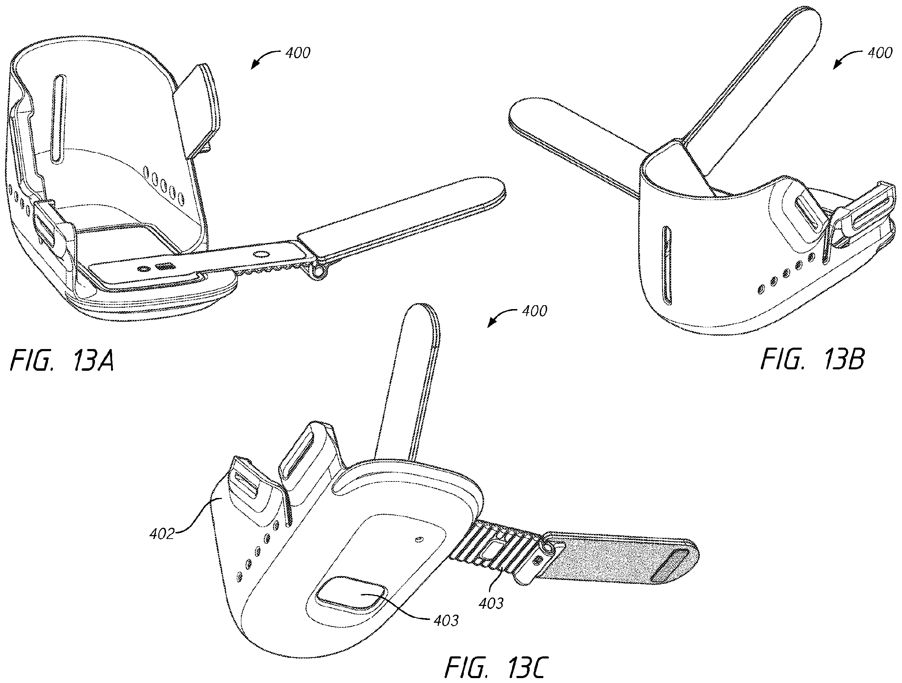

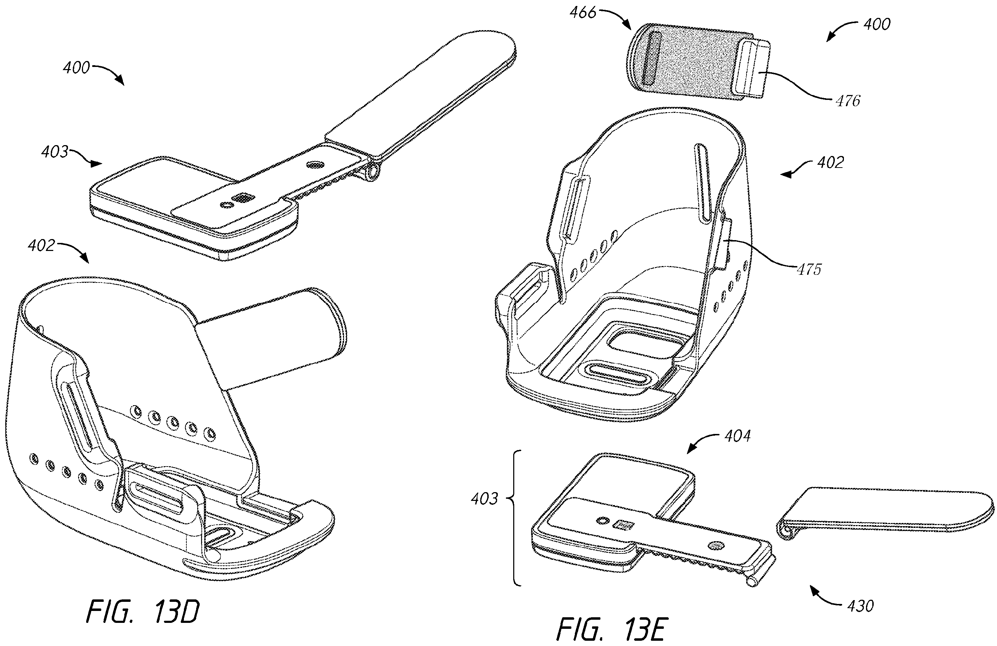

FIGS. 13A-13E illustrate various perspective views of the system 400 of FIGS. 12A-12B. The system 400 can include the wearable device 402. The wearable device 402 can be configured to receive and/or secure an electronic device including one or more sensors for monitoring information relating to physiological, motion, and/or location of the subject 1. For example, the wearable device can be configured to receive and/or secure the sensor component 403 (which may also be referred to herein as a "sensor assembly") or a portion thereof, as described further herein. Such sensor component 403 can include the sensor hub 404 and the sensor strap 430. In some implementations, the system 400 can include the wearable device 402, the sensor hub 404 and the sensor strap 430. As shown in FIGS. 13A-13C, the wearable device 402 and the sensor component 403 can form a unitary structure configured to be secured to the subject's foot. FIG. 13D illustrates the sensor component 403 disconnected from the wearable device 402. FIG. 13E illustrates an exploded view of system 400. The wearable device 402 can have a wearable device strap 466 configured the same or similar to the wearable device strap 366 of the wearable device 302, and as shown in FIG. 13E, the wearable device strap 466 can be removably connectable to the wearable device 402 (although in some implementations, the wearable device strap 466 can be integrally formed with the wearable device 402). Also shown in FIG. 13E, the sensor component 403 can include the sensor hub 404 and the sensor strap 430, with at least a portion of the sensor strap (e.g., a securement section) configured to be removably connectable thereto. Although the figures illustrate implementations of the system 400 in which the wearable device 402 and the sensor hub 404 and the sensor strap 430 are removably connectable to one another (or, in other words, where the wearable device 402 and the sensor component 403 are removably connectable to one another), various ones of these components may be integrally formed with one another. For example, in some variants, the wearable device 402 and sensor hub 404 are integrally formed and are removably connectable to the sensor strap 430 or at least a portion thereof. As another example, in some variants, the sensor hub 404 and the sensor strap 430 are integrally formed and are removably connectable to the wearable device 402. As another example, in some variants, the wearable device 402, the sensor hub 404, and sensor strap 430 are integrally formed with one another (or, in other words, the wearable device 402 and the sensor component 403 are integrally formed with one another). Implementations of the system 400 in which wearable device 402 is removably connectable from sensor hub 404 and/or sensor strap 430 can advantageously allow for a wearable device 402 of various sizes (e.g., small, medium, and large) and/or shapes to be utilized with the system 400, for example, so as to accommodate various sizes and/or shapes of a subject's foot 2, ankle 3, heel 4, and/or lower leg 5. In this way, the system 400 can be customized to a subject 1 by selecting an appropriately configured wearable device 402 while allowing for all other aspects of the system 400, such as the sensor hub 404 and sensor strap 430 or sensor component 403, to remain the same and/or be universal across subjects. In some implementations, for example as shown in FIG. 13E, the sensor hub 404 and the sensor strap 430 form the sensor component 403 that can be removably connected to the wearable device 402. In some implementations, at least a portion of the sensor strap 430 (e.g., a securement portion) and/or the wearable device strap 466 can come in various sizes and/or lengths (e.g., small, medium, large) to advantageously provide a customized fit with any of the various sizes of the wearable device 402.

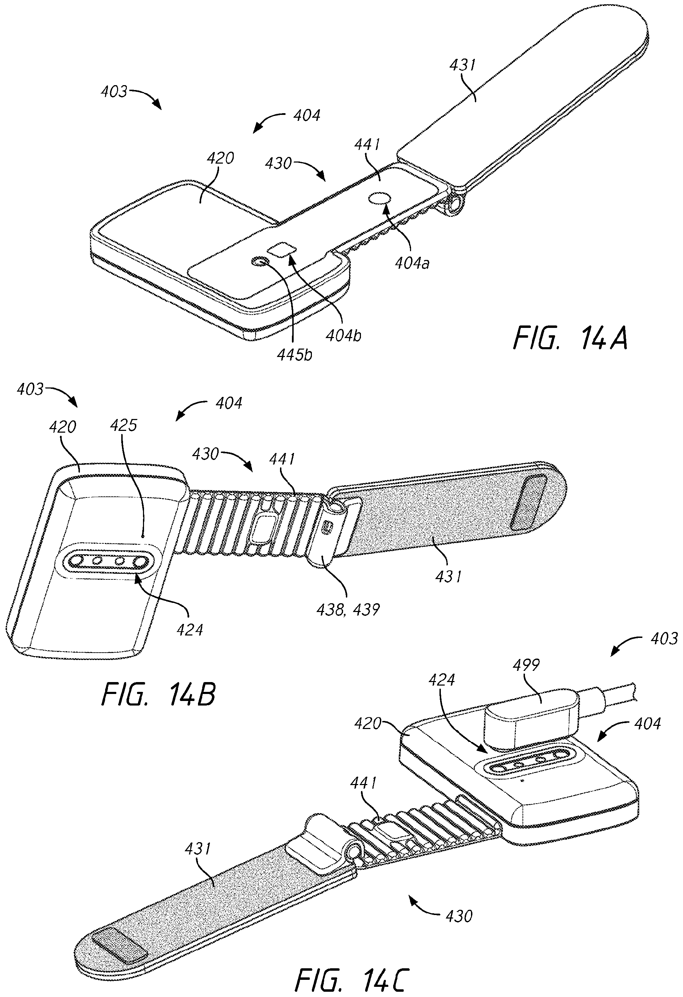

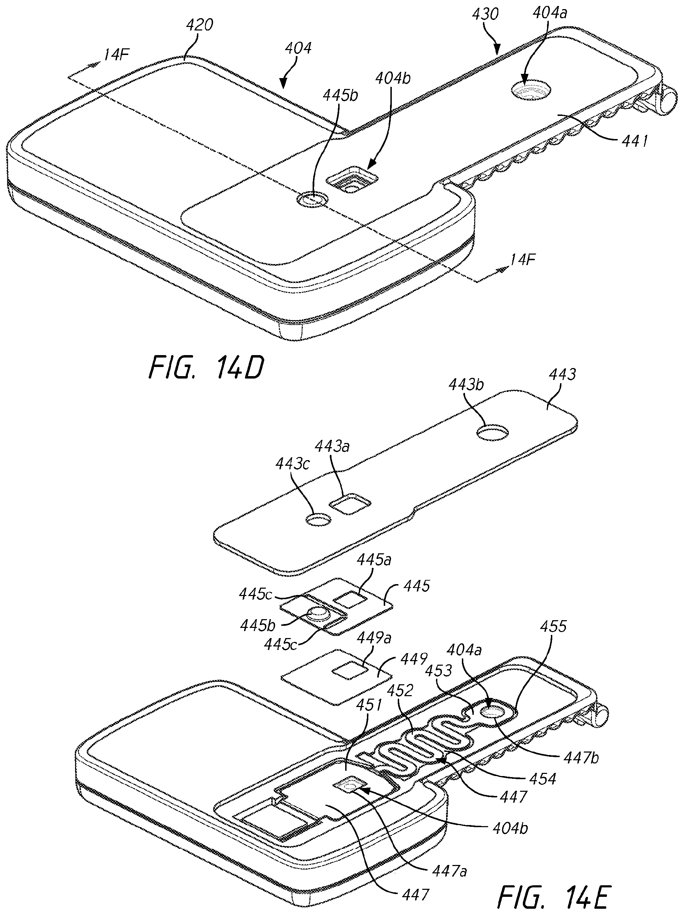

FIGS. 14A-14G illustrate various perspective views of the sensor component 403 of the system 400 and/or components/aspects thereof. As shown, the sensor component 403 can include a sensor hub 404 having a main body 420 and a sensor strap 430 (which also may be referred to herein as "strap") connected to and extending outward from the main body 420. The main body 420 can include a generally rounded rectangular housing having a top, sides, and a bottom. In some implementations, the main body 420 of the sensor hub 404 has a length and/or a width that are greater than a height of the sensor hub 404. The sensor strap 430 can include the one or more emitters 404a and/or the one or more detectors 404b and can be configured to secure the system 400 to the subject's foot 2 as described herein (for example, alone or in combination with wearable device strap 466 of wearable device 402). The sensor strap 430 can be the same or similar, or include any of the functionality and/or features of the sensor straps 130 and/or 330 described herein. The sensor strap 430 can be configured to operably position the one or more emitters 404a. Similarly, the sensor strap 430 can be configured to operably position the one or more detectors 404b. In some implementations, the sensor hub 403 is configured to operably position the one or more detectors 404b. Furthermore, in some implementations wherein the one or more detectors 404b are positioned by the sensor strap 430 (not shown), the one or more emitters 404a can be operably positioned by the sensor hub 404. The one or more emitters 404a and the one or more detectors 404b can be in electrical communication with one or more processors of the sensor hub 404 via a circuit layer 447 disposed at least partially within the sensor strap 430 and at least partially within the sensor hub 404 (for example, as shown in FIG. 14E). The sensor strap 430 can have a top that sits substantially flush (e.g., substantially coplanar) with the main body 420 (e.g., with the top of the main body 420) of the sensor hub 404. Additionally, the sensor strap 430 can extend from the main body 420 in a direction substantially perpendicular to the main body 420.

The sensor hub 404 can include an electrical connector 424 configured to releasably connect to a charger 499 to provide power to the sensor hub 404 and/or the sensor strap 430 (e.g., the sensory assembly 403) and any rechargeable batteries therein. In some implementations, the charger 499 can releasably connect to the electrical connector 424 of the sensor hub 404 with the aid of one or more magnets. In some implementations (not shown), the charger 499 can connect to an electrical port of the sensor hub 404 (e.g., a micro USB-C port or similar). The electrical connector 424 can be disposed at the bottom of the sensor hub 404. The sensor hub 404 can also include a status indicator 425 configured to indicate a status of the sensor hub 404, of the sensor strap 430, and/or the sensor component 403. The status indicator 425 can be disposed at the bottom of the sensor hub 404 and can be configured similar or the same as any of the status indicators described herein.

Strap 430 can include a sensor section 441 and a securement section 431 as shown in at least FIGS. 14A-14C. The sensor section 441 and the securement section 431 can be similar to or the same as the sensor section 341 and the securement section 331 of the sensor strap 330 described herein. The sensor section 441 can connect to and extend outward from the main body 420 of the sensor hub 404, while the securement section 431 can be disposed at an end of the sensor strap 430 opposite the sensor section 441. The securement section 431 can include one or more features for securing the system 400 to the subject's foot 2, similar or the same as the securement section 331 of the sensor strap 330. In some implementations, the sensor section 441 and the securement section 431 can be configured to releasably connect with each other, for example, via connectors 438, 439 which can be similar or identical to connectors 338, 339 as described elsewhere herein. In some implementations, at least a portion of the strap 530 is stretchable. For example, at least a portion of the sensor section 541 and/or at least a portion of the securement section 531 can be configured to be stretchable. In some implementations, the sensor section 541 is more stretchable than the securement section 531.

FIGS. 14D-14G illustrate perspective views of the sensor hub 404 and sensor strap 430 that progressively show the various aspects, components, and/or features that the sensor hub 404 and sensor strap 430 can include. The sensor hub 404 and sensor strap 430 can include any or all of the features and/or functionality of the sensor hub 306, sensor dock 304, and sensor strap 330 described herein. As shown in FIG. 14D, the sensor hub 440 and the sensor strap 430 can include a cover 443 (which may also be referred to as a "cover plate") configured to cover at least some of the electrical circuitry of the sensor hub 404 and the sensor strap 430, such as the circuit layer 447. The cover 443 can be similar or the same as the cover 343 described herein. The cover 443 can fit into a recess of the sensor hub 404 and the sensor strap 430, such that the cover 443 and the surface of the sensor hub 404 and the sensor strap 430 adjacent the cover 443 form a substantially flush surface. The cover 443 can include openings configured to overlie the one or more detectors 404b and the one or more emitters 404a similar or the same as the openings 143a, 343a and 143b, 343b of the covers 143, 343 described herein. In some implementations, such openings are covered by transparent material (for example, to prevent ingress of liquid therethrough. However, in alternative implementations, such openings are not covered. As shown in FIG. 14E (in which aspects of the sensor strap 441 have been removed from view), in addition to the cover 443, the sensor hub 404 and/or strap 430 can include a stiffener 445 (which may be a plate made of metal, for example) configured to increase the stiffness of a portion of the sensor hub 404 and/or strap 430 that is positioned adjacent a bottom of the subject's foot when the sensor hub 404 is connected to the wearable device 402. The stiffener 445 can be disposed below the cover 443 and can include an opening 445a configured to allow optical radiation to pass through (e.g., so as not to block optical radiation from being emitted by the one or more emitters 404a or optical radiation from being received by the one or more detectors 404b). As shown, the stiffener 445b can include the thermally conductive probe 445b described above configured to transmit thermal energy from the bottom of the subject's foot toward temperature sensor 404c. The thermally conductive probe 445b can configured as a rounded protrusion that protrudes up from the stiffener 445 and at least partially through an opening 443c of the cover 443. In some implementations, the thermally conductive probe 445b is configured to contact the bottom of the subject's foot (e.g., skin of the subject) when the system 400 is in use. In some implementations, the thermally conductive probe 445b is formed in the stiffener 445. To aid in its thermal conductivity, the thermally conductive probe 445b (and the stiffener 445) can be made of a conductive material, such as stainless steel (e.g., 430 SS). Furthermore, the stiffener 445 can include one or more slits 445c positioned adjacent the thermally conductive probe 445b configured to thermally isolate the thermally conductive probe 445b and/or a portion of the stiffener 445 from other portions of the stiffener 445 to aid in transmitting thermal energy from the bottom of the subject's foot toward temperature sensor 404c. As shown, the stiffener 445 can include two slits 445c oriented substantially perpendicular to one another to effectively thermally isolate the thermally conductive probe 445b. In some variants, sensor hub 404 includes a thermally conductive probe (for example, similar to probe 445b), but: does not include stiffener 445; and/or such probe does not extend from stiffener 445. In such variants, such probe can still direct thermal energy towards temperature sensor 404c.

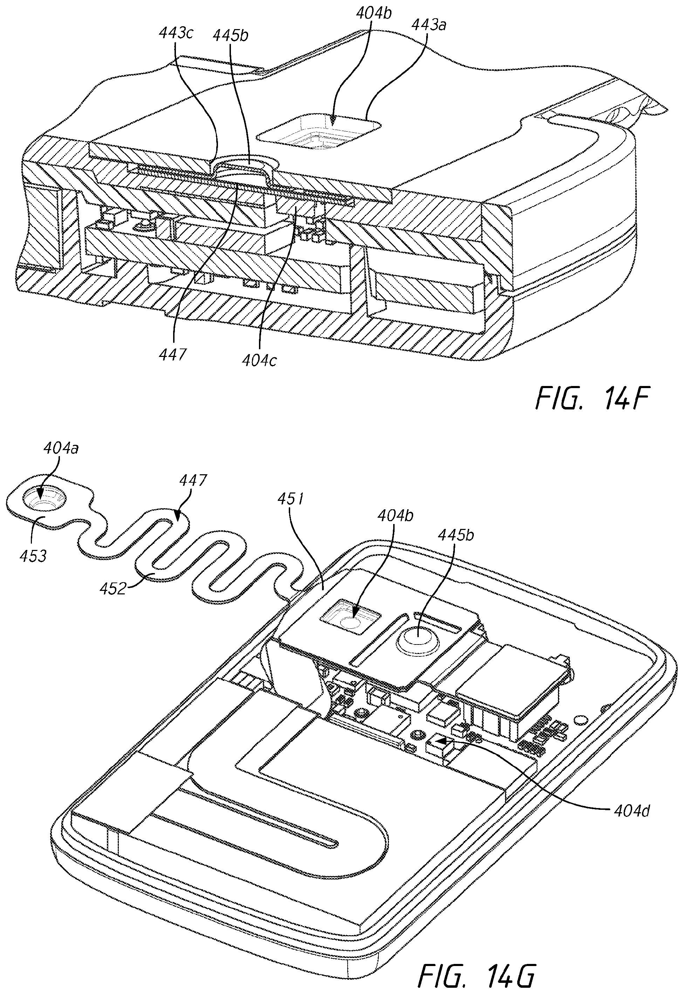

Also shown in FIG. 14E is the circuit layer 447, which can be disposed below the cover 443 and the stiffener 445 and positioned at least partially within the sensor hub 404 and/or at least partially within the sensor strap 430. As mentioned above, the circuit layer 447 can electrically connect the one or more emitters 404a and the one or more detectors 404b to the various other electrical components of the sensor hub 404 (e.g., one or more processors of the sensor hub 404). In some implementations, the circuit layer 447 also electrically connects temperature sensor 404c of the sensor dock 404 to the various other electrical components of the sensor hub 404. The circuit layer 447 can be configured as a flexible circuit that can bend freely with the sensor strap 430. In some implementations, the circuit layer 447 can have a length that is greater than a distance between where the circuit layer 447 electrically connects to the one or more emitters 404a and the one or more detectors 404b. For example, the circuit layer 447 can include one or more bends (e.g., can be serpentine). As shown in at least FIG. 14E, the circuit layer 447 can include a portion 453 configured to electrically connect to the one or more emitters 404a, a portion 451 configured to electrically connect to the one or more detectors 404b, and a portion 452 comprising at least one bend spanning between the portions 453 and 451. The portion 453 can be positioned within a recess 455 of the sensor strap 430. At least a portion of the portion 452 can be positioned within a recess 454 of the sensor strap 430. The portion 451 can be positioned within the sensor hub 404. Thus, at least a portion of the circuit 447 can be positioned within the sensor strap 430 and at least a portion of the circuit 447 can be positioned within the sensor hub 404. Similar to the openings in the cover 443, the circuit layer 447 can include an opening 447a and an opening 447b configured to overlie the one or more detectors 404b and one or more emitters 404a, respectively, and allow optical radiation to be received and/or emitted therethrough. Further shown, an adhesive layer 449 can be disposed between the stiffener 445 and the circuit layer 447 to join each to one another, the adhesive layer comprising an opening 449a similar to the openings 445a and 447a. With reference to FIG. 14F, in some implementations, circuit layer 447 can be positioned between thermally conductive probe 445b (and stiffener 445) and temperature sensor 404c.

FIG. 14G shows a view of the sensor hub 404 with a portion of its main body 420 and portions of the sensor strap 430 removed from view. In this view, the temperature sensor 404d can be seen positioned away from the thermally conductive probe 445b and the temperature sensor 404c (whose relative positions are best shown in the cross-sectional view of FIG. 14F). The temperature sensor 404d can be configured to measure an ambient temperature of the environment, while the temperature sensor 404c can be configured to measure a body temperature of the subject 1 via thermally conductive probe 445b when the system 400 is in use. In some implementations, the temperature sensor 404d and the temperature sensor 404c can be used in combination to determine at least a body temperature of the subject 1, similar or identical to the temperature sensors described and/or illustrated in U.S. Pat. Pub. No. 2021/0290072, titled "Wearable Device for Noninvasive Body Temperature Measurement". For example, a difference in temperature measured via temperature sensor 404c and via temperature sensor 404d can be used in the determination of a body temperature of the subject. Furthermore, the system 400 can include and/or incorporate any of the methods of determining temperature of a subject described in U.S. Pat. App. No. US 17/206,907.

As thermal energy is transmitted to the temperature sensor 404c (e.g., via the thermally conductive probe 445b), the temperature sensor 404c can determine a body temperature of the subject and/or can generate and transmit one or more signals responsive to the thermal energy to one or more processors of the sensor hub 404. The temperature sensor 404c can be or include a thermocouple and/or a thermistor, for example. The temperature sensor 404c can be a chip that is electrically and mechanically coupled with the circuit layer 447. The temperature sensor 404c can be configured to generate one or more signals responsive to detected thermal energy, determine body temperature, and/or transmit such generated one or more signals and/or such determined body temperature to the one or more processors of the sensor hub 404 continuously and/or intermittently. For example, temperature sensor 404c can be configured to generate one or more signals responsive to detected thermal energy, determine body temperature, and/or transmit such generated one or more signals and/or such determined body temperature every 0.5 seconds, 1 second, 2 second, 3 seconds, 4 seconds, 5 seconds, 10 seconds, 30 seconds, 1 minute, 2 minute, 3 minutes, 4 minutes, 5 minutes, or at other intervals.

The temperature sensor 404d can be configured to generate one or more signals responsive to detected thermal energy, determine temperature, and/or transmit such generated one or more signals and/or such determined temperature to the one or more processors of the sensor hub 404 continuously and/or intermittently. For example, temperature sensor 404d can be configured to generate one or more signals responsive to detected thermal energy, determine temperature, and/or transmit such generated one or more signals and/or such determined temperature every 0.5 seconds, 1 second, 2 second, 3 seconds, 4 seconds, 5 seconds, 10 seconds, 30 seconds, 1 minute, 2 minute, 3 minutes, 4 minutes, 5 minutes, or at other intervals. Such generated one or more signals, determined temperature, and/or transmission of such generated one or more signals and/or determined temperature can be simultaneous or non-simultaneous with the generated one or more signals, determined body temperature, and/or transmitted one or more signals and/or determined body temperature from temperature sensor 404c.

Advantageously, incorporating both of temperature sensors 404c, 404d can allow the sensor hub 404 to more accurately determine a body temperature of the subject. For example, the one or more processors of the sensor hub 404 can utilize temperature data from the temperature sensor 404d in order to adjust or "correct" temperature data received from the temperature sensor 404c in order to more accurately determine the subject's body temperature. For example, the one or more processors of the sensor hub 404 can compare temperature data received from both of the temperature sensors 404c, 404d and determine a corrected body temperature based on such comparison. The one or more processors can apply weight factors to one or both of temperature data received from temperature sensors 404c, 404d and/or otherwise compare such received data to determine a corrected body temperature.

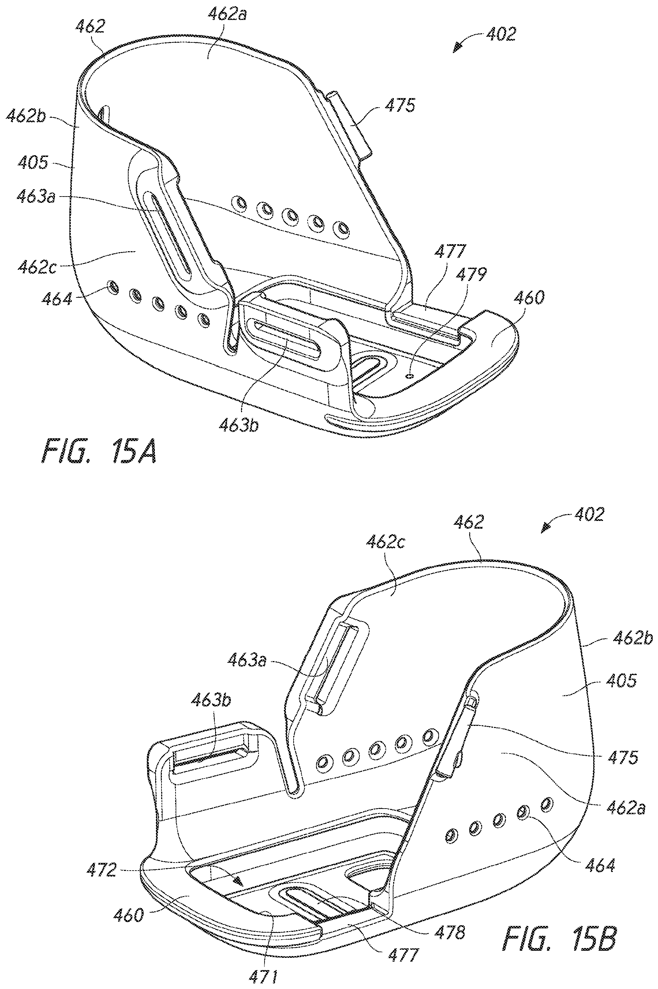

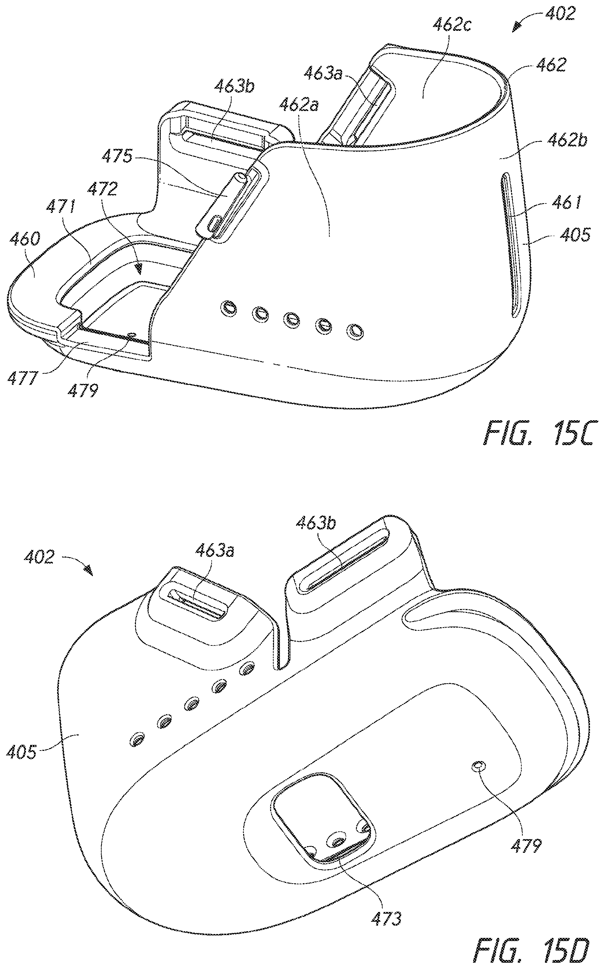

FIGS. 15A-15E illustrate perspective views of the wearable device 402 of the system 400 of FIGS. 12A-12B in accordance with some implementations of this disclosure. The wearable device 402 can be configured to receive and support the foot 2, ankle 3, heel 4, and/or lower leg 5 of the subject 1. The wearable device 402 can be similar to and include any of the features, functionality, and/or components as the wearable devices 102, 302 described herein. For example, the wearable device 402 can be made of a resilient and/or flexible material, similar or the same as the wearable devices 102, 302 described herein. The wearable device 402 can have a base 460 and a wall 462. The wall 462 can extend from the base 460. For example, the wall 462 can extend from a periphery of the base 460. In some implementations, the wall 462 can extend around a portion of a perimeter edge of the base 460. The base 460 and the wall 462 can form a main body 405 of the wearable device 402. In some implementations, the wearable device 402 can have a main body 405 including the base 460, an opening 471 in the base 460 defining a cavity 472, and the wall 462 extending from the base 460. In some implementations, the main body 405 additionally includes the wearable device strap 466 (which can also be referred to herein as an "additional strap") configured to connect to (e.g., releasably connect to) and extend from the wall 462. In some implementations, the system 400 only includes strap 430 and does not include any other straps (e.g., does not include wearable device strap 466). Furthermore, in some implementations, the system 400 only includes strap 430 and does not include any other straps (e.g., does not include strap section 431) and such strap 430 is configured to be secured to a portion of the wearable device 402.

The base 460 (which can also be referred to herein as "bottom portion") of the wearable device 402 can be configured to contact at least a portion of the bottom portion of the subject's foot 2 when the system 400 is in use. For example, the base 460 can be configured to contact a heel, an arch, a ball, and/or one or more toes of the subject's foot 2. The opening 471 in the base 460 can be configured to be positioned adjacent a bottom portion of the subject's foot 2 when the system 400 is in use. For example and as shown, the opening 471 can extend through the base 460 and be positioned such that it underlies the ball and/or a portion of the arch of the subject's foot 2 when the wearable device is secured to the subject's foot 2. The cavity 472 defined by the opening 471 in the base 460 can extending below the base 460 and away from the bottom portion of the subject's foot 2 when the system 400 is in use. The cavity 472 can be configured to removably receive the sensor hub 404 and/or at least a portion of the sensor strap 430, for example, when the sensor hub 404 and the sensor strap 430 are connected to the wearable device 402. In other words, the cavity 472 can be configured to removably receive the sensor component 403 when the sensor component is connected to the wearable device 402. The wearable device 402 can, as shown, include an opening 473 configured to aid in removing the sensor component 403 (e.g., the sensor hub 404 and the sensor strap 430) from the wearable device 402 when desired to do so. For example, the opening 473 can be disposed within the cavity 472 and comprise a through-opening through the bottom of the wearable device 402 that a subject can use to push upon the sensor component 403 for removal thereof from the wearable device 402. Such opening 473 can also aid a subject in securing the sensor component 403 within the cavity 472. The wearable device 402 can also, as shown, include an opening 479 configured to substantially align with the status indicator 425 of the sensor hub 404 when the sensor hub 404 is connected to the wearable device 402. The opening 479 can be disposed within the cavity 472 and comprise a through-opening through the bottom of the wearable device 402 that can allow a status of the sensor component 403 via status indicator 425 to be visible when the system 400 is in use. In some implementations, the cavity 472 can include a protrusion 478 configured to interact with the electrical connector 424 of the sensor hub 404. For example, the protrusion 478 can be configured as a raised oblong that fits within a corresponding oblong recess of the electrical connector 424 of the sensor hub 404. Such protrusion 478 can aid in securing the sensor hub 404 with the wearable device 402.

The wall 462 of the wearable device 402 can extend upward from a periphery of the base 460 and include a side portion 462a (which can also be referred to herein as a "sidewall portion"), a back portion 462b (which can also be referred to herein as a "back wall portion"), and a side portion 462c (which can also be referred to herein as a "sidewall portion") configured to wrap around and support portions of the subject's foot 2, such as side(s) of the subject's foot 2, the subject's ankle 3, the subject's heel 4, and/or the subject's lower leg 5. The wall 462 can be discontinuous such that it does not enclose the complete periphery of the base 460, leaving space for the sensor strap 430 to extend from the opening 471 and a recess 477 in the base 460. Furthermore, the wall 462 can be discontinuous such that the toes of the subject's foot 2 are not enclosed. The wall 462 can have a variable height as it extends upward from the base. For example and as shown in FIGS. 15A-15E, the wall 462 can have a maximum height at the back portion 462b so as to surround and support the subject's heel 4, and a reduced height at side portions 462a and 462c. In some implementations, the main body 405 of the wearable device 402 can include a plurality of hole(s) 464 for venting and/or for tuning the resilience of the wearable device 402. For example, the wall 462 and/or the base 460 can have a plurality of hole(s) 464 therethrough. Furthermore and in some implementations, the main body 405 of the wearable device 402 can include an opening 461 located in the back portion 462b of the wall 462. The opening 461 can be configured to allow the wearable device 402 to adapt to the heel 4 of the subject's foot 2.

With continued reference to FIGS. 14A-14E, the wearable device 402 can also include one or more features for securing to the subject's foot 2. For example and as shown, the main body 405 of the wearable device 402 can include the wearable device strap 466 mentioned previously that can connect to and extend outward from the wall 462, as well as a strap slot 463a (which can also be referred to herein as an "opening") configured to interact with wearable device strap 466. The wearable device strap 466 can be configured to wrap over a portion of the subject's foot 2, ankle 3, and/or lower leg 5, have its end placed through the strap slot 463a, and secure back upon itself similar or the same as the wearable device strap 366. The wearable device strap 466 can connect to and extend from side portion 462a of the wall 462 at a location adjacent where the wearable device 402 would receive the subject's ankle 3 if secured to the subject 1. Further, the wearable device strap 466 can connect to and extend from the side portion 462a at an acute angle with respect to the base 460 when viewed from a side of the wearable device 402. The strap slot 463a can comprise a slot through the side portion 462c of the wall 462 (e.g., a side of the wall 462 opposite from where from the wearable device strap 466 connect and extends from) configured to receive the wearable device strap 466 therethrough. In some implementations, the wearable device strap 466 can be configured to releasably connect with the wall 462 via connectors 475 and 476 similar or the same as the wearable device strap 366 via connectors 375 and 376.

The main body 405 of the wearable device 402 can also include one or more features for releasably securing to the sensor strap 430 when the sensor hub 404 is connected to the wearable device 402 via cavity 472. For example and as shown, the base 460 can include a portion adjacent the opening 471 that extends out beyond where the bottom of the subject's foot 2 is received when the system 400 is worn that includes a strap slot 463b (which can be the same as or similar to the strap slot 463a, and which can also be referred to herein as an "opening") that can interact with the sensor strap 430 similar to or the same as how the strap slot 463a interacts with wearable device strap 466 or similar to or the same as how the strap slot 363b interacts with the sensor strap 330. To customize the fit of the system 400 to the subject's foot 2, the wearable device strap 466 and/or the sensor strap 430 can be wrapped over the subject's foot 2, the ends of the straps placed through strap slots 463a and 463b, and the ends secured back upon the straps similar or the same as described with respect to the system 300. In some implementations, the portion of the base 460 comprising the strap slot 463b can deform when the sensor strap 430 is secured to the strap slot 463b, such as shown in FIGS. 12A-12B (e.g., it can bend and/or fold against the foot 2). In some implementations, the strap slot 463b can be formed in a portion of the wall 462 (e.g., a portion of the wall 462c) rather than be included as an extension of the base 460.

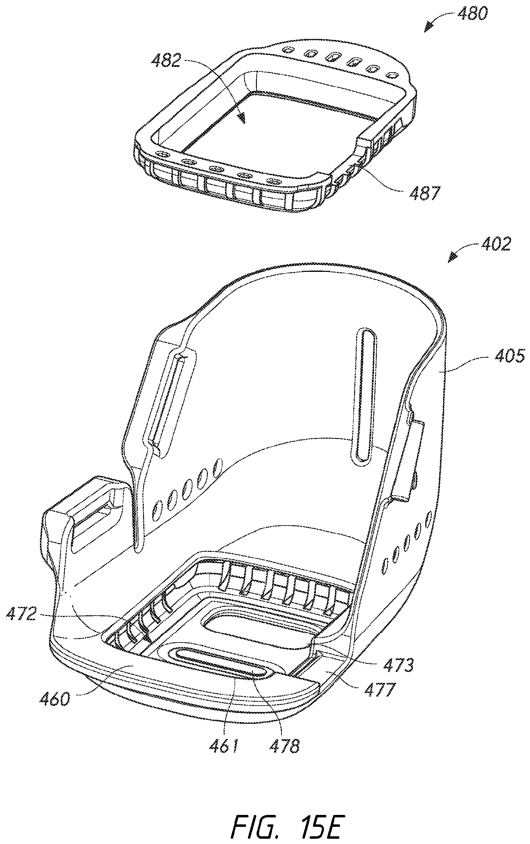

In some implementations and as shown in FIG. 15E, the wearable device 402 can include the main body 405 and a frame 480 (which can also be referred to herein as a "holder"). The main body 402 can comprise a first material that is resilient and flexible. For example, the main body 402 can comprise silicone rubber. The frame 480 can comprise a second material that is more rigid than the first material. For example, the frame 480 can comprise polycarbonate. The frame 480 can be configured to releasably receive at least a portion of the sensor component 403 when the sensor component 403 is connected to the wearable device 402. In other words, the frame 480 can be configured to releasably receive the sensor hub 404 and/or at least a portion of the sensor strap 430 for securing the sensor hub 404 and/or the sensor strap 430 to the wearable device 402. For this, the base 460 of the wearable device 402 can be configured to receive the frame 480 therein. For example, the cavity 472 can be configured to receive the frame 480. The frame 480 and the main body 405 can be integrally formed. For example, the main body 405 can be overmolded over the frame 480 to produce the wearable device 402. The frame 480 can have an opening 482 configured to releasably receive the sensor hub 404 when the sensor component 403 is connected to the wearable device 402. Furthermore, the frame 480 can have a recess 487 configured to receive at least a portion of the sensor strap 430 when the sensor hub 404 is connected to the cavity 482. The recess 487 of the frame and the recess 477 of the main body 405 can allow the sensor strap 430 to extend away from the sensor hub 404 and the wearable device 402 such that the top of the sensor hub 404 and/or the sensor strap 430 can form a substantially flush surface (e.g., be substantially coplanar) with the base 460 of the wearable device 402. Furthermore, the opening 482 of the frame 480 and the cavity 472 of the main body 405 can together be configured to releasably connect to the sensory assembly 403 (e.g., the sensor hub 404). In some implementations, the frame 480 can include one or more features to aid in securing with the sensor component 403 (e.g., the sensor hub 404). For example, the frame 480 can include one or more protrusions or ridges that extend inward towards the opening 482 configured to provide a friction fit with at least a portion of the sensor component 403.

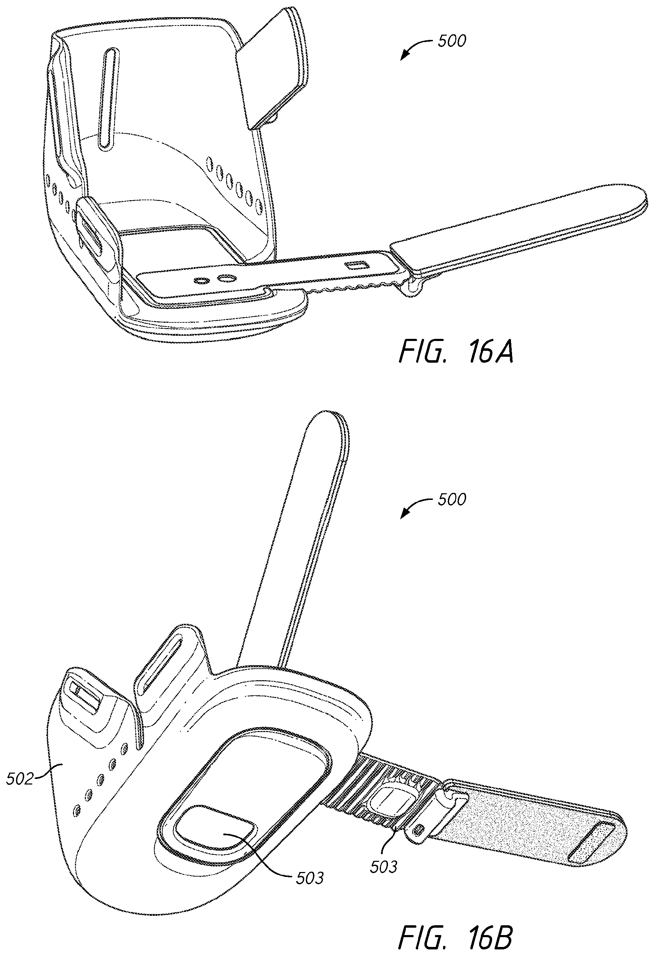

FIGS. 16A-16D illustrate various perspective views of another implementation of a system 500 (which can also be referred to herein as a "wearable system," "wearable sensor system," or "wearable physiological sensor system") configured to be secured to the subject's foot 2 and measure at least one physiological parameter of the subject 1. The system 500 can have similar and/or the same features, aspects, functionality, and/or components as any of the systems described herein, such as systems 100, 300, 400 and/or any variants thereof. For example, the system 500 can have a wearable device 502 configured the same as or similar to the wearable device 402 in some or many respects. As another example, the system 500 can include a sensor hub 504 and a sensor strap 530 ( together which can be referred to as a sensor component or sensor assembly 503), configured the same or similar to the sensor hub 404 and the sensor strap 430 of the sensor component 403, respectively. The system 400 can be secured to the subject's foot 2, ankle 3, heel 4, and/or lower leg 5 similar or identical to how the system 400 can be secured to the subject 1. Furthermore, the system 500 can include one or more emitters 504a (for example, in an emitter package), one or more detectors 504b (for example, in a detector package), and one or more temperature sensors 504c that are similar or identical to the one or more emitters 404a, the one or more detectors 404b, and the one or more temperature sensors 404c of the system 400. The system 500 can also include an additional temperature sensor (in addition to temperature sensor 504c) that can be similar or identical to temperature sensor 404d and which can be located in sensor hub 504, for example. Such additional temperature sensor can be spaced apart from the temperature sensor 504c and function with the temperature sensor 504c the same or similar to as described above with respect to temperature sensors 404c and 404d.

The system 500 can include any of the features or components discussed with respect to FIG. 3 above. The system 500 can wirelessly communicate with one or more separate device(s), which can be for example, a patient monitor 10a, a mobile phone 10b, a camera, a hub, or any other separate device(s) described herein via any of a variety of wireless communication protocols such as any of those discussed herein with respect to the systems 100, 300, 400. Furthermore, the system 500 can wirelessly transmit subject physiological data and/or physiological parameters to separate device(s) (such as patient monitor 10a, mobile phone 10b, a camera, a hub, or other separate device(s)) as described herein with respect to the systems 100, 300, 400.

When secured to the subject's foot 2, the system 500 can operably position one or more emitters 504a and one or more detectors 504b adjacent portions of the foot 2, for example, at generally opposite sides of the subject's foot 2. For example, the system 500 can position the one or more emitters 504a and the one or more detectors 504b such that at least some of the optical radiation emitted by the one or more emitters 504a passes through tissue of the subject 1 before being detected by the one or more detectors 504b. As another example, the system 500 can position the one or more detectors 504a adjacent a top and/or a side of the subject's foot 2 and the one or more emitters 504b adjacent a bottom of the subject's foot 2. The one or more emitters 504a and the one or more detectors 504b do not need to be vertically aligned with one another and on opposite sides of the subject's foot in order for the system 500 to operate. In some implementations, the positioning of the one or more emitters 404a and the one or more detectors 404b can be reversed. In some implementations, at least a portion of the sensor strap 530 and/or at least a portion of the sensor hub 504 operably positions the one or more emitters 504a and/or the one or more detectors 504b as described, for example, when sensor hub 504 is secured to a portion of wearable device 502. In some implementations, at least a portion of the sensor component 503 operably positions the one or more emitters 504a and/or the one or more detectors 504b as described. The system 500 can operably position a thermally conductive probe 545b configured to transmit thermal energy adjacent a bottom of the subject's foot (e.g., such that it contacts skin of the subject 1) to transmit thermal energy from the bottom of the subject's foot toward the temperature sensor 504c.

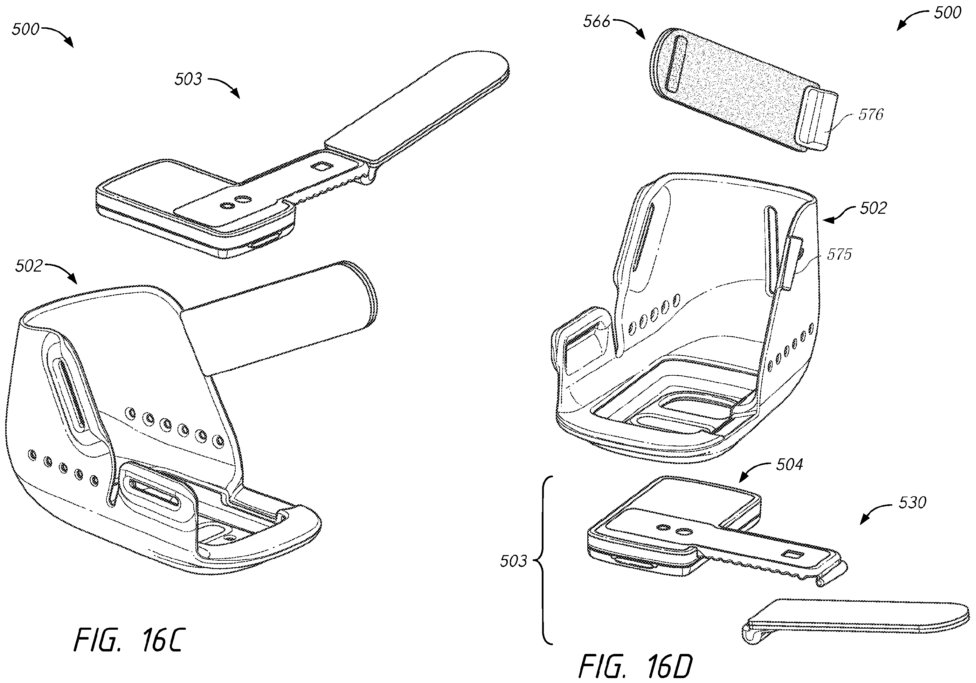

The system 500 can include the wearable device 502. The wearable device 502 can be configured to receive and/or secure an electronic device including one or more sensors for monitoring information relating to physiological, motion, and/or location of the subject 1. For example, the wearable device can be configured to receive and/or secure the sensor component 503 (which may also be referred to herein as a "sensor assembly") or a portion thereof, as described further herein. Such sensor component 503 can include the sensor hub 504 and the sensor strap 530. In some implementations, the system 500 can include the wearable device 502, the sensor hub 504 and the sensor strap 530. As shown in FIGS. 16A-16B, the wearable device 502 and the sensor component 503 can be secured to one another and secured to the subject's foot. FIG. 16C illustrates the sensor component 503 disconnected from the wearable device 502. FIG. 16D illustrates an exploded view of system 500. The wearable device 502 can have a wearable device strap 566 configured the same or similar to the wearable device strap 466 of the wearable device 402, and as shown in FIG. 16D, the wearable device strap 566 can be removably connectable to the wearable device 502 (although in some implementations, the wearable device strap 566 can be integrally formed with the wearable device 502). Also shown in FIG. 16D, the sensor component 503 can include the sensor hub 504 and the sensor strap 530, with at least a portion of the sensor strap (e.g., a securement section) configured to be removably connectable thereto.

Although the figures illustrate implementations in which the wearable device 502 and the sensor hub 504 and the sensor strap 530 are removably connectable to one another (or, in other words, where the wearable device 502 and the sensor component 503 are removably connectable to one another), various ones of these components may be integrally formed with one another. For example, in some variants, the wearable device 502 and sensor hub 504 are integrally formed and are removably connectable to the sensor strap 530 or at least a portion thereof. As another example, in some variants, the sensor hub 504 and the sensor strap 530 are integrally formed and are removably connectable to the wearable device 502. As another example, in some variants, the wearable device 502, the sensor hub 504, and sensor strap 530 are integrally formed with one another (or, in other words, the wearable device 502 and the sensor component 503 are integrally formed with one another). Implementations of the system 500 in which wearable device 502 is removably connectable from sensor hub 504 and/or sensor strap 530 can advantageously allow for a wearable device 502 of various sizes (e.g., small, medium, and large) and/or shapes to be utilized with the system 500, for example, so as to accommodate various sizes and/or shapes of a subject's foot 2, ankle 3, heel 4, and/or lower leg 5. In this way, the system 500 can be customized to a subject 1 by selecting an appropriately configured wearable device 502 while allowing for all other aspects of the system 500, such as the sensor hub 504 and sensor strap 530 or sensor component 503, to remain the same and/or be universal across subjects. In some implementations, for example as shown in FIG. 16D, the sensor hub 504 and the sensor strap 530 form the sensor component 503 that can be removably connected to the wearable device 502. In some implementations, at least a portion of the sensor strap 530 (e.g., a securement portion) and/or the wearable device strap 566 can come in various sizes and/or lengths (e.g., small, medium, large) to advantageously provide a customized fit with any of the various sizes of the wearable device 502.

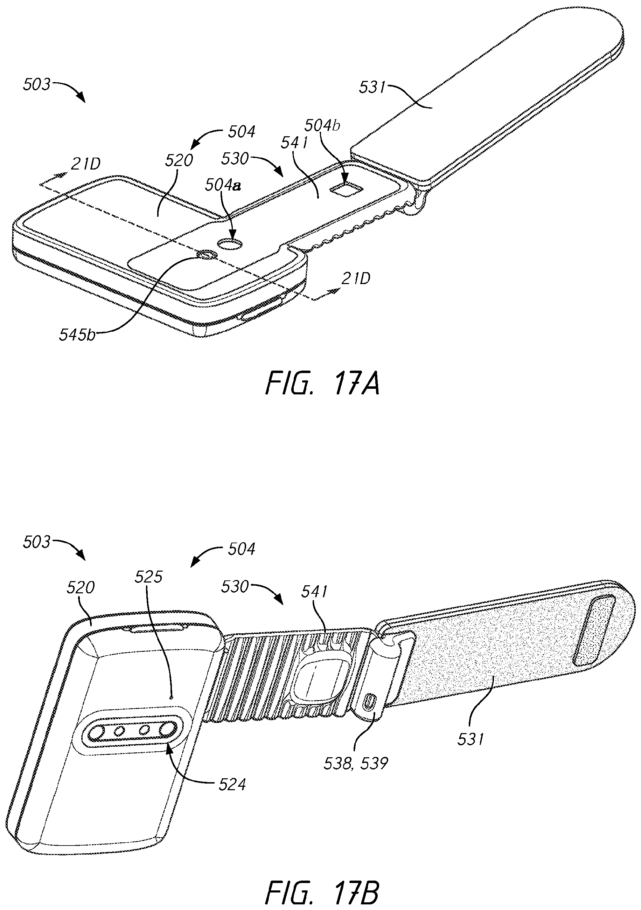

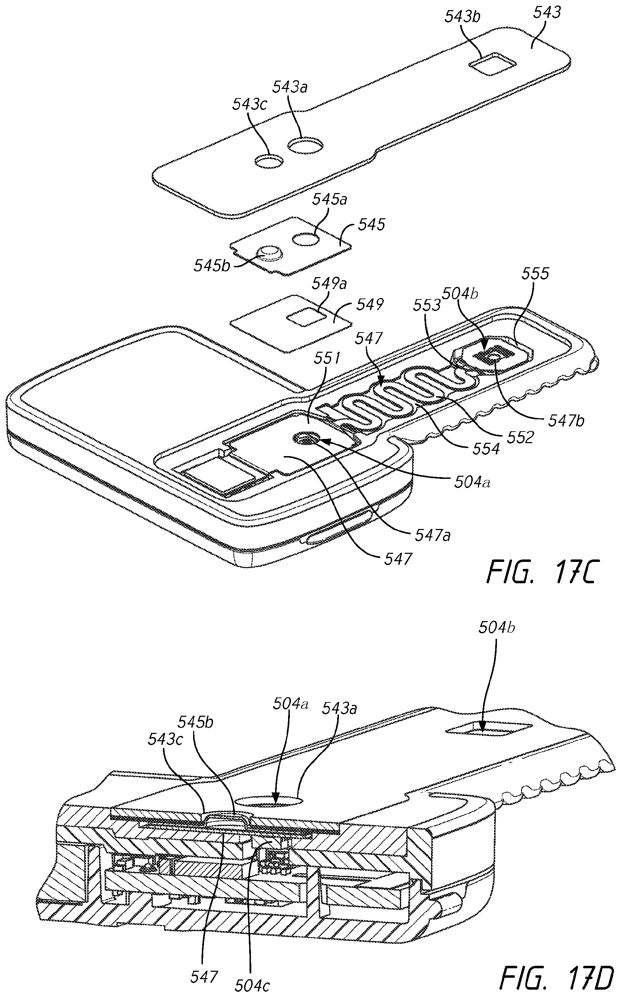

FIGS. 17A-17D illustrate various perspective views of the sensor component 503 of the system 500 and/or components/aspects thereof. As shown, the sensor component 503 can include a sensor hub 504 and a sensor strap 530 (which also may be referred to herein as "strap") connected to and extending outward from the sensor hub 504. The sensor hub 504 can include a generally rounded rectangular housing having a top, sides, and a bottom. In some implementations, the sensor hub 504 has a length and/or a width that are greater than a height of the sensor hub 504. The sensor strap 530 can include the one or more emitters 504a and/or the one or more detectors 504b and can be configured to secure the system 500 to the subject's foot 2 as described herein (for example, alone or in combination with wearable device strap 566 of wearable device 502). The sensor strap 530 can be the same or similar, or include any of the functionality and/or features of the sensor strap 430 described herein. In some implementations, the one or more detectors 504b are positioned within a portion of the sensor strap 530 and the one or more emitters 504a are positioned within a portion of the sensor hub 504. The one or more emitters 504a and the one or more detectors 504b can be in electrical communication with one or more processors of the sensor hub 504 via a circuit layer 547 disposed at least partially within the sensor strap 530 and at least partially within the sensor hub 504 (for example, as shown in FIG. 17C). The sensor strap 530 can have a top that sits substantially flush (e.g., substantially coplanar) with the sensor hub 504 (e.g., with the top of the sensor hub 504). Additionally, the sensor strap 530 can extend from the sensor hub 504 in a direction substantially perpendicular to the sensor hub 504. In some implementations, the sensor hub 504 can have a main body 520.

With reference to FIG. 17B, the sensor hub 504 can include an electrical connector 524 configured to releasably connect to a charger, for example charger 499. Electrical connector 524 can be configured in a similar or identical manner as described elsewhere herein with respect to electrical connector 424.

Strap 530 can include a sensor section 541 (which may be referred to as a "first section") and a securement section 531 (which may be referred to as a "second section") which can be similar or identical to sensor section 441 and securement section 431 of system 400 as described herein. For example, sensor section 541 and securement section 531 can be releaseably connectable to one another via connectors 538, 539 which can be similar or identical to connectors 338, 438, 339, 439 as described elsewhere herein. In some implementations, at least a portion of the strap 530 is stretchable. For example, at least a portion of the sensor section 541 and/or at least a portion of the securement section 531 can be configured to be stretchable. In some implementations, the sensor section 541 is more stretchable than the securement section 531.

FIGS. 17C-17D further illustrate perspective views of the sensor hub 504 and sensor strap 530 that progressively show the various aspects, components, and/or features that the sensor hub 504 and sensor strap 530 can include. The sensor hub 504 and sensor strap 530 can include any or all of the features and/or functionality of the sensor hub 406, sensor dock 404, and sensor strap 430 described herein. As shown in FIG. 17C, the sensor hub 540 and the sensor strap 530 can include a cover 543 (which may also be referred to as a "cover plate") configured to cover at least some of the electrical circuitry of the sensor hub 504 and the sensor strap 530, such as the circuit layer 547. The cover 543 can be similar or the same as the cover 443 described herein. The cover 543 can fit into a recess of the sensor hub 504 and the sensor strap 530, such that the cover 543 and the surface of the sensor hub 504 and the sensor strap 530 adjacent the cover 543 form a substantially flush surface. The cover 543 can include openings 543b and 543a configured to overlie the one or more detectors 504b and the one or more emitters 504a similar or the same as the openings 443b and 443a of the cover 443 described herein. In some implementations, such openings are covered by transparent material (for example, to prevent ingress of liquid therethrough. However, in alternative implementations, such openings are not covered. As shown in FIG. 17C (in which aspects of the sensor strap 541 have been removed from view), in addition to the cover 543, the sensor hub 504 and/or strap 530 can include a stiffener 545 (which can be a plate made of metal, for example) configured to increase the stiffness of a portion of the sensor hub 504 and/or strap 530 that is positioned adjacent a bottom of the subject's foot when the sensor hub 504 is connected to the wearable device 502. The stiffener 545 can be disposed below the cover 543 and can include an opening 545a configured to allow optical radiation to pass through (e.g., so as not to block optical radiation from being emitted by the one or more emitters 504a or optical radiation from being received by the one or more detectors 504b). As shown, the stiffener 545b can include the thermally conductive probe 545b described above configured to transmit thermal energy from the bottom of the subject's foot toward temperature sensor 504c. The thermally conductive probe 545b can configured as a rounded protrusion that protrudes up from the stiffener 545 and at least partially through an opening 543c of the cover 543. In some implementations, the thermally conductive probe 545b is configured to contact the bottom of the subject's foot (e.g., skin of the subject) when the system 500 is in use. In some implementations, the thermally conductive probe 545b is formed in the stiffener 545. To aid in its thermal conductivity, the thermally conductive probe 545b (and the stiffener 545) can be made of a conductive material, such as stainless steel (e.g., 430 SS). In some variants, sensor hub 504 includes a thermally conductive probe (for example, similar to probe 545b), but: does not include stiffener 545; and/or such probe does not extend from stiffener 545. In such variants, such probe can still direct thermal energy towards temperature sensor 504c.

Also shown in FIG. 17C is the circuit layer 547, which can be disposed below the cover 543 and the stiffener 545 and positioned at least partially within the sensor hub 504 and/or at least partially within the sensor strap 530. As mentioned above, the circuit layer 547 can electrically connect the one or more emitters 504a and the one or more detectors 504b to the various other electrical components of the sensor hub 504 (e.g., one or more processors of the sensor hub 504). In some implementations, the circuit layer 547 also electrically connects temperature sensor 504c of the sensor dock 504 to the various other electrical components of the sensor hub 504. The circuit layer 547 can be configured as a flexible circuit that can bend freely with the sensor strap 530. In some implementations, the circuit layer 547 can have a length that is greater than a distance between where the circuit layer 547 electrically connects to the one or more emitters 504a and the one or more detectors 504b. For example, the circuit layer 547 can include one or more bends (e.g., can be serpentine). As shown in at least FIG. 17C, the circuit layer 547 can include a portion 553 configured to electrically connect to the one or more detectors 504a, a portion 551 configured to electrically connect to the one or more emitters 504b, and a portion 552 comprising at least one bend spanning between the portions 553 and 551. The portion 553 can be positioned within a recess 555 of the sensor strap 530. At least a portion of the portion 552 can be positioned within a recess 554 of the sensor strap 530. The portion 551 can be positioned within the sensor hub 504. Thus, at least a portion of the circuit 547 can be positioned within the sensor strap 530 and at least a portion of the circuit 547 can be positioned within the sensor hub 504. Similar to the openings in the cover 543, the circuit layer 547 can include an opening 547b and an opening 547a configured to overlie the one or more detectors 504b and one or more emitters 504a, respectively, and allow optical radiation to be received and/or emitted therethrough. Further shown, an adhesive layer 549 can be disposed between the stiffener 545 and the circuit layer 547 to join each to one another, the adhesive layer comprising an opening 549a similar to the openings 545a and 547a. With reference to FIG. 17D, in some implementations, circuit layer 547 can be positioned between thermally conductive probe 545b (and stiffener 545) and temperature sensor 504c.

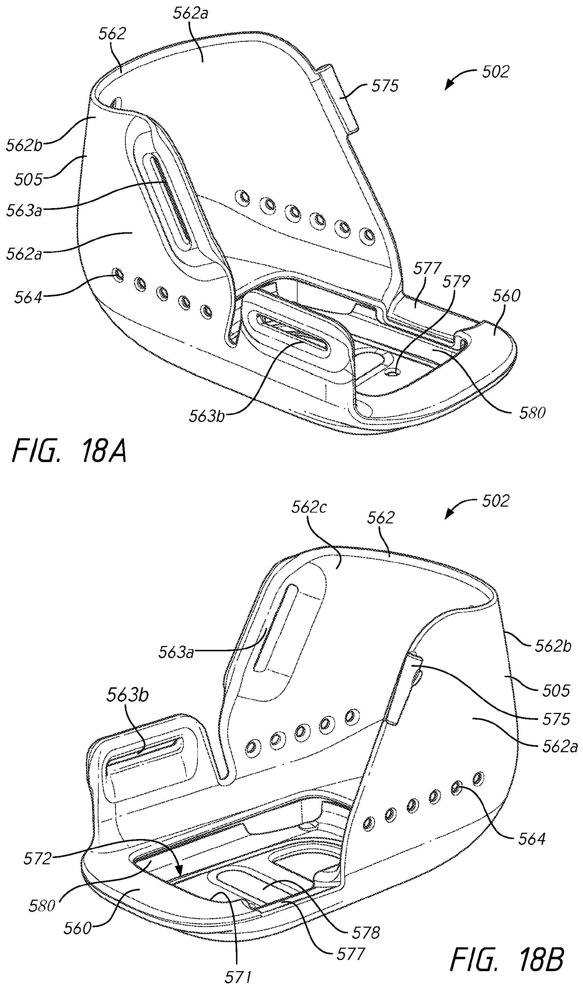

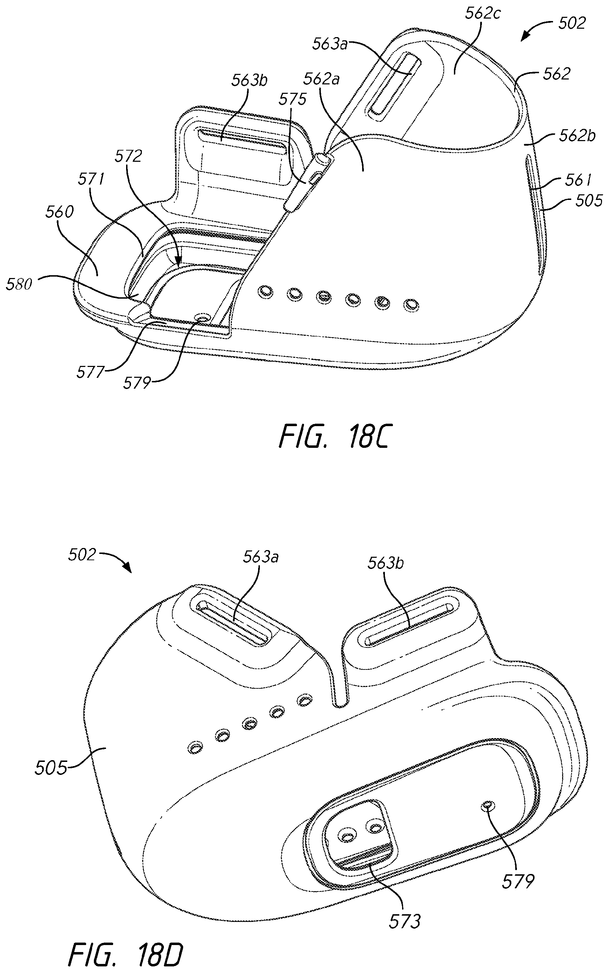

FIGS. 18A-18D illustrate perspective views of the wearable device 502 of the system 500 of FIGS. 16A-16B in accordance with some implementations of this disclosure. The wearable device 502 can be configured to receive and support the foot 2, ankle 3, heel 4, and/or lower leg 5 of the subject 1. The wearable device 502 can be similar to and include any of the features, functionality, and/or components as the wearable devices 102, 302, 402 described herein. For example, the wearable device 502 can be made of a resilient and/or flexible material, similar or the same as the wearable devices 102, 302, 402 described herein. The wearable device 502 can have a base 560 and a wall 562. The wall 562 can extend from the base 560. For example, the wall 562 can extend from a periphery of the base 560. In some implementations, the wall 562 can extend around a portion of a perimeter edge of the base 560. The base 560 and the wall 562 can form a main body 505 of the wearable device 502. In some implementations, the wearable device 502 can have a main body 505 including the base 560, an opening 571 in the base 560 defining a cavity 572, and the wall 562 extending from the base 560. In some implementations, the main body 505 additionally includes the wearable device strap 566 (which can also be referred to herein as an "additional strap") configured to connect to (e.g., releasably connect to) and extend from the wall 562. In some implementations, the system 500 only includes strap 530 and does not include any other straps (e.g., does not include wearable device strap 566). Furthermore, in some implementations, the system 500 only includes strap 530 and does not include any other straps (e.g., does not include strap section 531) and such strap 530 is configured to be secured to a portion of the wearable device 502.

The base 560 (which can also be referred to herein as "bottom portion") of the wearable device 502 can be configured to contact at least a portion of the bottom portion of the subject's foot 2 when the system 500 is in use. For example, the base 560 can be configured to contact a heel, an arch, a ball, and/or one or more toes of the subject's foot 2. The opening 571 in the base 560 can be configured to be positioned adjacent a bottom portion of the subject's foot 2 when the system 500 is in use. For example and as shown, the opening 571 can extend through the base 560 and be positioned such that it underlies the ball and/or a portion of the arch of the subject's foot 2 when the wearable device is secured to the subject's foot 2. The cavity 572 defined by the opening 571 in the base 560 can extending below the base 560 and away from the bottom portion of the subject's foot 2 when the system 500 is in use. The cavity 572 can be configured to removably receive the sensor hub 504 and/or at least a portion of the sensor strap 530, for example, when the sensor hub 504 and the sensor strap 530 are connected to the wearable device 502. In other words, the cavity 572 can be configured to removably receive the sensor component 503 when the sensor component is connected to the wearable device 502. The wearable device 502 can, as shown, include an opening 573 configured to aid in removing the sensor component 503 (e.g., the sensor hub 504 and the sensor strap 530) from the wearable device 502 when desired to do so. For example, the opening 573 can be disposed within the cavity 572 and comprise a through-opening through the bottom of the wearable device 502 that a subject can use to push upon the sensor component 503 for removal thereof from the wearable device 502. Such opening 573 can also aid a subject in securing the sensor component 503 within the cavity 572. The wearable device 502 can also, as shown, include an opening 579 configured to substantially align with the status indicator 525 of the sensor hub 504 when the sensor hub 504 is connected to the wearable device 502. The opening 579 can be disposed within the cavity 572 and comprise a through-opening through the bottom of the wearable device 502 that can allow a status of the sensor component 503 via status indicator 525 to be visible when the system 500 is in use. In some implementations, the cavity 572 can include a protrusion 578 configured to interact with the electrical connector 524 of the sensor hub 504. For example, the protrusion 578 can be configured as a raised oblong that fits within a corresponding oblong recess of the electrical connector 524 of the sensor hub 504. Such protrusion 578 can aid in securing the sensor hub 504 with the wearable device 502.

The wall 562 of the wearable device 502 can extend upward from a periphery of the base 560 and include a side portion 562a (which can also be referred to herein as a "sidewall portion"), a back portion 562b (which can also be referred to herein as a "back wall portion"), and a side portion 562c (which can also be referred to herein as a "sidewall portion") configured to wrap around and support portions of the subject's foot 2, such as side(s) of the subject's foot 2, the subject's ankle 3, the subject's heel 4, and/or the subject's lower leg 5. The wall 562 can be discontinuous such that it does not enclose the complete periphery of the base 560, leaving space for the sensor strap 530 to extend from the opening 571 and a recess 577 in the base 560. Furthermore, the wall 562 can be discontinuous such that the toes of the subject's foot 2 are not enclosed. The wall 562 can have a variable height as it extends upward from the base. For example and as shown in FIGS. 18A-18D, the back portion 562b of the wall 562 can have a height that can surround and support the subject's heel 4, and a greater height at side portions 562a and 562c. In some implementations, the main body 505 of the wearable device 502 can include a plurality of hole(s) 564 for venting and/or for tuning the resilience of the wearable device 502. For example, the wall 562 and/or the base 560 can have a plurality of hole(s) 564 therethrough. Furthermore and in some implementations, the main body 505 of the wearable device 502 can include an opening 561 located in the back portion 562b of the wall 562. The opening 561 can be configured to allow the wearable device 502 to adapt to the heel 4 of the subject's foot 2.

The wearable device 502 can also include one or more features for securing to the subject's foot 2. For example, the main body 505 of the wearable device 502 can include the wearable device strap 566 mentioned previously that can connect to and extend outward from the wall 562, as well as a strap slot 563a (which can also be referred to herein as an "opening") configured to interact with wearable device strap 566. The wearable device strap 566 can be configured to wrap over a portion of the subject's foot 2, ankle 3, and/or lower leg 5, have its end placed through the strap slot 563a, and secure back upon itself similar or the same as the wearable device strap 466. The wearable device strap 566 can connect to and extend from side portion 562a of the wall 562 at a location adjacent where the wearable device 502 would receive the subject's ankle 3 if secured to the subject 1. Further, the wearable device strap 566 can connect to and extend from the side portion 562a at an acute angle with respect to the base 560 when viewed from a side of the wearable device 502. The strap slot 563a can comprise a slot through the side portion 562c of the wall 562 (e.g., a side of the wall 562 opposite from where from the wearable device strap 566 connect and extends from) configured to receive the wearable device strap 566 therethrough. In some implementations, the wearable device strap 566 can be configured to releasably connect with the wall 562 via connectors 575 and 576 similar or the same as the wearable device strap 466 via connectors 475 and 476.

The main body 505 of the wearable device 502 can also include one or more features for releasably securing to the sensor strap 530 when the sensor hub 504 is connected to the wearable device 502 via cavity 572. For example and as shown, the base 560 can include a portion adjacent the opening 571 that extends out beyond where the bottom of the subject's foot 2 is received when the system 500 is worn that includes a strap slot 563b (which can be the same as or similar to the strap slot 563a, and which can also be referred to herein as an "opening") that can interact with the sensor strap 530 similar to or the same as how the strap slot 563a interacts with wearable device strap 566 or similar to or the same as how the strap slot4 interacts with the sensor strap 430. To customize the fit of the system 500 to the subject's foot 2, the wearable device strap 566 and/or the sensor strap 530 can be wrapped over the subject's foot 2, the ends of the straps placed through strap slots 563a and 563b, and the ends secured back upon the straps similar or the same as described with respect to the system 400. In some implementations, the portion of the base 560 comprising the strap slot 563b can deform when the sensor strap 530 is secured to the strap slot 563b. In some implementations, the strap slot 563b can be formed in a portion of the wall 562 (e.g., a portion of the wall 562c) rather than be included as an extension of the base 560.

**In** some implementations, the wearable device 502 can include the main body 505 and a frame 580 (which can also be referred to herein as a "holder"), which can be the same or similar to the arrangement of the wearable device 402. The main body 505 can comprise a first material that is resilient and flexible. For example, the main body 505 can comprise silicone rubber. The frame 580 can comprise a second material that is more rigid than the first material. For example, the frame 580 can comprise polycarbonate. The frame 580 can be configured to releasably receive at least a portion of the sensor component 503 when the sensor component 503 is connected to the wearable device 502. In other words, the frame 580 can be configured to releasably receive the sensor hub 504 and/or at least a portion of the sensor strap 530 for securing the sensor hub 504 and/or the sensor strap 530 to the wearable device 502. For this, the base 560 of the wearable device 502 can be configured to receive the frame 580 therein. For example, the cavity 572 can be configured to receive the frame 580. The frame 580 and the main body 505 can be integrally formed. For example, the main body 505 can be overmolded over the frame 580 to produce the wearable device 502. The frame 580 can have an opening configured to releasably receive the sensor hub 504 when the sensor component 503 is connected to the wearable device 502. Furthermore, the frame 580 can have a recess configured to receive at least a portion of the sensor strap 530 when the sensor hub 504 is connected to the cavity 582. The recess of the frame and the recess 577 of the main body 505 can allow the sensor strap 530 to extend away from the sensor hub 504 and the wearable device 502 such that the top of the sensor hub 504 and/or the sensor strap 530 can form a substantially flush surface (e.g., be substantially coplanar) with the base 560 of the wearable device 502. Furthermore, the opening of the frame 580 and the cavity 572 of the main body 505 can together be configured to releasably connect to the sensory assembly 503 (e.g., the sensor hub 404). In some implementations, the frame 580 can include one or more features to aid in securing with the sensor component 503 (e.g., the sensor hub 504). For example, the frame 580 can include one or more protrusions or ridges that extend inward towards the opening configured to provide a friction fit with at least a portion of the sensor component 503.

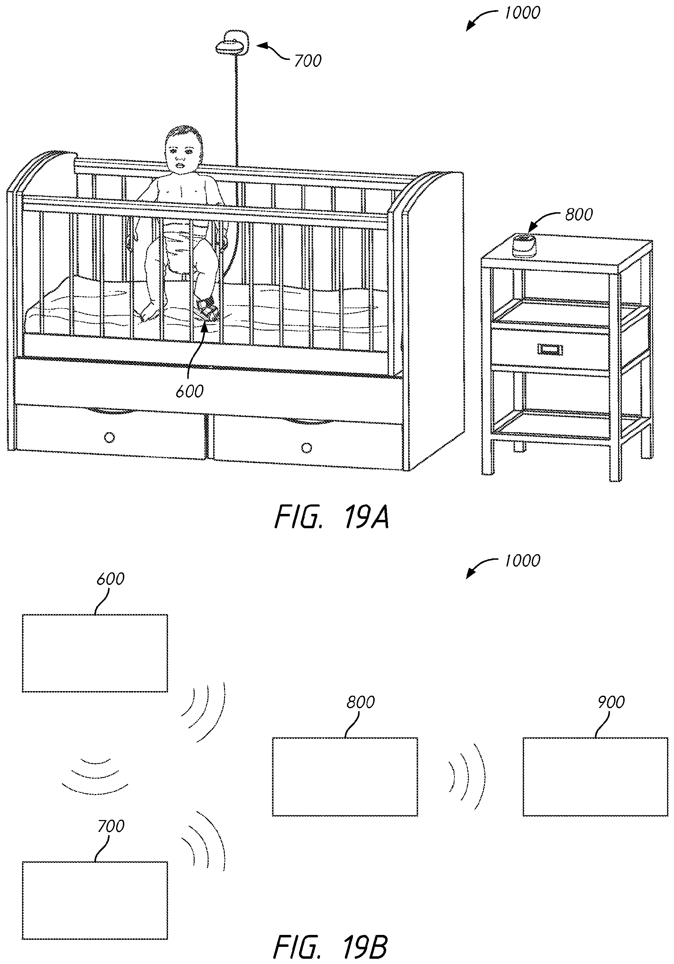

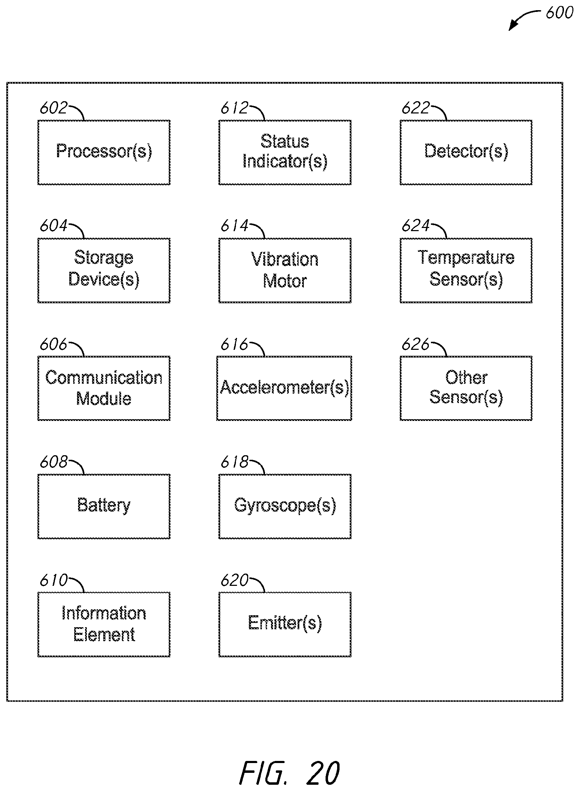

FIGS. 19A-19B illustrate a monitoring system 1000 that can be utilized to monitor at least one physiological parameter, motion, and/or location of a subject, including any one or more of the physiological parameters described herein and/or others. The monitoring system 1000 can include a system 600, a camera 700, and a hub 800. The system 600 can be the same as or similar to the system 500, and as shown in FIG. 19A can be secured to the subject's foot. In this example, the subject can be an infant (which can also be referred to as a "baby" or a "child" herein) and the monitoring system 1000 can be adapted to monitor at least one physiological parameter, motion, and/or location of the infant. In some implementations, the system 600 can be the same or similar to and include any of the functionality and/or features of any of the systems described herein, such as systems 100, 300, and/or 400. The camera 700 can be positioned to observe the subject. For example, the camera 700 can be mounted to a wall near a crib of the infant where the camera can observe the infant, to furniture near the infant, or the like. The hub 800 can be positioned within wireless communication distance of the system 600 and/or the camera 700. For example, the hub 700 can be positioned within the room of the infant and/or within a room of the infant's parents and/or care providers. Various other optional aspects of system 600, camera 700, and hub 800 are described below with respect to FIGS. 20-22D.

As shown in FIG. 19B, the components of the monitoring system 1000 can be configured to wirelessly communicate with one another and/or one or more separate electronic device(s) 900. For example and as shown, the system 600 can be configured to wirelessly communicate with the hub 800 and vice versa, and/or the camera 700 and vice versa. Continuing with this example, the camera 700 can be configured to wirelessly communicate with the hub 800 and vice versa. Furthermore, the hub 800 and/or the camera 700 can be configured to wirelessly communicate with the separate electronic device 900 and vice versa. In some implementations, the hub 800 can receive all information wirelessly provided by the system 600 and/or camera 700 and transfer such information wirelessly to the separate electronic device 900. In some implementations, the camera 700 can receive all information wirelessly provided by the system 600 and transfer such information wirelessly to the separate electronic device 900. The separate electronic device 900 can be any of the electronic devices described herein or others, such as a patient monitor, a cell phone, a server, a soundbar, and/or a speaker. Communication between system 600, camera 700, and/or hub 800 with electronic device 900 can be via a network. Such a configuration can advantageously provide access to the monitoring system 1000 by, for example, parents and/or care providers of the infant who may be located in a different location in which system 1000 is located. Wireless communication can be via any of the wireless communication protocols described herein. For example, the components of the monitoring system 1000 can be configured to communicate via Bluetooth and/or WiFi. As another example, the components of the monitoring system 1000 can be configured to pair with one another via NFC. Although FIG. 19A illustrates hub 800 being in proximity to camera 700 and system 600, in some situations, hub 800 can be in a different location as camera 700 and/or system 600 (for example, a different room of a house).

FIG. 20 illustrates a schematic diagram of certain features which can be incorporated in the system 600 as well as any other implementations of system(s) described herein. Any of the features described with respect to system 600 can be incorporated into any of the other systems described herein (such as system 100, 200, 300, 400, and/or 500). Similarly, system 600 can be embodied in a form as shown and/or described herein with respect to any of the systems 100, 200, 300, 400, and/or 500. As one example, in some implementations, FIG. 20 may represent a schematic diagram of sensor component 503 of system 500.

As shown, the system 600 can include one or more emitters 620, one or more detectors 622, and one or more temperature sensors 624, one or more processors 602, one or more storage devices 604, a communication module 606, a battery 608, an information element 610, one or more other sensors 626, one or more status indicators 612, a vibration motor 614, one or more accelerometers 616, and/or one or more gyroscopes 618. As a nonlimiting example, the one or more emitters 620, one or more detectors 622, one or more temperature sensors 624, one or more processors 602, one or more storage devices 604, communication module 606, battery 608, information element 610, one or more other sensors 626, one or more status indicators 612, vibration motor 614, one or more accelerometers 616, and one or more gyroscopes 618 can be the same as or similar to or include any one or more features and/or functionality of the one or more emitters 104a, one or more detectors 104b, one or more temperature sensors 104c, one or more processors 106a, one or more storage devices 106b, communication module 106c, battery 106d, information element 106e, one or more other sensors 106f which can include one or more accelerometers and/or one or more gyroscopes, one or more status indicators 106g, and vibration motor 106h described herein.

The one or more accelerometers 616 and/or one or more gyroscopes 618 of the system 600 can be utilized to determine movement, position, orientation, location, and/or other characteristics of the subject and/or a portion of the subject's body (for example, foot 2, ankle 3, heel 4, and/or lower leg 5). For example, the one or more accelerometers 616 and/or one or more gyroscopes 618 of the system 600 can be utilized to determine if the subject is laying down, on their back, on their side, on their stomach, on all fours, on their knees, partially standing, standing, sleeping, awake, moving, and/or not moving. In some implementations, the one or more accelerometers 616 and/or one or more gyroscopes 618 of the system 600 can be determine movement, position, orientation, location, and/or other characteristics of the subject and/or a portion of the subject's body similar or identical that described and/or illustrated in U.S. Pat. Pub. No. 2023/0045000, titled "Patient Monitoring Device with Improved User Interface".

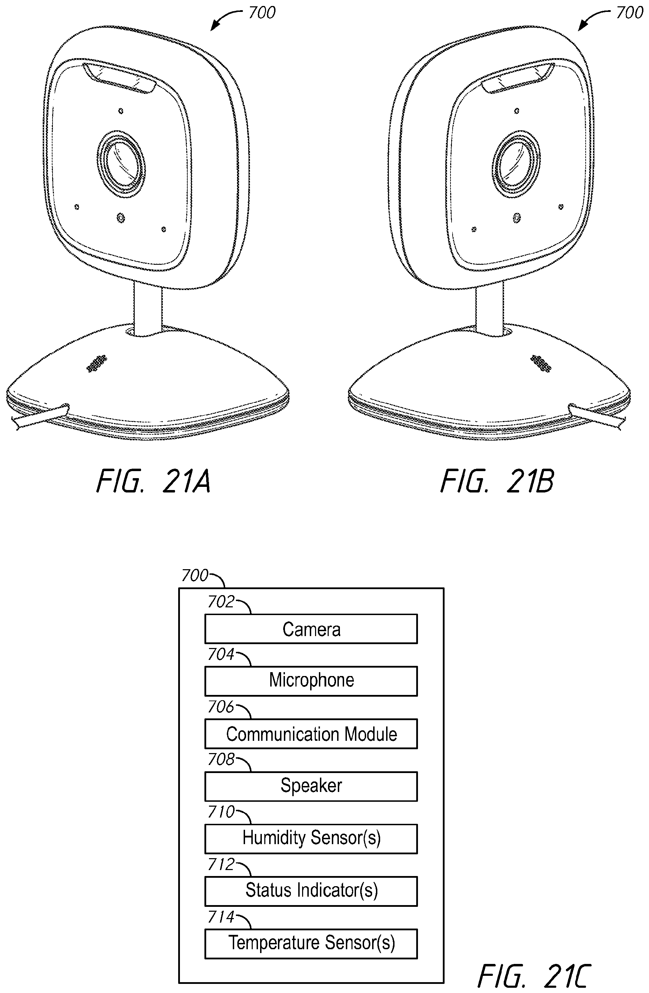

FIGS. 21A-21B illustrate various perspective views of an implementation of the camera 700 of the monitoring system 1000 of FIGS. 19A-19B. As mentioned, the camera 700 can be configured to monitor the subject when the monitoring system 1000 is in use. For this, the camera 700 can incorporate any one or more features and/or functionality of the charging station 200 described herein as well as additional features and/or functionality. For example, the camera 700 can include a camera 702, a microphone 704, a communication module 706, a speaker 708, one or more humidity sensors 710, one or more status indicators 712, and/or one or more temperature sensors 714 as shown in FIG. 21C. The camera 702 can be configured for high definition capture of the subject in day, night, high light, low light, and/or no light environments. In some implementations, the camera 702 can be configured for night vision. The resolution of the camera 702 can be 720, 1080, 2k, 4k, or any resolution that provides the camera the ability to monitor the subject and/or its environment. The microphone 704 can be configured to capture/monitor sound from the subject and/or its environment. The communication module 706 can be configured the same or similar to any of the communication modules described herein and can facilitate wireless communication of information collected and/or processed by the camera 700 (e.g., by one or more processors of the camera) to other components of the monitoring system 1000 and/or separate electronic device 900 connected thereto. The one or more humidity sensors 710 and one or more temperature sensors 714 can be configured to monitor the humidity and temperature of the environment in which the camera 700 is located. The camera 700 can be configured to allow communication between a parent/care giver and an infant subject via the microphone 704 and speaker 708. Furthermore, in some implementations the camera 700 can be configured to sound an alarm depending on one or more physiological parameters, motion, and/or location of the subject being monitored by the monitoring system 1000. The camera 700 can be configured to be wall or ceiling mounted, furniture mounted (e.g., to a portion of a crib), or otherwise positionable such that it can monitor the subject and/or its environment.

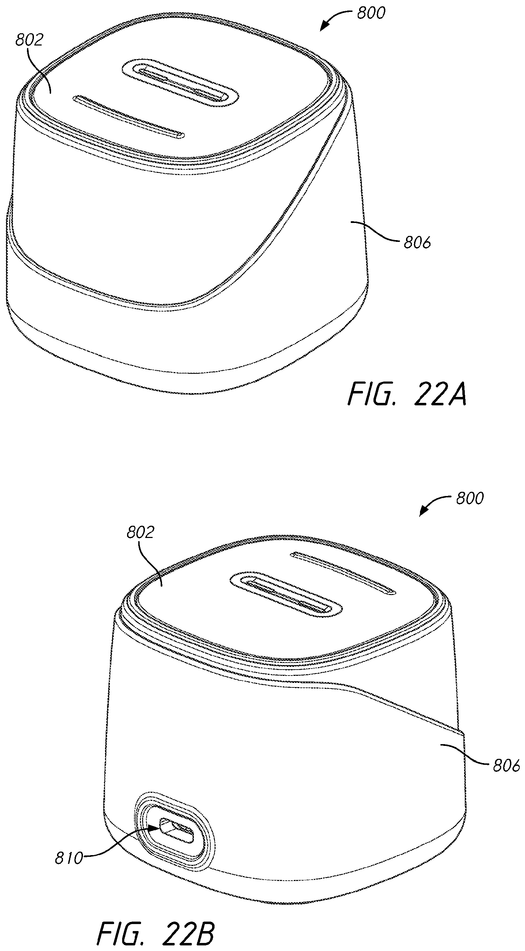

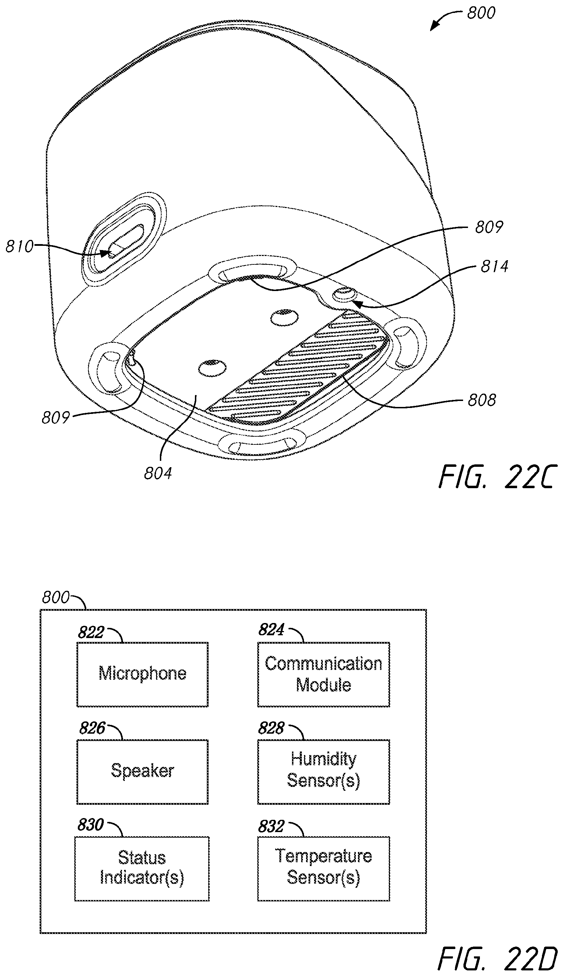

FIGS. 22A-22C illustrate various perspective views of the hub 800 of the monitoring system 1000 of FIG. 19A-19B. The hub 800 can be similar to and incorporate any one or more features and/or functionality of the charging station 200 described herein. For example and as shown in FIG. 22D, the hub 800 can include a communication module 824, a speaker 826, and a status indicator 830 the same as or similar to such components of the charging station 200. Furthermore, the hub 800 can include a top surface or button 802, a body 806, an electrical connector 810, a bottom surface 804, opening(s) 808, opening(s) 809, and reset button 814 the same or similar to the top surface or button 202, body 206, electrical connector 210, bottom surface 204, opening(s) 208, opening(s) 209, and reset button 214 of the charging station 200. For example, the button 802 can be pressed to snooze an alarm of the hub 800, to power on or off the hub 800, or to otherwise interact with the hub 800. The hub 800 can differ from the charging station 200 in that instead of being configured to receive and charge a sensor hub, the hub 800 can include a microphone 822, one or more humidity sensors 828, and/or one or more temperature sensors 832. The microphone 822, one or more humidity sensors 828, and one or more temperature sensors 832 can be configured to monitor the sound, humidity, and temperature of the environment in which the hub 800 is located. As shown in FIG. 19A, in some implementations it can be desirable to locate the hub 800 in the environment proximate the subject. In some cases, it can be advantageous to locate the hub 800 in an environment proximate parents and/or care providers of the subject when the subject is an infant/child. In some implementations, more than one hub 800 can be provided with the monitoring system 1000. The hub 800 can be configured to allow communication between a parent/care giver and an infant subject via the microphone 822 and speaker 826. Furthermore, in some implementations the hub 800 can be configured to sound an alarm depending on one or more physiological parameters, motion, and/or location of the subject being monitored by the monitoring system 1000.

The monitoring system 1000 can advantageously be configured to monitor at least one physiological parameter, motion, and/or location of the subject 1 as described herein. For example, the monitoring system 1000 can be configured to monitor, measure, or otherwise determine vital signs of the subject 1, which can include SpO2, heart/pulse rate, respiratory rate, temperature, oxygen saturation, and/or pulse rate of the subject 1. The monitoring system 1000 can also be configured to produce an alarm (e.g., alarm a parent and/or care provider of the subject 1) based on any one or more of the physiological parameters, motion, and/or location of the subject 1.

Any of the components of the monitoring system 1000 can be configured to pair with one another wirelessly. For example, components of the monitoring system 1000 can be configured to pair with one another via NFC and/or Bluetooth (e.g., low energy Bluetooth). Furthermore, components of the monitoring system 1000 can be configured to pair with separate electronic device(s), such as separate electronic device 900, wirelessly which can include via NFC and/or Bluetooth (e.g., low energy Bluetooth). In some implementations, no buttons or button presses may be required to pair components of the monitoring system 1000 and/or components of the monitoring system 1000 with one or more separate electronic devices. Furthermore, and as described herein, any of the components of the monitoring system 1000 can be configured to communicate with one another or with separate electronic devices 900 via WiFi.

In some implementations, the monitoring system 1000 can include software configured to allow a subject, or their parents or care givers when the subject is an infant, to access and/or interact with the monitoring system 1000. For example, the monitoring system 1000 can include an application accessible via the separate electronic device 900 that the subject/parents thereof/care givers thereof can use to access and/or interact with the monitoring system 1000 and/or any of its components. Such software can include one or more algorithms to enable the monitoring system 1000 to process data, physiological parameters, motion, and/or location measured and/or determined by any one or more of the components of the monitoring system 1000.

When the subject is an infant as described herein, the monitoring system 1000 can be configured to enable parents and/or care givers of the infant to monitor various aspects of the infant's health, wellbeing, and/or safety. For example, the monitoring system 1000 can be configured to allow a parent/care giver to set a monitoring zone around the infant. Such a monitoring zone can be a sleeping environment or a play environment, among others, of the infant. The monitoring system 1000 can be configured to monitor such monitoring zone and alarm the parent/care provider if needed. For example, the monitoring system 1000 can be configured to detect: infant safety within such monitoring zone (e.g., hand or foot outside of crib, infant about to fall or climb on or out of various structures); if any foreign objects (e.g., pillows, toys, pets, household items, cellular phone) are in, have moved within, and/or have entered such monitoring zone; infant positioning within such monitoring zone (e.g., laying down, one their back, on their side, on their stomach, on all fours, on their knees, partially standing, standing, face up, face down, breathing blocked, breathing unblocked, and/or a relative positioning of the infant relative to aspects of their environment, such as in a center of a crib or near a side thereof); and/or infant activity within such monitoring zone (e.g., sleeping, awake, moving/walking/crawling, not moving, crying, start of crying, breathing, and/or not breathing). Components of the monitoring system 1000 can advantageously work in combination for the detection of the various aspects above. For example, the camera 700 and the system 600 can work in combination to provide positioning information about the infant subject (e.g., the accelerometer(s) 616 and/or gyroscope(s) 618 of the system 600 can work in combination with the camera 700 to determine positioning of the infant subject and/or portions thereof). As another example, the camera 700 and the system 600 can work in combination to provide any one or more of the vital signs of the infant subject (e.g., the camera 700 can be configured to monitor and determine vital signs of the subject such as heart rate, respiration rate, and others). In some implementations, the monitoring zone can include anything or everything within view of the camera 700 and/or anything within range of the microphone of the camera 700 and/or the hub 800. In some implementations, the monitoring zone can be set to include a crib, a bassinet, a play area, a bathing area, or other area of the infant subject. In some implementations, the monitoring system 1000 can be configured to provide universal and continuous access to video, sound, and vitals of the subject. The monitoring system 1000 can be configured to provide customizable alerts and/or alarms based on aspects of the infant subject and/or their environment. In some implementations, the monitoring system 1000 can provide live vital tracking with access to detailed data history, a knowledge and/or video library, connectivity to social media, and/or a connection to physician(s)/hospital(s)/care provider(s).

In some implementations, the monitoring system 1000 includes only the system 600 and the camera 700. In some implementations, the monitoring system 1000 includes only the system 600, the camera 700, and the one or more separate electronic device(s) 900. In some implementations, the monitoring system 1000 includes the system 600, the camera 700, the hub 800, and the one or more separate electronic device(s) 900. In some implementations of the monitoring system 1000 including at least the camera 700 and the hub 800, the camera 700 and the hub 800 are configured to be placed in different rooms from one another.

Although examples and certain orientations and configurations of various aspects of the systems described in this disclosure (e.g., systems 100, 300, 400, 500, and 600) have been provided, alternative orientations and configurations for such aspects are to be considered included as a part of this disclosure. For example, in some implementations, the wearable device straps 166, 366, 466, 566 and/or 666 can be omitted. In such implementations, the sensor straps 130, 330, 430, 530 and/or 630 can be the only components of the sensor docks 104 and/or 304 and/or sensor assemblies 103, 303, 403, 503 and/or 603 (and/or of the systems 100, 300, 400, 500 and/or 600) that secure the wearable devices 102, 302, 402, 502 and/or 602, respectively, to the subject's foot 2. As another example, in some implementations, the wearable device straps 166, 366, 466, 566 and/or 666 can be combined with and/or coupled with the sensor straps 130, 330, 430, 530 and/or 630 (e.g., so as to form one larger strap that can be configured to secure the wearable devices 102, 302, 402, 502 and/or 602 to the subject's foot 2). Any of the straps described herein can be configured to wrap around at least a portion of the subject's foot 2, which can include the bottom, the top, one or more sides, and/or in some cases an entirety of the subject's foot 2. Further, although certain methods of securement for the straps described herein have been provided, other methods can be used, including via magnets and/or adhesives. Furthermore, in some implementations the wearable devices 102, 302, 402, 502 and/or 602 described herein can comprise a fabric or non-resilient material. In such implementations, the wearable devices 102, 302, 402, 502 and/or 602 and any straps thereof can be configured to wrap around portions of the subject's foot to secure the systems 100, 300, 400, 500 and/or 600 to the subject. In some implementations, the wearable devices 102, 302, 402, 502 and/or 602 described herein can comprise portions that are resilient, flexible, and/or rigid. In some implementations, the wearable devices 102, 302, 402, 502 and/or 602 described herein can comprise portions made of silicone rubber and portions made of fabric.

Other orientations and configurations of the sensor straps 130, 330, 430, 530 and/or 630 are also to be considered included as a part of this disclosure. For example, although the sensor straps 130, 330, 430, 530 and 630 can be described herein as including one or more emitters (e.g., such as emitters 104a, 304a, 404a, and 504a), one or more detectors (e.g., such as detectors 104b, 304b, 404b, and 504b), and/or one temperature sensors (e.g., such as temperature sensors 104c, 304c, 404c and/or 404d, 505c and/or an additional temperature sensor), more than each of such emitters, detectors, and/or temperature sensors can be included in the sensor straps 130, 330, 430, 530, 630 and/or the sensor hubs 404, 504, 604. In some implementations, the sensor straps 130, 330 and/or 430 can include an array of emitters, an array of detectors, and/or an array of temperature sensors. Such arrays can be configured to extend along the length of the sensor straps 130, 330, and/or 430 such that multiple emitters can be located proximate to each other, multiple detectors can be located proximate to each other, and/or multiple temperature sensors can be located proximate to each other. Advantageously, such arrays can facilitate the measure of at least one physiological parameter of the subject, for example, by providing the systems options for which emitters, detectors, and/or temperature sensors to utilize for measurements (e.g., the system can cycle through such sensors and use ones that provide the best signal for physiological parameter determination). Additionally, although the emitters 104a, 304a, 404a, 504a and 604a in some implementations have been described herein as being adjacent tissue of the top of the subject's foot 2 and the detectors 104b, 304b, 404b, 504b and 604b in some implementations have been described herein as being adjacent tissue of the bottom of the subject's foot 2 when the systems 100, 300, 400, 500 and 600, respectively, are secured to the subject's foot 2, their locations can be swapped (e.g., detector(s) can be configured to be adjacent tissue of the top of the subject's foot 2 and emitter(s) can be configured to be adjacent tissue of the bottom of the subject's foot 2).

Alternative configurations of the holders 170 and 370 are also to be considered included as a part of this disclosure. For example, although the holders 170 and 370 have been shown herein as having an enclosed perimeter so as to form the cavities 172 and 372 for receiving the sensor dock 104/sensor hub 106 and the sensor dock 304/sensor hub 306, respectively, in some implementations the holders 170 and/or 370 can have an open side to facilitate the releasable connection/disconnection of the sensor hubs 106 and/or 306 with the sensor docks 104 and/or 304.

The systems described herein, such as the systems 100, 300, 400, 500 and 600 and/or any of their components can be configured to be waterproof, water resistant, drip proof, shock proof, dust proof, and/or dust resistant. While the systems have been described as having a rechargeable battery, the battery can be nonrechargeable or single use. In some implementations, a battery of the system (such as battery 106d and/or the implementation of such a battery 165) can be rechargeable but non-removable from the device. In such a case, the system can include a charge port configured to receive a power cable for charging and/or an electrical connector configured to receive a charger. Further in such a case, the system can be used by the subject while charging (e.g., the system can be in an operational mode while charging). In some variants, a sensor hub of any of the systems described herein (such as sensor hubs 106 and 306) can be permanently connected to a sensor dock of any of the systems described herein (such as sensor docks 104 and 304). In such variants, the combined sensor hub/sensor dock can have a charge port or electrical connector for charging.

In some implementations, any or all of the components of the systems as described herein can be configured to be reusable (which may also be referred to herein as "durable"). For example, in reference to the system 100, the wearable device 102, the sensor dock 104, and the sensor hub 106 can all be configured to be reusable (e.g., for days, weeks, months, or more). In such a case, all components can be sanitized between uses and/or between subjects. In some implementations, all components of the systems as described herein can be configured to be reusable except for the wearable devices as described herein, such as wearable devices 102, 302, 402, 502 and/or 602. In some cases, all components of the systems as described herein can be configured to be reusable between subjects except for the wearable devices as described herein, such as wearable devices 102, 302, 402, 502 and/or 602 (e.g., subjects do not share use of a wearable device). In some implementations, the sensor hubs as described herein, such as sensor hubs 106 and 306, last longer than all other components of the system and can be reused if desired between subjects. In some implementations, one or more components of the systems as described herein and any portions thereof can be configured as single use (which may be referred to herein as "disposable"). In such implementations, the sensor hubs and sensor docks as described herein can be integrated, a part of, and/or otherwise combined with the wearable devices as described herein to provide for a single and fully integrated device that can be secured to the subject's foot 2. Furthermore, in such an implementation, the systems can include a single use battery and/or non-rechargeable battery (e.g., a zinc-air battery). In some cases, all components of the systems as described herein can be configured to be single use except for the sensor hubs (e.g., sensor hubs 106, 306, 404, 504, 604).

### Additional Considerations and Terminology

Certain categories of persons, such as caregivers, clinicians, doctors, nurses, and friends and family of a subject, may be used interchangeably to describe a person providing care to the subject. Furthermore, subjects, patients, or users used herein interchangeably refer to a person who is wearing a sensor or is connected to a sensor or whose measurements are used to determine a physiological parameter or a condition. Parameters may be, be associated with, and/or be represented by, measured values, display icons, alphanumeric characters, graphs, gages, power bars, trends, or combinations. Real time data may correspond to active monitoring of a subject, however, such real time data may not be synchronous to an actual physiological state at a particular moment. Measurement value(s) of a parameter such as any of those discussed herein, unless specifically stated otherwise, or otherwise understood with the context as used is generally intended to convey a measurement or determination that is responsive to and/or indicative of the physiological parameter.

Conditional language used herein, such as, among others, "can," "could," "might," "may," "e.g.," and the like, unless specifically stated otherwise, or otherwise understood within the context as used, is generally intended to convey that certain features, elements, and/or steps are optional. Thus, such conditional language is not generally intended to imply that features, elements, and/or steps are in any way required or that one or more implementations necessarily include logic for deciding, with or without other input or prompting, whether these features, elements, and/or steps are included or are to be always performed. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain implementations require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain implementations, the terms "generally parallel" and "substantially parallel" refer to a value, amount, or characteristic that departs from exactly parallel by less than or equal to 10 degrees, 5 degrees, 3 degrees, or 1 degree. As another example, in certain implementations, the terms "generally perpendicular" and "substantially perpendicular" refer to a value, amount, or characteristic that departs from exactly perpendicular by less than or equal to 10 degrees, 5 degrees, 3 degrees, or 1 degree.

Although certain implementations and examples have been described herein, it will be understood by those skilled in the art that many aspects of the systems and devices shown and described in the present disclosure may be differently combined and/or modified to form still further implementations or acceptable examples. All such modifications and variations are intended to be included herein within the scope of this disclosure. A wide variety of designs and approaches are possible. No feature, structure, or step disclosed herein is essential or indispensable.

Any methods disclosed herein need not be performed in the order recited. The methods disclosed herein may include certain actions taken by a practitioner; however, they can also include any third-party instruction of those actions, either expressly or by implication.

The methods and tasks described herein may be performed and fully automated by a computer system. The computer system may, in some cases, include multiple distinct computers or computing devices (e.g., physical servers, workstations, storage arrays, cloud computing resources, etc.) that communicate and interoperate over a network to perform the described functions. Each such computing device typically includes a processor (or multiple processors) that executes program instructions or modules stored in a memory or other non-transitory computer-readable storage medium or device (e.g., solid state storage devices, disk drives, etc.). The various functions disclosed herein may be embodied in such program instructions, and/or may be implemented in application-specific circuitry (e.g., ASICs or FPGAs) of the computer system. Where the computer system includes multiple computing devices, these devices may, but need not, be co-located. The results of the disclosed methods and tasks may be persistently stored by transforming physical storage devices, such as solid state memory chips and/or magnetic disks, into a different state. The computer system may be a cloud-based computing system whose processing resources are shared by multiple distinct business entities or other users.

Depending on the implementation, certain acts, events, or functions of any of the processes or algorithms described herein can be performed in a different sequence, can be added, merged, or left out altogether (for example, not all described operations or events are necessary for the practice of the algorithm). Moreover, in certain implementations, operations or events can be performed concurrently, e.g., through multi-threaded processing, interrupt processing, or multiple processors or processor cores or on other parallel architectures, rather than sequentially.

Various illustrative logical blocks, modules, routines, and algorithm steps that may be described in connection with the disclosure herein can be implemented as electronic hardware (e.g., ASICs or FPGA devices), computer software that runs on general purpose computer hardware, or combinations of both. Various illustrative components, blocks, and steps may be described herein generally in terms of their functionality. Whether such functionality is implemented as specialized hardware versus software running on general-purpose hardware depends upon the particular application and design constraints imposed on the overall system. The described functionality can be implemented in varying ways for each particular application, but such implementation decisions should not be interpreted as causing a departure from the scope of the disclosure.

Moreover, various illustrative logical blocks and modules that may be described in connection with the disclosure herein can be implemented or performed by a machine, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can include electrical circuitry configured to process computer-executable instructions. A processor can include an FPGA or other programmable device that performs logic operations without processing computer-executable instructions. A processor can also be implemented as a combination of computing devices, e.g., a combination of a DSP and a microprocessor, a plurality of microprocessors, one or more microprocessors in conjunction with a DSP core, or any other such configuration. Although described herein primarily with respect to digital technology, a processor device may also include primarily analog components. For example, some or all of the rendering techniques described herein may be implemented in analog circuitry or mixed analog and digital circuitry. A computing environment can include any type of computer system, including, but not limited to, a computer system based on a microprocessor, a mainframe computer, a digital signal processor, a portable computing device, a device controller, or a computational engine within an appliance, to name a few.

The elements of any method, process, routine, or algorithm described in connection with the disclosure herein can be embodied directly in hardware, in a software module executed by a processor device, or in a combination of the two. A software module can reside in RAM memory, flash memory, ROM memory, EPROM memory, EEPROM memory, registers, hard disk, a removable disk, a CD-ROM, or any other form of a non-transitory computer-readable storage medium. An exemplary storage medium can be coupled to the processor device such that the processor device can read information from, and write information to, the storage medium. In the alternative, the storage medium can be integral to the processor device. The processor device and the storage medium can reside in an ASIC. The ASIC can reside in a user terminal. In the alternative, the processor device and the storage medium can reside as discrete components in a user terminal.

## Claims

1. A system (100, 300, 400, 500) for measuring at least one physiological parameter of a subject (1), the system (100, 300, 400, 500) comprising:
a wearable device (102, 302, 402, 502) configured to be secured to a foot (2) of the subject (1); and
a sensor component (103, 303, 403, 503) removably securable to the wearable device (102, 302, 402, 502) and comprising one or more sensors for measuring said at least one physiological parameter of the subject (1), said sensor component (103, 303, 403, 503) further comprising a sensor strap (130, 330, 430, 530) configured to be wrapped around a portion of the subject's foot (2) and secured to a portion of the wearable device (102, 302, 402, 502), the sensor strap (130, 330, 430, 530) configured to secure the wearable device (102, 302, 402, 502) and the sensor component (103, 303, 403, 503) to the subject's foot (2).

2. The system (100, 300, 400, 500) of Claim 1, wherein:
said sensor strap (130, 330, 430, 530) comprises a first portion of the sensor component (103, 303, 403, 503) that is configured to be wrapped around said portion of the subject's foot (2) and secured to a first portion of the wearable device (102, 302, 402, 502); and
a second portion of the sensor component (103, 303, 403, 503) is configured to be removably secured to a second portion of the wearable device (102, 302, 402, 502).

3. The system (100, 300, 400, 500) of Claim 2, wherein the wearable device (102, 302, 402, 502) defines a first volume configured to receive the subject's foot (2) and a second volume configured to removably receive said second portion of the sensor component (103, 303, 403, 503).

4. The system (100, 300, 400, 500) of Claim 3, wherein the wearable device (102, 302, 402, 502) comprises:
a base (160, 360, 460, 560) configured to contact at least a portion of a bottom of the subject's foot (2), said second volume of said wearable device (102, 302, 402, 502) formed by a cavity (172, 372, 472, 572) of said base (160, 360, 460, 560); and
a wall (162, 362, 462, 562) extending outward from the base (160, 360, 460, 560) and configured to surround a heel (4) and at least a portion of one or more sides of the subject's foot (2).

5. The system (100, 300, 400, 500) of Claim 4, wherein:
the wearable device (102, 302, 402, 502) further comprises a frame (480, 580) arranged within said cavity (172, 372, 472, 572), said frame (480, 580) configured to removably secure said second portion of the sensor component (103, 303, 403, 503);
said base (160, 360, 460, 560) and said wall (162, 362, 462, 562) form a unitary structure made of a first material; and
said frame (480, 580) is made of a second material that is more rigid than the first material.

6. The system (100, 300, 400, 500) of any of Claims 4-5, wherein said first portion of the wearable device (102, 302, 402, 502) is arranged on a portion of said wall (162, 362, 462, 562).

7. The system (100, 300, 400, 500) of Claim 6, wherein said first portion of the wearable device (102, 302, 402, 502) comprises an opening in said portion of said wall (162, 362, 462, 562), and wherein said sensor strap (130, 330, 430, 530) is configured to be inserted through said opening.

8. The system of any of Claims 4-7, wherein said first volume is defined by said base (160, 360, 460, 560) and said wall (162, 362, 462, 562) at a location above said cavity (172, 372, 472, 572) of said base (160, 360, 460, 560).

9. The system (100, 300, 400, 500) of any of Claims 4-8, wherein said wall (162, 362, 462, 562) extends around a portion of a perimeter edge of said base (160, 360, 460, 560).

10. The system (100, 300, 400, 500) of Claim 9, wherein said wall (162, 362, 462, 562) extends around less than an entirety of said perimeter edge of said base (160, 360, 460, 560).

11. The system of any of Claims 4-10, wherein said wall (162, 362, 462, 562) does not extend around an entirety of said cavity (172, 372, 472, 572).

12. The system (100, 300, 400, 500) of any of Claims 2-11, wherein said sensor component (103, 303, 403, 503) comprises:
a sensor hub (106, 306, 404, 504) comprising one or more processors (106a), said sensor hub (106, 306, 404, 504) configured to be removably secured to said second portion of the wearable device (102, 302, 402, 502), wherein said sensor strap (130, 330, 430, 530) is connected to and extends outward from the sensor hub (106, 306, 404, 504);
one or more emitters (104a, 304a, 404a, 504a) configured to emit optical radiation into tissue of the subject's foot (2), said one or more emitters (104a, 304a, 404a, 504a) located within the sensor hub (106, 306, 404, 504); and
one or more detectors (104b, 304b, 404b, 504b) configured to detect at least a portion of the emitted optical radiation after passing through the tissue and output at least one signal responsive to the detected optical radiation, said one or more detectors (104b, 304b, 404b, 504b) located within the sensor strap (130, 330, 430, 530), wherein the one or more processors (106a) of the sensor hub (106, 306, 404, 504) are configured to receive the at least one signal outputted by the one or more detectors (104b, 304b, 404b, 504b) to determine said at least one physiological parameter of the subject (1).

13. The system (100, 300, 400, 500) of Claim 12, wherein the system (100, 300, 400, 500) is configured such that, when the sensor hub (106, 306, 404, 504) is secured to said second portion of the wearable device (102, 302, 402, 502) and the sensor strap (130, 330, 430, 530) is secured to said first portion of the wearable device (102, 302, 402, 502): the one or more detectors (104b, 304b, 404b, 504b) are positioned adjacent a top or side portion of the subject's foot (2); and the one or more emitters (104a, 304a, 404a, 504a) are positioned adjacent a bottom portion of the subject's foot (2).

14. The system (100, 300, 400, 500)_of any of Claims 12-13, wherein the sensor hub (106, 306, 404, 504) and the sensor strap (130, 330, 430, 530) form a unitary structure.

15. The system (100, 300, 400, 500) of any of Claims 12-14, wherein the sensor strap (130, 330, 430, 530) comprises:
a first section (141, 341, 441, 541) connected to and extending outward from the sensor hub (106, 306, 404, 504), wherein the one or more detectors (104b, 304b, 404b, 504b) are positioned within the first section (141, 341, 441, 541); and
a second section (331, 431, 531) that is releasably connectable to the first section (341, 441, 541), wherein the second section (331, 431, 531) is configured to secure to said first portion of the wearable device (102, 302, 402, 502).

## Patentansprüche

1. System (100, 300, 400, 500) zum Messen mindestens eines physiologischen Parameters eines Subjekts (1), wobei das System (100, 300, 400, 500) Folgendes umfasst:
eine tragbare Vorrichtung (102, 302, 402, 502), die dazu konfiguriert ist, an einem Fuß (2) des Subjekts (1) befestigt zu werden; und
eine Sensorkomponente (103, 303, 403, 503), die entfernbar an der tragbaren Vorrichtung (102, 302, 402, 502) befestigbar ist und einen oder mehrere Sensoren zum Messen des mindestens einen physiologischen Parameters des Subjekts (1) umfasst, wobei die Sensorkomponente (103, 303, 403, 503) ferner einen Sensorriemen (130, 330, 430, 530) umfasst, der dazu konfiguriert ist, um einen Abschnitt des Fußes (2) des Subjekts gelegt und an einem Abschnitt der tragbaren Vorrichtung (102, 302, 402, 502) befestigt zu werden, wobei der Sensorriemen (130, 330, 430, 530) dazu konfiguriert ist, die tragbare Vorrichtung (102, 302, 402, 502) und die Sensorkomponente (103, 303, 403, 503) an dem Fuß (2) des Subjekts zu befestigen.

2. System (100, 300, 400, 500) nach Anspruch 1, wobei:
der Sensorriemen (130, 330, 430, 530) einen ersten Abschnitt der Sensorkomponente (103, 303, 403, 503) umfasst, der dazu konfiguriert ist, um den Abschnitt des Fußes (2) des Subjekts gelegt und an einem ersten Abschnitt der tragbaren Vorrichtung (102, 302, 402, 502) befestigt zu werden; und
ein zweiter Abschnitt der Sensorkomponente (103, 303, 403, 503) dazu konfiguriert ist, entfernbar an einem zweiten Abschnitt der tragbaren Vorrichtung (102, 302, 402, 502) befestigt zu werden.

3. System (100, 300, 400, 500) nach Anspruch 2, wobei die tragbare Vorrichtung (102, 302, 402, 502) einen ersten Raum, der dazu konfiguriert ist, den Fuß (2) des Subjekts aufzunehmen, und einen zweiten Raum, der dazu konfiguriert ist, den zweiten Abschnitt der Sensorkomponente (103, 303, 403, 503) entfernbar aufzunehmen, definiert.

4. System (100, 300, 400, 500) nach Anspruch 3, wobei die tragbare Vorrichtung (102, 302, 402, 502) Folgendes umfasst:
eine Basis (160, 360, 460, 560), die dazu konfiguriert ist, mit mindestens einem Abschnitt einer Unterseite des Fußes (2) des Subjekts in Kontakt zu kommen, wobei der zweite Raum der tragbaren Vorrichtung (102, 302, 402, 502) durch einen Hohlraum (172, 372, 472, 572) der Basis (160, 360, 460, 560) gebildet wird; und
eine Wand (162, 362, 462, 562), die sich von der Basis (160, 360, 460, 560) nach außen erstreckt und so konfiguriert ist, dass sie eine Ferse (4) und mindestens einen Abschnitt einer oder mehrerer Seiten des Fußes (2) des Subjekts umgibt.

5. System (100, 300, 400, 500) nach Anspruch 4, wobei:
die tragbare Vorrichtung (102, 302, 402, 502) ferner einen Rahmen (480, 580) umfasst, der innerhalb des Hohlraums (172, 372, 472, 572) angeordnet ist, wobei der Rahmen (480, 580) dazu konfiguriert ist, den zweiten Abschnitt der Sensorkomponente (103, 303, 403, 503) entfernbar zu befestigen;
die Basis (160, 360, 460, 560) und die Wand (162, 362, 462, 562) eine aus einem ersten Material hergestellte einstückige Struktur bilden; und
der Rahmen (480, 580) aus einem zweiten Material, das steifer als das erste Material ist, hergestellt ist.

6. System (100, 300, 400, 500) nach einem der Ansprüche 4-5, wobei der erste Abschnitt der tragbaren Vorrichtung (102, 302, 402, 502) auf einem Abschnitt der Wand (162, 362, 462, 562) angeordnet ist.

7. System (100, 300, 400, 500) nach Anspruch 6, wobei der erste Abschnitt der tragbaren Vorrichtung (102, 302, 402, 502) eine Öffnung in dem Abschnitt der Wand (162, 362, 462, 562) umfasst und wobei der Sensorriemen (130, 330, 430, 530) dazu konfiguriert ist, durch die Öffnung eingeführt zu werden.

8. System nach einem der Ansprüche 4-7, wobei der erste Raum durch die Basis (160, 360, 460, 560) und die Wand (162, 362, 462, 562) an einer Stelle über dem Hohlraum (172, 372, 472, 572) der Basis (160, 360, 460, 560) definiert ist.

9. System (100, 300, 400, 500) nach einem der Ansprüche 4-8, wobei sich die Wand (162, 362, 462, 562) um einen Abschnitt einer Umfangskante der Basis (160, 360, 460, 560) herum erstreckt.

10. System (100, 300, 400, 500) nach Anspruch 9, wobei sich die Wand (162, 362, 462, 562) um weniger als die gesamte Umfangskante der Basis (160, 360, 460, 560) herum erstreckt.

11. System nach einem der Ansprüche 4-10, wobei sich die Wand (162, 362, 462, 562) nicht um den gesamten Hohlraum (172, 372, 472, 572) herum erstreckt.

12. System (100, 300, 400, 500) nach einem der Ansprüche 2-11, wobei die Sensorkomponente (103, 303, 403, 503) Folgendes umfasst:
einen Sensorhub (106, 306, 404, 504), der einen oder mehrere Prozessoren (106a) umfasst, wobei der Sensorhub (106, 306, 404, 504) dazu konfiguriert ist, entfernbar an dem zweiten Abschnitt der tragbaren Vorrichtung (102, 302, 402, 502) befestigt zu werden, wobei der Sensorriemen (130, 330, 430, 530) mit dem Sensorhub (106, 306, 404, 504) verbunden ist und sich von diesem nach außen erstreckt;
einen oder mehrere Strahler (104a, 304a, 404a, 504a), die dazu konfiguriert sind, optische Strahlung in Gewebe des Fußes (2) des Subjekts auszusenden, wobei sich der eine oder die mehreren Strahler (104a, 304a, 404a, 504a) innerhalb des Sensorhubs (106, 306, 404, 504) befinden;
und
einen oder mehrere Detektoren (104b, 304b, 404b, 504b), die dazu konfiguriert sind, mindestens einen Teil der ausgesendeten optischen Strahlung nach dem Durchdringen des Gewebes zu detektieren und mindestens ein Signal als Reaktion auf die detektierte optische Strahlung auszugeben, wobei sich der eine oder die mehreren Detektoren (104b, 304b, 404b, 504b) innerhalb des Sensorriemens (130, 330, 430, 530) befinden, wobei der eine oder die mehreren Prozessoren (106a) des Sensorhubs (106, 306, 404, 504) dazu konfiguriert sind, das mindestens eine von dem einen oder den mehreren Detektoren (104b, 304b, 404b, 504b) ausgegebene Signal zu empfangen, um den mindestens einen physiologischen Parameter des Subjekts (1) zu bestimmen.

13. System (100, 300, 400, 500) nach Anspruch 12, wobei das System (100, 300, 400, 500) so konfiguriert ist, dass, wenn der Sensorhub (106, 306, 404, 504) an dem zweiten Abschnitt der tragbaren Vorrichtung (102, 302, 402, 502) befestigt ist und der Sensorriemen (130, 330, 430, 530) an dem ersten Abschnitt der tragbaren Vorrichtung (102, 302, 402, 502) befestigt ist: der eine oder die mehreren Detektoren (104b, 304b, 404b, 504b) neben einem oberen oder seitlichen Abschnitt des Fußes (2) des Subjekts positioniert sind; und der eine oder die mehreren Strahler (104a, 304a, 404a, 504a) neben einem unteren Abschnitt des Fußes (2) des Subjekts positioniert sind.

14. System (100, 300, 400, 500) nach einem der Ansprüche 12-13, wobei der Sensorhub (106, 306, 404, 504) und der Sensorriemen (130, 330, 430, 530) eine einstückige Struktur bilden.

15. System (100, 300, 400, 500) nach einem der Ansprüche 12-14, wobei der Sensorriemen (130, 330, 430, 530) Folgendes umfasst:
einen ersten Bereich (141, 341, 441, 541), der mit dem Sensorhub (106, 306, 404, 504) verbunden ist und sich von diesem nach außen erstreckt, wobei der eine oder die mehreren Detektoren (104b, 304b, 404b, 504b) innerhalb des ersten Bereichs (141, 341, 441, 541) positioniert sind; und
einen zweiten Bereich (331, 431, 531), der lösbar mit dem ersten Bereich (341, 441, 541) verbindbar ist, wobei der zweite Bereich (331, 431, 531) zur Befestigung an dem ersten Abschnitt der tragbaren Vorrichtung (102, 302, 402, 502) konfiguriert ist.

## Revendications

1. Système (100, 300, 400, 500) pour mesurer au moins un paramètre physiologique d'un sujet (1), le système (100, 300, 400, 500) comprenant :
un dispositif portable (102, 302, 402, 502) configuré pour être assujetti à un pied (2) du sujet (1) ; et
un composant capteur (103, 303, 403, 503) pouvant être assujetti de manière amovible au dispositif portable (102, 302, 402, 502) et comprenant un ou plusieurs capteurs pour mesurer ledit au moins un paramètre physiologique du sujet (1), ledit composant capteur (103, 303, 403, 503) comprenant en outre une sangle de capteur (130, 330, 430, 530) configurée pour être enroulée autour d'une partie du pied du sujet (2) et assujettie à une partie du dispositif portable (102, 302, 402, 502), la sangle de capteur (130, 330, 430, 530) configurée pour assujettir le dispositif portable (102, 302, 402, 502) et le composant capteur (103, 303, 403, 503) au pied du sujet (2).

2. Système (100, 300, 400, 500) selon la revendication 1, dans lequel :
ladite sangle de capteur (130, 330, 430, 530) comprend une première partie du composant capteur (103, 303, 403, 503) qui est configurée pour être enroulée autour de ladite partie du pied du sujet (2) et assujettie à une première partie du dispositif portable (102, 302, 402, 502) ; et
une deuxième partie du composant capteur (103, 303, 403, 503) est configurée pour être assujettie de manière amovible à une deuxième partie du dispositif portable (102, 302, 402, 502).

3. Système (100, 300, 400, 500) selon la revendication 2, dans lequel le dispositif portable (102, 302, 402, 502) définit un premier volume configuré pour recevoir le pied du sujet (2) et un deuxième volume configuré pour recevoir de manière amovible ladite deuxième partie du composant capteur (103, 303, 403, 503).

4. Système (100, 300, 400, 500) selon la revendication 3, dans lequel le dispositif portable (102, 302, 402, 502) comprend :
une base (160, 360, 460, 560) configurée pour entrer en contact avec au moins une partie d'un dessous du pied du sujet (2), ledit deuxième volume dudit dispositif portable (102, 302, 402, 502) formé par une cavité (172, 372, 472, 572) de ladite base (160, 360, 460, 560) ; et
une paroi (162, 362, 462, 562) s'étendant vers l'extérieur à partir de la base (160, 360, 460, 560) et configurée pour entourer un talon (4) et au moins une partie d'un ou de plusieurs côtés du pied du sujet (2).

5. Système (100, 300, 400, 500) selon la revendication 4, dans lequel :
le dispositif portable (102, 302, 402, 502) comprend en outre un cadre (480, 580) agencé à l'intérieur de ladite cavité (172, 372, 472, 572), ledit cadre (480, 580) configuré pour assujettir de manière amovible ladite deuxième partie du composant capteur (103, 303, 403, 503) ;
ladite base (160, 360, 460, 560) et ladite paroi (162, 362, 462, 562) forment une structure unitaire constituée d'un premier matériau ; et
ledit cadre (480, 580) est constitué d'un deuxième matériau qui est plus rigide que le premier matériau.

6. Système (100, 300, 400, 500) selon l'une quelconque des revendications 4 à 5, dans lequel ladite première partie du dispositif portable (102, 302, 402, 502) est agencée sur une partie de ladite paroi (162, 362, 462, 562).

7. Système (100, 300, 400, 500) selon la revendications 6, dans lequel ladite première partie du dispositif portable (102, 302, 402, 502) comprend une ouverture dans ladite partie de ladite paroi (162, 362, 462, 562), et dans lequel ladite sangle de capteur (130, 330, 430, 530) est configurée pour être insérée à travers ladite ouverture.

8. Système selon l'une quelconque des revendications 4 à 7, dans lequel ledit premier volume est défini par ladite base (160, 360, 460, 560) et ladite paroi (162, 362, 462, 562) à un emplacement au-dessus de ladite cavité (172, 372, 472, 572) de ladite base (160, 360, 460, 560).

9. Système (100, 300, 400, 500) selon l'une quelconque des revendications 4 à 8, dans lequel ladite paroi (162, 362, 462, 562) s'étend autour d'une partie d'un bord périmétrique de ladite base (160, 360, 460, 560).

10. Système (100, 300, 400, 500) selon la revendications 9, dans lequel ladite paroi (162, 362, 462, 562) s'étend autour de moins d'une totalité dudit bord périmétrique de ladite base (160, 360, 460, 560).

11. Système selon l'une quelconque des revendications 4 à 10, dans lequel ladite paroi (162, 362, 462, 562) ne s'étend pas autour d'une totalité de ladite cavité (172, 372, 472, 572).

12. Système (100, 300, 400, 500) selon l'une quelconque des revendications 2 à 11, dans lequel ledit composant capteur (103, 303, 403, 503) comprend :
un concentrateur de capteur (106, 306, 404, 504) comprenant un ou plusieurs processeurs (106a), ledit concentrateur de capteur (106, 306, 404, 504) configuré pour être assujetti de manière amovible à ladite deuxième partie du dispositif portable (102, 302, 402, 502), dans lequel ladite sangle de capteur (130, 330, 430, 530) est connectée à et s'étend vers l'extérieur à partir du concentrateur de capteur (106, 306, 404, 504) ;
un ou plusieurs émetteurs (104a, 304a, 404a, 504a) configurés pour émettre un rayonnement optique dans un tissu du pied du sujet (2), lesdits un ou plusieurs émetteurs (104a, 304a, 404a, 504a) situés à l'intérieur du concentrateur de capteur (106, 306, 404, 504) ; et
un ou plusieurs détecteurs (104b, 304b, 404b, 504b) configurés pour détecter au moins une partie du rayonnement optique émis après avoir traversé le tissu et fournir en sortie au moins un signal en réponse au rayonnement optique détecté, lesdits un ou plusieurs détecteurs (104b, 304b, 404b, 504b) situés à l'intérieur de la sangle de capteur (130, 330, 430, 530), les un ou plusieurs processeurs (106a) du concentrateur de capteur (106, 306, 404, 504) étant configurés pour recevoir les un ou plusieurs signaux émis par les un ou plusieurs détecteurs (104b, 304b, 404b, 504b) pour déterminer ledit au moins un paramètre physiologique du sujet (1).

13. Système (100, 300, 400, 500) selon la revendication 12, dans lequel le système (100, 300, 400, 500) est configuré de telle sorte que, lorsque le concentrateur de capteur (106, 306, 404, 504) est assujetti à ladite deuxième partie du dispositif portable (102, 302, 402, 502) et que la sangle de capteur (130, 330, 430, 530) est assujettie à ladite deuxième partie du dispositif portable (102, 302, 402, 502) : les un ou plusieurs détecteurs (104b, 304b, 404b, 504b) sont positionnés adjacents à une partie de dessus ou de côté du pied du sujet (2) ; et les un ou plusieurs émetteurs (104a, 304a, 404a, 504a) sont positionnées adjacents à une partie de dessous du pied du sujet (2).

14. Système (100, 300, 400, 500) selon l'une quelconque des revendications 12 à 13, dans lequel ledit concentrateur de capteur (106, 306, 404, 504) et la sangle de capteur (130, 330, 430, 530) forment une structure unitaire.

15. Système (100, 300, 400, 500) selon l'une quelconque des revendications 12 à 14, dans lequel la sangle de capteur (130, 330, 430, 530) comprend :
une première section (141, 341, 441, 541) connectée à et s'étendant vers l'extérieur à partir du concentrateur de capteur (106, 306, 404, 504), dans lequel les un ou plusieurs détecteurs (104b, 304b, 404b, 504b) sont positionnées à l'intérieur de la première section (141, 341, 441, 541) ; et
une deuxième section (331, 431, 531) qui peut être connectée de manière amovible à la première section (341, 441, 541), la deuxième section (331, 431, 531) étant configurée pour s'assujettir à ladite première partie du dispositif portable (102, 302, 402, 502).
